# EUROPEAN PATENT SPECIFICATION

(11) **EP 3 500 301 B1**
(45) Date of publication and mention of the grant of the patent: **30.08.2023**
(21) Application number: 17841965.1
(22) Date of filing: 15.08.2017
(51) Int. Cl.: C07K 16/14, C07K 16/28, C07K 16/46, A61K 39/395, A61K 45/06, A61K 51/10, A61P 17/06, A61P 35/00, A61P 35/02

(54) **MONOVALENT ASYMMETRIC TANDEM FAB BISPECIFIC ANTIBODIES**
MONOVALENTE ASYMMETRISCHE TANDEM-FAB-BISPEZIFISCHE ANTIKÖRPER
ANTICORPS BISPÉCIFIQUES DE FAB EN TANDEM MONOVALENTS ASYMÉTRIQUES

(30) Priority: 16.08.2016 WO PCT/CN2016/095546; 09.12.2016 WO PCT/CN2016/109267
(43) Date of publication of application: 26.06.2019
(73) Proprietor: Epimab Biotherapeutics Inc., Pudong New District Shanghai 201112 (CN)
(72) Inventor: WU, Chengbin, Shanghai 201112 (CN)
(74) Representative: Abel & Imray LLP
(86) International application number: PCT/US2017/046875
(87) International publication number: WO 2018/035084

(56) References cited:
- WO-A1-01/77342
- WO-A1-2013/026831
- WO-A1-2015/103072
- WO-A1-2015/134411
- WO-A1-2016/020309
- WO-A1-2016/044224
- US-A1- 2007 065 912
- US-A1- 2012 321 626
- US-A1- 2015 093 387
- US-A1- 2016 120 999
- YIREN XU ET AL: "Production of bispecific antibodies in "knobs-into-holes" using a cell-free expression system", MABS, vol. 7, no. 1, 26 November 2014 (2014-11-26), pages 231-242, XP55372358, US ISSN: 1942-0862, DOI: 10.4161/19420862.2015.989013

## Description

### Field of the Invention

The present invention relates to engineered bispecific antibodies, compositions thereof, and methods of making and using such bispecific antibodies.

### Background of the Invention

Efforts over the last fifty years in the engineering of new forms of antibodies have led to the demonstration and availability of a variety of bispecific and multi-specific binding formats. The diversity of formats includes, for example, single chain Fv antibodies (scFv, Huston et al., Proc. Natl. Acad. Sci. USA, 85: 5879-5883 (1988)), tetravalent IgG-scFv fusions (Coloma and Morrison, Nat. Biotechnol., 15: 159-163 (1997)), diabodies (Holliger et al., Proc. Natl. Acad. Sci. USA, 90: 6444-6448 (1993)), tandem scFv molecules (see e.g. Bargou et al., Science 321, 974-977 (2008)), tetravalent IgG-like dual variable domain antibodies ("DVD-Ig", Wu et al., Nat. Biotechnol., 25: 1290-1297 (2007)), tetravalent Fabs-in-tandem immunoglobulins ("FIT-Ig"), (WO 2015/103072, Epimab Biotheraupeutics), bivalent rat/mouse hybrid bispecific IgG (Lindhofer et al., J. Immunol., 155: 219-225 (1995)), and bispecific crossmab binding proteins (see, e.g., WO 2013/026831 (Roche Glycart AG); WO 2014/167022 (Engmab AG)). Of particular interest for possible use in treating disease, has been the design and production of various engineered bispecific antibodies that can bind two different epitopes or antigens, thereby obviating the need for combination therapies. See, for example, reviews in Spiess et al., Molec. Immunol., 67: 95-106 (2015), Riethmüller, Cancer Immun., 12: 12-18 (2012), Kontermann, Acta Pharmacologica Sinica, 26 (1): 1-9 (2005), Marvin et al., Acta Pharmacologica Sinica, 26(6): 649-658 (2005).

There is a growing interest in the development of bispecific antibodies for treating various cancers. Of particular interest is the potential use of bispecific antibodies to retarget T cells to kill various tumor cells. In an example of such a "T cell retargeting" approach, a bispecific antibody may be designed that binds to a surface antigen on a target cancer cell and also to an activating component of the T cell receptor (TCR) complex on an immature T cell, such as CD3. The simultaneous binding of the bispecific antibody to both cell types provides a temporary association (cell to cell synapse) between target cell and T cell leading to activation of cytotoxic T lymphocytes (CTLs) that attack the targeted cancer cells. Hence, the T cells have been artificially re-targeted to specific target cancer cells independently of peptide antigen presentation by the target cell or the specificity of the T cell as normally required for MHC-restricted activation of CTLs. In this context, it is especially important that CTLs are only activated when a target cell is presenting the bispecific antibody to them, i.e., when the immunological synapse is mimicked, and not simply upon binding of the antibody to the T cell antigen.

A bispecific antibody format that has continued to be of interest for retargeting T cells to tumor cells is the "Bispecific T cell Engager" or "BiTE" antibody, for example, comprising two scFv antibodies linked by a standard glycine-serine (G₄S) linker in which one scFv provides a binding site for a tumor antigen (such as the 17-1A tumor antigen) and the other scFv provides a binding site for the CD3 antigen on T cells (Mack et al., Proc. Natl. Acad. Sci. USA, 92: 7021-7025 (1995)). An anti-CD3 × anti-CD19 BiTE antibody, blinatumomab, has been approved by the United States Food and Drug Administration (FDA) for the treatment of a rare form of a B cell acute lymphoblastic leukemia (ALL). Other bispecific antibody formats that have been investigated for possible use in retargeting T cells to attach cancer cells are tetravalent tandem diabodies ("TandAb," Kipriyanov et al., J. Mol. Biol., 293: 41-56 (1999); Arndt et al., Blood, 94: 2562-2568 (1999)) and dual affinity retargeting protein ("DART", Johnson et al., J. Mol. Biol., 399: 436-449 (2010)). Bispecific antibodies have also been studied for use in retargeting cytotoxic effector cells such as NK cell and macrophage to attack tumor cells (for example, Weiner et al., Cancer Res., 55: 4586-4593 (1995).

Currently, it is not clear what is the most preferred approach to use for retargeting T cells or other cells to attack cancer cells in a treatment of a human subject. For example, activation of T cells can set off an intense and sustained release of powerful cytokines. Such a "cytokine storm" can have deleterious effects not only on local tissue but also systemically in a patient. Accordingly, methods of retargeting of T cells or other cells to treat a cancer may comprise one or more steps carried out *in vitro, ex vivo,* or *in vivo.*

Many bispecific formats, such as BiTE, diabody, DART, and TandAb, use a single chain format to link different variable domains via peptide linkers to achieve bispecificity. Since these formats do not contain an Fc region, they normally have very short *in vivo* half-lives and are physically unstable (Spiess et al. (2015), *op. cit*.)*.* Typically, Fc-containing bispecific formats are designed to increase half-life and may also provide Fc effector function. A number of such Fc-containing bispecific formats employ the knobs-into-holes ("KiH") technology (Ridgway et al, Protein Eng., 9: 617-621 (1996)) to improve assembly and stability of the Fc region, as well as to promote heterodimerization between the CH3 domains of heavy chains from two different antibodies, leading to a bilaterally heterogeneous, bispecific antibody, although some of the formats still may have other issues, such as susceptibility to light chain mispairing. Light chain mispairing can lead to inefficient production of the intended format. Accordingly, a variety of formats have been designed in an effort to address this problem.

WO2013026831 relates to bispecific antigen binding molecules, polynucleotides encoding such molecules, vectors and host cells comprising such polynucleotides, methods for producing the bispecific antigen binding molecules and methods of using the bispecific antigen binding molecules in the treatment of disease.

WO2016020309 relates to bispecific antigen binding molecules for T cell activation and redirection to specific target cells, polynucleotides encoding such bispecific antigen binding molecules, vectors and host cells comprising such polynucleotides, methods for producing the bispecific antigen binding molecules and methods of using the bispecific antigen binding molecules in the treatment of disease.

As is evident from the discussion above, there is a wide variety of bispecific antibody formats that have been designed and studied as possible formats for developing new therapeutic antibodies. Yet, to date, no one format has emerged as providing the comprehensive set of properties that would lend itself to the development of new therapeutic antibodies for treating most diseases. Given the increasing number of possible applications of bispecific binding proteins, and the varied results associated with currently available formats, there remains a need for improved formats that can be engineered to address the particular challenges associated with developing antibodies for treating specific diseases.

### Summary

The present invention, which is defined in the claims, addresses the above need by providing tandemly linked Fab-based bispecific antibodies that are engineered to bind two different epitopes, whether present on the same target antigen or present on two different target antigens. A bispecific antibody according to the invention, as claimed herein, has two Fab units in which each Fab unit binds only one of the epitopes or antigens bound by the antibody and is referred to as a "monovalent asymmetric tandem Fab bispecific antibody" or "MAT-Fab bispecific antibody" or, simply, a "MAT-Fab antibody". A MAT-Fab bispecific antibody of the invention, as claimed herein, is monovalent (one binding site) for each of the two different epitopes or two different antigens bound by the MAT-Fab antibody. A MAT-Fab bispecific antibody according to the invention, as claimed herein, is a tetrameric protein comprising: a "heavy polypeptide chain" (or "MAT-Fab heavy chain"), an "Fc polypeptide chain" (or "MAT-Fab Fc chain"), and two different light chains ("MAT-Fab first light chain" and "MAT-Fab second light chain").

A Fab fragment of an immunoglobulin is composed of two components that covalently associate to form an antibody binding site. The two components are each a variable domain-constant domain chain (VH-CH1 or VL-CL), and therefore each V-C chain of a Fab may be described as one "half" of a Fab binding unit. The heavy chain of the MAT-Fab antibody comprises half of a first and half of a second Fab unit (V-C) fused in tandem, followed by an Fc region comprising a hinge region, CH2 domain, and a C-terminal CH3 domain. The MAT-Fab Fc chain comprises an amino-terminal hinge region, which is linked to a CH2 domain, which in turn is linked to a carboxyl terminal CH3 domain in the same order as found in a naturally occurring IgG molecule. The Fc chain of a MAT-Fab bispecific antibody according to the invention does not, however, contain any portion of the Fab units or any other functional domain attached to its amino terminal hinge region or its carboxyl terminal CH3 domain that is essential for formation of the functional Fab binding units. Each of the two MAT-Fab light chains provides the other half (V-C) of each Fab binding unit. The MAT-Fab light chains and heavy chain are designed so that each light chain associates with its corresponding V-C on the heavy chain to form the intended Fab binding units and to prevent interfering mispairing of light chains to the wrong V-C on the heavy chain. Since the MAT-Fab Fc chain is desired to dimerize with the Fc region of the MAT-Fab heavy chain, it is preferred that the MAT-Fab Fc chain will be essentially identical to the corresponding portion of the MAT-Fab heavy chain except for knobs-into-holes (KiH) mutations designed to promote preferential pairing of the CH3 domains of the heavy chain and the MAT-Fab Fc chain over homodimerization of two MAT-Fab heavy chains or two MAT-Fab Fc chains. Optionally, the CH3 domains of the MAT-Fab heavy and Fc chains may be further advantageously modified by introduction of a cysteine residue, to promote additional disulfide bond formation, or by introduction of one or more salt bridges, such additions leading to improved stability of the heterodimer. A salt bridge comprises a hydrogen bond and an electrostatic interaction such as can occur between glutamate and lysine residues.

Assembly of a functional MAT-Fab bispecific antibody of the invention is achieved by association of each MAT-Fab light chain to its corresponding half Fab segment on the heavy chain to form a complete Fab binding unit and heterodimerization of the Fc domain of the heavy chain with the Fc domain of the MAT-Fab Fc chain. Heterodimerization of the Fc domain of the heavy chain with the Fc domain of the Fc chain is directed and favored using one or more known mutations of the "knobs-into-holes" ("KiH") technology in which the CH3 domain of one Fc region on one chain is mutated to form a protruding structural knob that disfavors homodimerization with identical knob-containing chains. The CH3 domain of the other Fc region of the other chain is mutated to form a structural hole that efficiently pairs with the CH3 domain having a mutation that provides a structural knob while also disfavoring homodimerization with identical hole-containing chains (Ridgway et al., Protein Eng., 9: 617-621 (1996); Atwell et al., J. Mol. Biol., 270: 26-35 (1997)). Thus, assembly of the four polypeptide chains provides a bispecific antibody that is monovalent for each epitope or antigen and structurally asymmetric in that all Fab units are formed by light chains associating with the single heavy chain.

The invention provides a monovalent asymmetric tandem Fab bispecific antibody ("MAT-Fab antibody", "MAT-Fab") that comprises four polypeptide chains (a), (b), (c) and (d):
(a) a heavy polypeptide chain ("heavy chain"), wherein said heavy chain comprises (from amino to carboxyl terminus): VL_{A}-CL-VH_{B}-CH1-hinge-CH2-CH3m1, wherein:
   VL_{A} is a human immunoglobulin light chain variable domain that is linked (fused) directly to CL, which is a human immunoglobulin light chain constant domain, wherein VL_{A}-CL is one half of a first Fab binding unit (recognizing antigen or epitope "A") and is linked (fused) directly to VH_{B}, wherein VHa is a human immunoglobulin heavy chain variable domain that is linked (fused) directly to CH1, which is a human immunoglobulin heavy chain CH1 constant domain, wherein VH_{B}-CH1 is one half of a second Fab binding unit (recognizing antigen or epitope "B"), and wherein VH_{B}-CH1 is linked (fused) directly to a hinge-CH2, wherein hinge-CH2 is the hinge-CH2 region of an immunoglobulin heavy chain and wherein the hinge-CH2 is linked (fused) directly to CH3m1, which is a first human immunoglobulin heavy chain CH3 constant domain that has been mutated with one or more knobs-into-holes (KiH) mutations to form a structural knob or structural hole in said CH3m1 constant domain, wherein A and B are different antigens or different epitopes;
(b) a first light chain comprising VH_{A}-CH1, wherein VH_{A} is a human immunoglobulin heavy chain variable domain that is linked (fused) directly to CH1, which is a human immunoglobulin heavy chain CH1 constant domain, and wherein VH_{A}-CH1 is the other half of said first Fab binding unit;
(c) a second light chain comprising VL_{B}-CL, wherein VL_{B} is a human immunoglobulin light chain variable domain that is linked (fused) directly to CL, which is a human immunoglobulin light chain constant domain, and wherein VL_{B}-CL is the other half of said second Fab binding unit; and
(d) an Fc chain comprising hinge-CH2-CH3m2, wherein hinge-CH2 is the hinge-CH2 region of an immunoglobulin heavy chain and wherein the hinge-CH2 is linked to CH3m2, which is a second human immunoglobulin heavy chain CH3 constant domain that has been mutated with one or more knobs-into-holes (KiH) mutations to form a structural knob or structural hole in said CH3m2 constant domain;
with the proviso that:
when the CH3m1 domain of the heavy chain has been mutated to form a structural knob, then the CH3m2 domain of the Fc chain has been mutated to form a structural hole to favor pairing of the CH3m1 domain with the CH3m2 domain; and
when the CH3m1 domain of the heavy chain has been mutated to form a structural hole, then the CH3m2 domain of the Fc chain has been mutated to form a structural knob to favor pairing of the CH3m 1 domain with the CH3m2 domain; and
optionally (with or without), comprising a mutation in both the CH3m1 domain of the heavy chain and the CH3m2 domain of the Fc chain to introduce a cysteine residue to favor disulfide bond formation in pairing the CH3m1 domain with the CH3m2 domain.

A further option is to engineer one or more salt bridges between the CH3m 1 and CH3m2 domains, by mutating either or both domains such that a residue in one of the domains is able to hydrogen bond and electrostatically interact (bond) with a residue in the other domain. For example, but not limited to, a salt bridge may be introduced by changing (mutating) an amino acid residue in the CH3m 1 domain to a glutamate or aspartate residue and changing (mutating) a residue in the CH3m2 domain to a lysine or arginine residue such that the glutamate or aspartate residue in the CH3m1 domain can hydrogen bond and electrostatically interact with the lysine or arginine residue in the CH3m2 domain.

The aforementioned complementary CH3 domain mutations made in the CH3m1 domain of the heavy chain and in the CH3m2 domain of the Fc chain favor heterodimer formation over homodimerization, that is, the respective CH3 domain mutations are designed to promote preferential pairing of the MAT-Fab Fc chain and the MAT-Fab heavy chain over homodimerization of two Fc chains or two heavy chains.

A feature of the structure of a MAT-Fab bispecific antibody described above is that all adjacent immunoglobulin heavy and light chain variable and constant domains are linked directly to one another without an intervening amino acid or peptide linker. Such direct linking of adjacent immunoglobulin domains (also described as having one domain "fused directly" to the adjacent domain) eliminates potentially immunogenic sites that could be formed by introducing one or more intervening amino acids, creating a peptide segment that would be heterogeneous or "foreign" to a given subject and would be recognized as such by the subject's immune system. Contrary to the general understanding in the field of antibody engineering and production, the absence of linkers between the CL domain and the VH_{B} domain on the MAT-Fab heavy chain, and therefore between the tandem Fab binding units, does not adversely affect the binding activity of either of the Fab binding units in the MAT-Fab antibody.

According to the structure of a MAT-Fab bispecific antibody described above, the CH3m1 domain of the heavy chain or the CH3m2 domain of the Fc chain comprises a knobs-into-holes (KiH) mutation to form a structural knob to favor pairing of one of the CH3 domains (i.e., CH3m1 or CH3m2) comprising the structural knob with the other CH3 domain (i.e., CH3m2 or CH3m1) that comprises a structural hole. Preferably, the one or more mutations is made to form a structural knob in the CH3m 1 domain of the heavy chain for pairing with a CH3m2 domain that comprises a complementary structural hole. Examples of mutations that change an amino acid residue to form a structural knob in the CH3 domain of a MAT-Fab antibody described herein include, but are not limited to, a change of a threonine residue to a tyrosine residue or a change of a threonine residue to a tryptophan residue. Examples of particular mutations that change an amino acid residue to form a structural knob in a CH3 domain of a MAT-Fab antibody described herein include, but are not limited to, a change from a threonine366 residue to a tyrosine residue (T366Y) and a change of a threonine366 residue to a tryptophan residue (T366W). Ridgway et al., Protein Eng., 9: 617-621 (1996); Atwell et al., J. Mol. Biol., 270: 26-35 (1997).

According to the structure of a MAT-Fab bispecific antibody described above, the CH3m1 domain of the heavy chain or the CH3m2 domain of the Fc chain comprises a knobs-into-holes (KiH) mutation to form a structural hole to favor pairing of one of the CH3 domains (i.e., CH3m1 or CH3m2) comprising a structural hole with the other CH3 domain (i.e., CH3m2 or CH3m1) comprising a structural knob. Preferably, the one or more mutations is made to form a structural hole in the CH3m2 domain of the Fc polypeptide chain for pairing with a CH3m1 that comprises a structural knob. Examples of mutations that change one or more residues to form a structural hole in a CH3 domain of a MAT-Fab bispecific antibody described herein include, but are not limited to, a change of a tyrosine residue to a threonine residue and a combination of a change of a threonine residue to a serine residue, a change of a leucine residue to an alanine residue, and a change of a tyrosine residue to a valine residue. A preferred mutation to form a structural hole in a CH3 domain of a MAT-Fab antibody of the invention is a change of a tyrosine407 residue to a threonine residue (Y407T). Ridgway et al., Protein Eng., 9: 617-621 (1996). A preferred combination of mutations to form a structural hole in a CH3 domain of a MAT-Fab antibody of the invention comprises a change of a threonine366 residue to a serine residue (T366S), a change of a leucine368 residue to an alanine residue (L368A), and a change of a tyrosine407 residue to a valine residue (Y407V). Atwell et al., J. Mol. Biol., 270: 26-35 (1997).

In another embodiment, a further mutation may be made to provide a cysteine residue, such as a change from a serine residue to a cysteine residue or a change from a tyrosine residue to a cysteine, in the CH3m1 domain of the heavy chain and in the CH3m2 domain of the Fc polypeptide chain to form an additional disulfide linkage when the CH3m1 domain of the heavy chain pairs with the CH3m2 domain of the Fc polypeptide chain of a MAT-Fab bispecific antibody of the invention. A specific non-limiting example of a cysteine insertion is a serine354 to cysteine substitution (S354C) and a tyrosine349 to cysteine substitution (Y349C), in complementary chains. Merchant et al., Nat. Biotechnol., 16: 677-681 (1998).

In a preferred embodiment, a MAT-Fab bispecific antibody of the invention is capable of binding one or two target antigens selected from the group consisting of: cytokines, cell surface proteins, enzymes, and receptors.

In another embodiment, a MAT-Fab bispecific antibody described herein is capable of modulating a biological function of one or two target antigens. More preferably, a MAT-Fab bispecific antibody described herein is capable of inhibiting or neutralizing one or more target antigens.

In an embodiment of the invention, a MAT-Fab bispecific antibody described herein is capable of binding two different cytokines. Such cytokines are selected from the group consisting of: lymphokines, monokines, and polypeptide hormones.

In another embodiment, a MAT-Fab bispecific antibody of the invention is capable of binding at least one target antigen expressed on a surface of a cell. More preferably, a MAT-Fab bispecific antibody of the invention binds two cell surface antigens. The two cell surface antigens may be on the same cell or two cells of the same type. More preferably, however, a MAT-Fab bispecific antibody of the invention binds an antigen expressed on the surface of a first cell and binds a second antigen expressed on the surface of a second cell, wherein the first and second cells are different types of cells. Preferably, a MAT-Fab bispecific antibody described herein binds a first cell surface antigen expressed on an effector cell of the immune system and also binds a second cell surface antigen that is expressed on the surface of a cell that is considered detrimental to an individual and therefore is desired to be eliminated or substantially reduced in population. Effector cells include T cells, NK cells, monocytes, neutrophils, and macrophages. Cells that are or may be considered detrimental to an individual in need of treatment, and therefore, that may be bound by a MAT-Fab antibody described herein include, but are not limited to, cancer cells, auto-reactive cells, and virus-infected cells. Accordingly, in a particularly preferred embodiment, a MAT-Fab bispecific antibody of the invention binds an antigen expressed on the surface of an effector cell and binds an antigen expressed on the surface of a cancer cell, an auto-reactive cell, or a virus-infected cell.

In a preferred embodiment, a MAT-Fab bispecific antibody of the invention binds a pair of target antigens selected from the group of antigen pairs consisting of: CD20 and CD3, CD3 and CD19, CD3 and Fc-gamma-RIIIA, CD3 and TPBG, CD3 and Epha10, CD3 and IL-5Rα, CD3 and TASCTD-2, CD3 and CLEC12A, CD3 and Prominin-1, CD3 and IL-23R, CD3 and ROR1, CD3 and IL-3Rα, CD3 and PSA, CD3 and CD8, CD3 and Glypican 3, CD3 and FAP, CD3 and EphA2, CD3 and ENPP3, CD3 and CD33, CD3 and CD133, CD3 and EpCAM, CD3 and CD19, CD3 and Her2, CD3 and CEA, CD3 and GD2, CD3 and PSMA, CD3 and BCMA, CD3 and A33, CD3 and B7-H3, CD3 and EGFR, CD3 and P-cadherin, CD3 and HMW-MAA, CD3 and TIM-3, CD3 and CD38, CD3 and TAG-72, CD3 and SSTR, CD3 and FRA, CD16 and CD30, CD64 and Her2, CD 137 and CD20, CD138 and CD20, CD19 and CD20, CD38 and CD20, CD20 and CD22, CD40 and CD20, CD47 and CD20, CD 137 and EGFR, CD137 and Her-2, CD 137 and PD-1, CD 137 and PDL-1, PD-1 and PD-L1, VEGF and PD-L1, Lag-3 and TIM- 3, OX40 and PD-1, TIM-3 and PD-1, TIM-3 and PDL-1, EGFR and DLL-4, VEGF and EGFR, HGF and VEGF, a first epitope of VEGF and a different second epitope of VEGF, VEGF and Ang2, EGFR and cMet, PDGF and VEGF, VEGF and DLL-4, OX40 and PD-L1, ICOS and PD-1, ICOS and PD-L1, Lag-3 and PD-1, Lag-3 and PD-L1, Lag-3 and CTLA-4, ICOS and CTLA- 4, CD138 and CD40, CD38 and CD138, CD38 and CD40, CD-8 and IL-6, CSPGs and RGM A, CTLA-4 and BTN02, CTLA-4 and PD-1, IGFl and IGF2, IGFl/2 and Erb2B, IGF-IR and EGFR, EGFR and CD13, IGF-IR and ErbB3, EGFR-2 and IGFR, a first epitope of Her2 and a second different epitope of Her2, Factor IXa and Met, Factor X and Met, VEGFR-2 and Met, VEGF-A and Angiopoietin-2 (Ang-2), IL-12 and TWEAK, IL-13 and IL-lβ, MAG and RGM A, NgR and RGM A, NogoA and RGM A, OMGp and RGM A, PDL-1 and CTLA-4, PD-1 and TIM-3, RGM A and RGM B, Te38 and TNFα, TNFα and Blys, TNFα and CD22, TNFα and a CTLA-4, TNFα and GP130, TNFα and IL-12p40, and TNFα and RANK ligand.

In another embodiment, a MAT-Fab bispecific antibody described herein binds CD3 on an effector cell. Examples of effector cells include, but are not limited to, T cells, natural killer (NK) cells, monocytes, neutrophils, and macrophages.

In another embodiment, a MAT-Fab bispecific antibody of the invention binds a surface antigen expressed on an effector cell. Preferably, the surface antigen is selected from the group consisting of: CD3, CD16 (also referred to as "FcyRIII"), and CD64 (also referred to as "FcyRI"). More preferably, a MAT-Fab antibody binds CD3 as expressed on a T cell, CD16 as expressed on a natural killer (NK) cell, or a CD64 as expressed on a macrophage, neutrophil, or monocyte.

In another embodiment, a MAT-Fab bispecific antibody of the invention binds a surface antigen that is a tumor-associated antigen. Preferred MAT-Fab embodiments may recognize tumor-associated antigens such as those selected from, without limitation, the group consisting of: CD 19, CD20, human epidermal growth factor receptor 2 ("Her2"), carcinoembryonic antigen ("CEA"), epithelial cell adhesion molecule ("EpCAM"), and receptor tyrosine kinase-like orphan receptor 1 (ROR1).

In another embodiment, a MAT-Fab bispecific antibody of the invention binds an antigen on an effector cell that will activate the effector cell and binds a cell surface antigen on a malignant B cell. Preferably, a MAT-Fab bispecific antibody of the invention binds an antigen on an effector cell that will activate the effector cell and binds a cell surface antigen on a malignant B cell of a cancer disorder selected from the group consisting of: acute lymphoblastic leukemia, Hodgkin's lymphoma, non-Hodgkin's lymphoma (NHL), precursor B cell lymphoblastic leukemia/lymphoma, mature B cell neoplasms, B cell chronic lymphocytic leukemialsmall lymphocytic lymphoma, B cell prolymphocytic leukemia, lymphoplasmacytic lymphoma, mantle cell lymphoma, follicular lymphoma, cutaneous follicle center lymphoma, marginal zone B cell lymphoma, hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma.

In a preferred embodiment, a MAT-Fab bispecific antibody binds to the target antigens CD3 and CD20.

In another embodiment, a MAT-Fab bispecific antibody described herein binds a surface antigen expressed on an effector cell and a tumor-associated antigen expressed on a tumor cell. In a preferred embodiment, a MAT-Fab bispecific antibody described herein binds CD3 on a T cell and CD20 on a malignant B cell. More preferably, the MAT-Fab bispecific antibody binds CD20 at its outer (N-terminal) Fab binding unit and binds CD3 at its inner (C-proximal) Fab binding unit.

In a preferred embodiment, a MAT-Fab bispecific antibody binds CD20 and CD3 and comprises four polypeptide chains that comprise the amino acid sequences in Tables 1-4 or the amino acid sequences in Tables 5-8.

In yet another embodiment, a MAT-Fab bispecific antibody of the invention is capable of binding one or two cytokines, cytokine-related proteins, or cytokine receptors.

In another embodiment, a MAT-Fab bispecific antibody of the invention is capable of binding one or more chemokines, chemokine receptors, and chemokine-related proteins.

In another embodiment, a MAT-Fab bispecific antibody of the invention is capable of binding CD3 on T cells as well as an antigen or epitope derived from viral envelope proteins that are presented on the surface of virus infected cells, such as HIV-infected CD4+ T cells.

In another embodiment, a MAT-Fab bispecific antibody of the invention is also capable of binding receptors, including lymphokine receptors, monokine receptors, and polypeptide hormone receptors.

In another embodiment, a MAT-Fab bispecific antibody described above is glycosylated. Preferably, the glycosylation is a human glycosylation pattern.

In another embodiment, the invention provides an isolated polynucleotide or isolated polynucleotides encoding all four of the polypeptide chains of a MAT-Fab bispecific antibody described herein. In a preferred embodiment, the one or more nucleic acids encode four polypeptide chains that comprise the amino acid sequences shown in Tables 1-4 or the amino acid sequences shown in Tables 5-8, below.

In another embodiment, the invention provides a vector comprising one or more isolated polynucleotide or isolated polynucleotides encoding all four of the polypeptides of a MAT-Fab bispecific antibody described herein. A vector may be an autonomously replicating vector or a vector that incorporates the isolated nucleic acid that is present in the vector into a host cell genome. An isolated polynucleotide or polynucleotides encoding all four polypeptide chains of a MAT-Fab antibody may also be inserted into a vector for carrying out various genetic analyses, for expressing a MAT-Fab antibody, or for characterizing or improving one or more properties of a MAT-Fab antibody described herein.

In another embodiment, a vector according to the invention may be used to replicate the isolated polynucleotide or isolated polynucleotides to provide more polynucleotide or polynucleotides encoding the polypeptides of a MAT-Fab bispecific antibody described herein.

In another embodiment, a vector according to the invention may be used to express an isolated polynucleotide or isolated polynucleotides encoding all four of the polypeptide chains of a MAT-Fab bispecific antibody described herein. Preferred vectors for cloning and expressing nucleic acids described herein include, but are not limited to, pcDNA, pTT (Durocher et al, Nucleic Acids Res., 30(2e9): 1-9 (2002)), pTT3 (pTT with additional multiple cloning sites), pEFBOS (Mizushima and Nagata, Nucleic Acids Res., 18(17): 5322 (1990)), pBV, pJV, pcDNA3.1 TOPO, pEF6 TOPO and pBJ.

The invention also provides an isolated host cell comprising a vector described above. Such an isolated host cell comprising a vector described herein may be an isolated prokaryotic cell or an isolated eukaryotic cell.

In an embodiment of the invention, an isolated prokaryotic host cell comprising a vector described herein is a bacterial host cell. The bacterial host cell may be a Gram positive, Gram negative, or Gram variable bacterial cell. Preferably, the bacterial host cell comprising a vector described herein is a Gram negative bacterium. Even more preferably, a bacterial host cell comprising a vector described herein is an *Escherichia coli* cell.

In an embodiment of the invention, an isolated host cell comprising a vector described herein is a eukaryotic host cell. Preferred isolated eukaryotic host cells comprising a vector described herein may include, without limitation, a mammalian host cell, an insect host cell, a plant host cell, a fungal host cell, a eukaryotic algal host cell, a nematode host cell, a protozoan host cell, and a fish host cell. Preferably, an isolated mammalian host cell comprising a vector described herein is selected from the group consisting of: a Chinese Hamster Ovary (CHO) cell, a COS cell, a Vero cell, an SP2/0 cell, an NS/0 myeloma cell, a human embryonic kidney (HEK293) cell, a baby hamster kidney (BHK) cell, a HeLa cell, a human B cell, a CV-1/EBNA cell, an L cell, a 3T3 cell, an HEPG2 cell, a PerC6 cell, and an MDCK cell. Preferred isolated fungal host cells comprising a vector described herein are selected from the group consisting of: *Aspergillus, Neurospora, Saccharomyces, Pichia, Hansenula, Schizosaccharomyces, Kluyveromyces, Yarrowia,* and *Candida.* More preferably, a *Saccharomyces* host cell comprising a vector described herein is a *Saccharomyces cerevisiae* cell.

Also provided is a method of producing a MAT-Fab bispecific antibody described herein comprising culturing an isolated host cell comprising a vector that comprises nucleic acid encoding all four polypeptide chains of a MAT-Fab bispecific antibody molecule under conditions sufficient to produce a MAT-Fab bispecific antibody.

Another aspect of the invention is a MAT-Fab bispecific antibody produced by a method described above.

A MAT-Fab bispecific antibody described herein may be conjugated to another compound, for example, within or at the C-terminus of either or both of the paired CH3m 1 and CH3m2 domains in a manner similar to that of other conjugated antibodies. Such compounds that may be conjugated to a MAT-Fab bispecific antibody include, but are not limited to, a cytotoxic agent, an imaging agent, and a therapeutic agent. Preferred imaging agents that may be conjugated to a MAT-Fab bispecific antibody include, without limitation, a radiolabel, an enzyme, a fluorescent label, a luminescent label, a bioluminescent label, a magnetic label, biotin, streptavidin, and avidin. Radiolabels that may be conjugated to a MAT-Fab bispecific antibody described herein include, but are not limited to, ³H, ¹⁴C, ³⁵S, ⁹⁰Y, ⁹⁹Tc, ¹¹¹In, ¹³¹I, ¹⁷⁷Lu, ¹⁶⁶Ho, and ¹⁵³Sm. Preferred cytotoxic or therapeutic compounds that may be conjugated to a MAT-Fab bispecific antibody described herein include, but are not limited to, an anti-metabolite, an alkylating agent, an antibiotic, a growth factor, a cytokine, an anti-angiogenic agent, an anti-mitotic agent, an anthracycline, a toxin, and an apoptotic agent.

In another embodiment, a MAT-Fab bispecific antibody described herein may be a crystallized MAT-Fab antibody that retains binding activity for the epitopes or the antigens bound by the non-crystallized MAT-Fab antibody. Such crystallized MAT-Fab bispecific antibody may also provide carrier-free controlled release of the MAT-Fab when administered to an individual. A crystallized MAT-Fab bispecific antibody may also exhibit a greater *in vivo* half-life when administered to an individual compared to the non-crystallized form.

An embodiment of the invention provides a composition for the release of a crystallized MAT-Fab bispecific antibody wherein the composition comprises a crystallized MAT-Fab bispecific antibody as described herein, an excipient ingredient, and at least one polymeric carrier. Preferably the excipient ingredient is selected from the group consisting of: albumin, sucrose, trehalose, lactitol, gelatin, hydroxypropyl-β-cyclodextrin, methoxypolyethylene glycol and polyethylene glycol. Preferably the polymeric carrier is a polymer selected from one or more of the group consisting of: poly(acrylic acid), poly(cyanoacrylates), poly(amino acids), poly(anhydrides), poly(depsipeptide), poly(esters), poly(lactic acid), poly(lactic-co-glycolic acid) or PLGA, poly(b-hydroxybutryate), poly(caprolactone), poly(dioxanone); polyethylene glycol), poly((hydroxypropyl) methacrylamide, poly[(organo)phosphazene], poly(ortho esters), poly(vinyl alcohol), poly(vinylpyrrolidone), maleic anhydride/alkyl vinyl ether copolymers, pluronic polyols, albumin, alginate, cellulose and cellulose derivatives, collagen, fibrin, gelatin, hyaluronic acid, oligosaccharides, glycaminoglycans, sulfated polysaccharides, blends thereof, and copolymers thereof.

Another embodiment provides a composition comprising a crystallized MAT-Fab bispecific antibody as claimed herein for use as a medicament.

In another embodiment, the invention provides a MAT-Fab bispecific antibody as claimed herein for use in a method of treating a disease or disorder in an individual, wherein said MAT-Fab bispecific antibody binds one or two epitopes or antigens that are considered to be detrimental to the individual wherein binding of said one or two epitopes or antigens by the MAT-Fab bispecific antibody provides a treatment for the disease or disorder.

A MAT-Fab bispecific antibody described herein is particularly useful in a method of treating a disorder comprising retargeting (or "recruiting") of effector cells (such as T cells, NK cells, monocytes, neutrophils, macrophages) to attack specific target cells that are considered detrimental to a human subject and, therefore, where it is desirable to eliminate or substantially reduce the population of the detrimental target cells. Preferred examples of such detrimental target cells are tumor cells, for example, blood (including lymph) tumor cells and solid tumor cells, (Satta et al., Future Oncol., 9(4): 527-539 (2013)); autoimmune disease cells, such as auto-reactive B cells (Zocher et al., Mol. Immunol., 41(5): 511-518 (2004)); as well as virus infected cells (such as HIV-infected CD4+ T cells (Sung et al., J. Clin. Invest., 125(11): 4077-4090 (2015)). In a retargeting method of the invention, a MAT-Fab antibody binds an antigen expressed on the surface of an effector cell and an antigen expressed on the surface of a target cell that is detrimental to a human subject, wherein binding of the MAT-Fab antibody to the antigen on the effector cell and to the antigen on the detrimental target cell mediates a cell-cell interaction that is desirable or beneficial, for example, wherein the MAT-Fab bispecific binding activates the effector cell to attack the detrimental target cell. The simultaneous binding of a MAT-Fab bispecific antibody described herein to a single target antigen on an effector cell and to a single target antigen on a detrimental target cell can activate the effector cell to attack the target cell advantageously without also eliciting a massive and undesirable release of cytokines ("cytokine storm") that can otherwise occur when effector cell antigens are dimerized using antibodies that bind two or more effector cell antigens simultaneously (i.e., cross-linking effector cell antigens).

Accordingly, the invention provides a MAT-Fab bispecific antibody as claimed herein that binds an antigen on an effector cell and that binds a disorder-associated antigen expressed on a target cell that is detrimental to a human subject for use in therapy of a disorder in said human subject, wherein the binding of the MAT-Fab bispecific antibody to both effector cell and the detrimental target cell causes a therapeutically beneficial cell-cell interaction between the effector and target cells. For treating many disorders, such beneficial cell-cell interaction will comprise activation of the effector cell to attack the detrimental target cell. In other situations, the beneficial effect may be to opsonize and/or clear one or both of the bound targeted cells.

Preferably, a MAT-Fab bispecific antibody as claimed herein for use in a method of the invention for retargeting an effector cell to attack a detrimental target cell comprises contacting the effector cell and the detrimental target cell with a MAT-Fab bispecific antibody described herein that binds an antigen expressed on an effector cell selected from the group consisting of: CD3, CD 16 (also referred to as "FcyRIII"), and CD64 (also referred to as "FcyRI"). More preferably, the MAT-Fab antibody binds CD3 as expressed on a T cell, CD16 as expressed on a natural killer (NK) cell, or a CD64 as expressed on a macrophage, neutrophil, or monocyte.

In an embodiment, the invention provides a MAT-Fab bispecific antibody as claimed herein for use in a method of treating a tumor in a human subject wherein said MAT-Fab antibody binds an antigen on an effector cell and also binds an antigen on a target tumor cell, wherein binding of the MAT-Fab antibody to the effector cell and the target tumor cell activates the effector cell to attack the tumor cell. Preferably, the antigen on the effector cell is CD3 as expressed on a T cell.

In a preferred embodiment, a MAT-Fab bispecific antibody as claimed herein for use in a method of treating a cancer characterized by tumor cells in a human subject comprises retargeting an effector cell to attack a target tumor cell comprising the step of contacting the effector cell and the target tumor cell with a MAT-Fab bispecific antibody described herein that binds an antigen on the effector cell and an antigen on the target tumor cell, wherein the antigen on the target tumor cell is a tumor-associated antigen selected from the group consisting of: CD 19, CD20, human epidermal growth factor receptor 2 ("Her2"), carcinoembryonic antigen ("CEA"), epithelial cell adhesion molecule (EpCAM), and receptor tyrosine kinase-like orphan receptor 1 (ROR1).

In another embodiment, the invention provides a MAT-Fab bispecific antibody as claimed herein for use in a method of treating a human subject for a B cell-associated tumor, wherein said MAT-Fab bispecific antibody binds an antigen on an effector T cell that will activate the T cell and that binds an antigen on malignant B cells. Preferably, the MAT-Fab bispecific antibody of the invention binds an antigen on malignant B cells of a cancer disorder selected from the group consisting of: acute lymphoblastic leukemia, Hodgkin's lymphoma, non-Hodgkin's lymphoma (NHL), precursor B cell lymphoblastic leukemia/lymphoma, mature B cell neoplasms, B cell chronic lymphocytic leukemia/small lymphocytic lymphoma, B cell prolymphocytic leukemia, lymphoplasmacytic lymphoma, mantle cell lymphoma, follicular lymphoma, cutaneous follicle center lymphoma, marginal zone B cell lymphoma, hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma.

In a particularly preferred embodiment, the MAT-Fab bispecific antibody as claimed herein for use in a method of treating a human subject for a B cell-associated tumor comprises administering a MAT-Fab bispecific antibody described herein that binds CD3 on a T cell and that binds CD20 on a malignant B cell. More preferably, the MAT-Fab bispecific antibody binds CD20 at its outer (N-terminal) Fab binding unit and binds CD3 at its inner (C-proximal) Fab binding unit.

In another embodiment, a MAT-Fab bispecific antibody as claimed herein for use in a method of treating a disorder according to the invention may comprise bringing a MAT-Fab antibody claimed herein into contact with effector cells and detrimental target cells in an *ex vivo* procedure in which effector cells extracted from a human subject in need of treatment are contacted with the MAT-Fab antibody outside the human subject and, after providing time for binding of the MAT-Fab antibody to the effector cells, the effector cells bound to the MAT-Fab antibody are then administered to the human subject so that complexes of MAT-Fab antibody bound to effector cells can then seek to bind detrimental target cells, such as tumor cells, inside the human subject. In another embodiment, effector cells and tumor cells extracted from a human subject in need of treatment are contacted with the MAT-Fab antibody outside the human subject and, after providing time for binding of the MAT-Fab antibody to the effector cells and tumor cells, the effector and tumor cells bound to MAT-Fab antibody are administered to the human subject.

In another embodiment, a MAT-Fab bispecific antibody as claimed herein may also be engineered to deliver a cytotoxic agent to a detrimental target cell, such as a tumor cell. In this embodiment, one Fab binding unit of a MAT-Fab antibody contains a binding site for a target antigen on a detrimental target cell and the other Fab binding unit contains a binding site for a cytotoxic agent. Such an engineered MAT-Fab antibody of the invention can be mixed with or otherwise contacted with the cytotoxic agent to which it will bind at its engineered Fab binding unit. The MAT-Fab antibody carrying the bound cytotoxic agent can then be brought into contact with the detrimental target cell to deliver the cytotoxic agent to the detrimental target cell. Such a delivering system is particularly effective when the MAT-Fab antibody binds to the detrimental target cell and then is internalized into the cell along with the bound cytotoxic agent so that the cytotoxic agent can be released inside the detrimental target cell.

The invention provides pharmaceutical compositions comprising a MAT-Fab bispecific antibody claimed herein and a pharmaceutically acceptable carrier. A pharmaceutical composition comprising a MAT-Fab bispecific antibody as claimed herein may also comprise an additional agent selected from the group consisting of: a therapeutic agent, an imaging agent, and a cytotoxic agent. Additionally, in accordance with the *ex vivo* methods described herein, a pharmaceutical composition according to the invention may comprise a MAT-Fab bispecific antibody as claimed herein complexed with an effector cell or a cytotoxic agent.

In another embodiment, a preferred pharmaceutical composition comprising a MAT-Fab bispecific antibody claimed herein may further comprise one or more other therapeutically active compounds for treating a disorder. Examples of preferred additional therapeutically active compounds that may be incorporated into a pharmaceutical composition of the invention include, but are not limited to, an antibiotic, an anti-viral compound, an anti-cancer compound (other than the MAT-Fab bispecific antibody), a sedative, a stimulant, a local anesthetic, a corticosteroid, an analgesic, an anti-histamine, a non-steroid anti-inflammatory drug (NSAID), and appropriate combinations thereof.

In another embodiment, a pharmaceutical composition disclosed above may be prepared for administration to an individual by at least one mode selected from the group consisting of: parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal.

In another embodiment, a MAT-Fab bispecific antibody claimed herein may be used in any of a variety of immunodetection assays or purification formats available in the art for detecting, quantitating, or isolating a target antigen or cells expressing a target antigen. Such assays and formats include, but are not limited to, immunoblot assays (for example, a Western blot); immunoaffinity chromatography, for example, wherein a MAT-Fab bispecific antibody is adsorbed or linked to a chromatography resin or bead; immunoprecipitation assays; immunochips; tissue immunohistochemistry assays; flow cytometry (including fluorescence activated cell sorting); sandwich immunoassays; immunochips, wherein a MAT-Fab antibody is immobilized or bound to a substrate; radioimmunoassays (RIAs); enzyme immunoassays (EIAs); enzyme-linked immunosorbent assay (ELISAs); competitive-inhibition immunoassays; fluorescence polarization immunoassay (FPIA); enzyme multiplied immunoassay technique (EMIT); bioluminescence resonance energy transfer (BRET); and homogenous chemiluminescent assays. Methods employing mass spectrometry are provided by the present disclosure and include, but are not limited to MALDI (matrix-assisted laser desorption/ionization) or by SELDI (surface-enhanced laser desorption/ ionization) that comprise a MAT-Fab antibody that binds a target antigen or epitope on an antigen or antigen fragment.

The invention further provides a method for detecting an antigen in a sample (such as, for example, a mixture, composition, solution, or biological sample) comprising contacting the sample with a MAT-Fab bispecific antibody claimed herein that binds a target antigen (or epitope) present in or suspected of being present in the sample. Biological samples that can serve as a sample for an immunodetection assay of the invention include, without limitation, whole blood, plasma, serum, various tissue extracts, tears, saliva, urine, and other bodily fluids.

The MAT-Fab bispecific antibody may be directly or indirectly labeled with a detectable substance to facilitate detection of the bound or unbound MAT-Fab bispecific antibody. In a preferred embodiment, a MAT-Fab bispecific antibody useful in a detection assay is engineered such that one of the Fab binding units binds a compound that generates a detectable signal and the other Fab binding unit binds the target antigen (or epitope) that is present in or suspected as being present in a sample. Suitable detectable substances are available in the art and include, without limitation, various enzymes, prosthetic groups, fluorescent materials, luminescent materials, and radioactive materials. A preferred enzyme useful in an immunodetection assay of the invention is one that can provide a detectable signal when brought into contact with one more reagents. Such enzymes include, but are not limited to, horseradish peroxidase, alkaline phosphatase, β-galactosidase, or acetylcholinesterase. Examples of suitable prosthetic group complexes include, without limitation, streptavidin/biotin and avidin/biotin. Examples of suitable fluorescent materials that may be used in an immunodetection assay of the invention include, but are not limited to, umbelliferone, fluorescein, fluorescein isothiocyanate, rhodamine, dichlorotriazinylamine fluorescein, dansyl chloride, and phycoerythrin. An example of a luminescent material that may be used in an immunodetection assay of the invention is luminol. Examples of suitable radioactive species that may be used in an immunodetection assay of the invention include, but are not limited to, ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, and ¹⁵³Sm.

Owing to the presence of a dimerized Fc region, a MAT-Fab bispecific antibody of the invention may also be labeled in its Fc region in an analogous manner to natural occurring antibody molecules, such as IgG antibodies.

### Brief Description of the Drawings

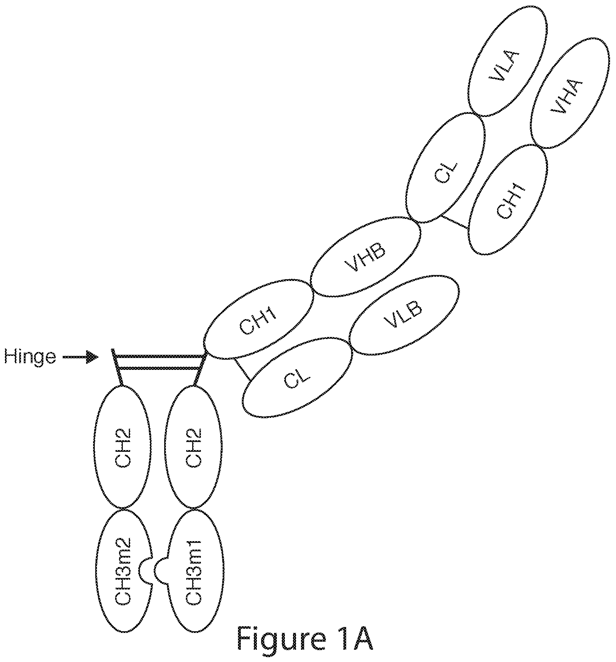

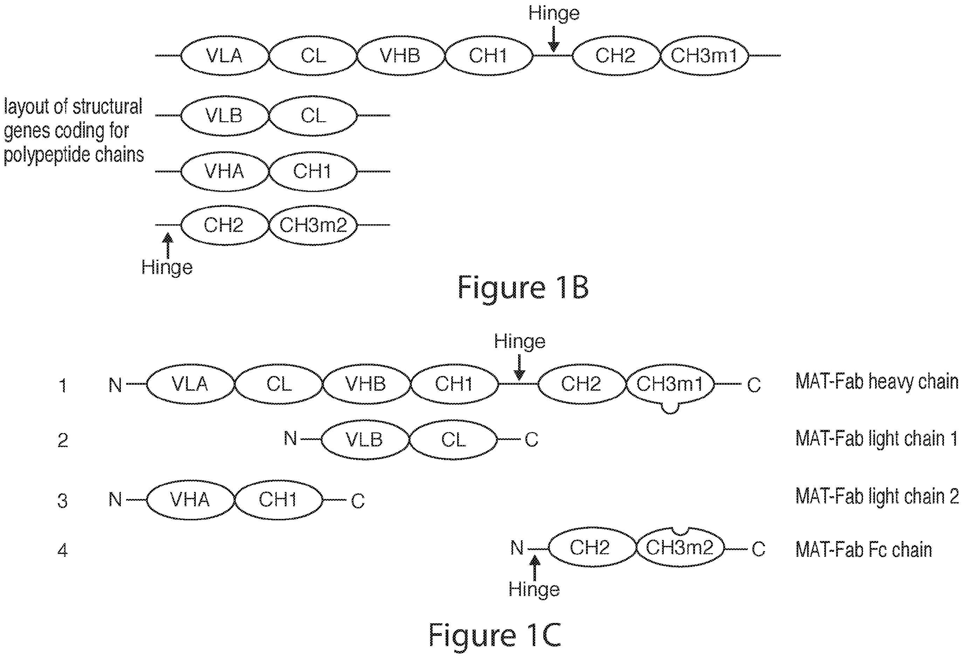

Figure 1A is a diagram showing the domain structure of a MAT-Fab bispecific antibody. Figure 1B is a diagram depicting the arrangement of structural genes inserted in expression vector constructs for the recombinant expression of each of the polypeptide chains that assemble to form a MAT-Fab binding protein of the present invention. Figure 1C is a diagram depicting the four expressed polypeptide chains (1-4) for making up a MAT-Fab binding protein illustrated in Fig. 1A, showing the order of immunoglobulin-like domains from N-terminus to C-terminus for each chain.

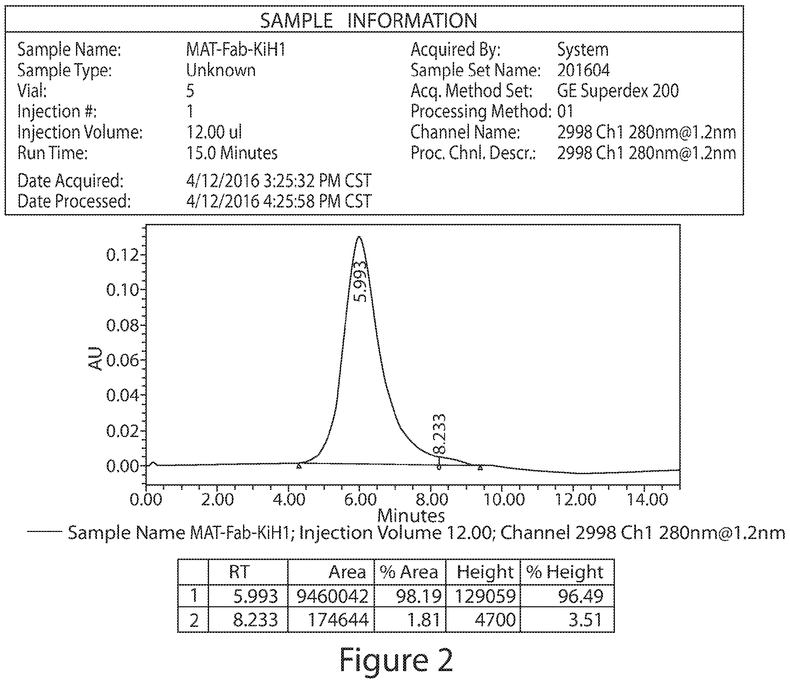

Figure 2 shows a profile and related data from an analysis of the MAT-Fab KiH1 bispecific antibody prepared as described in Example 1 using size exclusion chromatography (SEC). The SEC analysis revealed that 98.19 % of the MAT-Fab KiH1 antibody preparation after a single step purification by Protein A chromatography was present as a single species, indicating homogeneity of the tetrameric protein product.

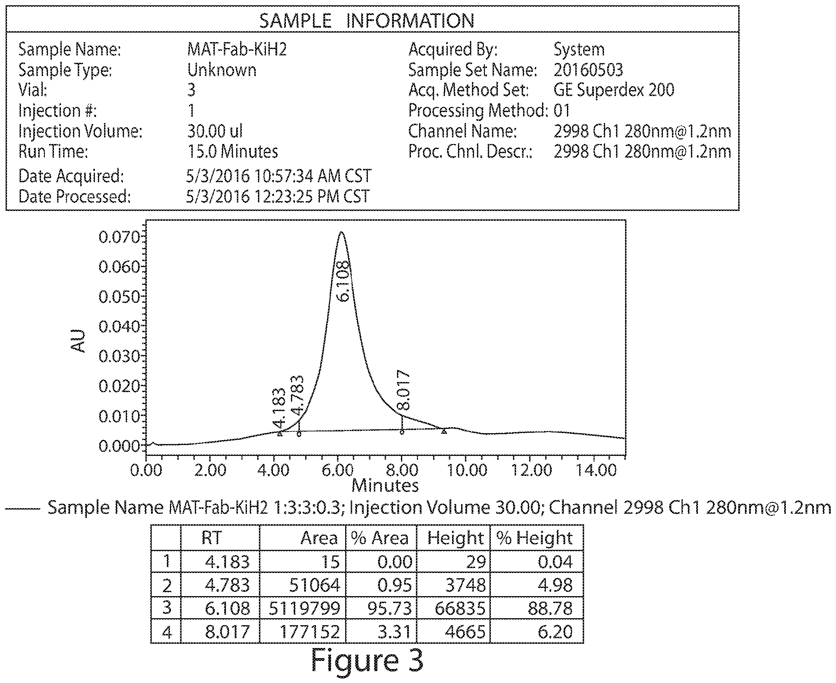

Figure 3 shows a profile and related data from an analysis of the MAT-Fab KiH2 bispecific antibody prepared as described in Example 1 using size exclusion chromatography (SEC). The SEC analysis revealed that 95.7 % of the MAT-Fab KiH2 antibody preparation after a single step purification by Protein A chromatography was present as a single species, indicating homogeneity of the tetrameric protein product.

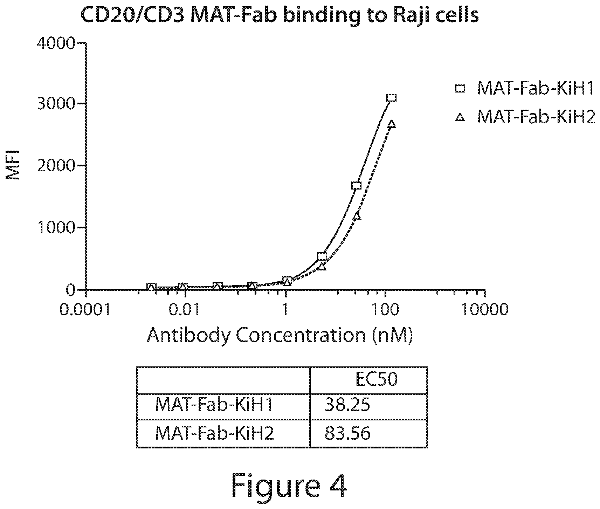

Figure 4 shows the results of an analysis of the ability of the MAT-Fab KiH 1 and MAT-Fab KiH2 bispecific antibodies to bind CD20 expressed on the surface of Raji cells using fluorescence activated cell sorting (FACS) as described in Example 1.

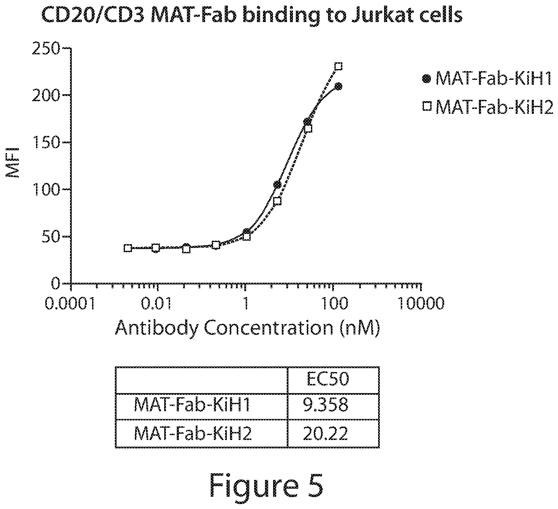

Figure 5 shows the results of an analysis of the ability of the MAT-Fab KiH 1 and MAT-Fab KiH2 bispecific antibodies to bind CD3 as expressed on the surface of Jurkat cells using fluorescence activated cell sorting (FACS) as described in Example 1.

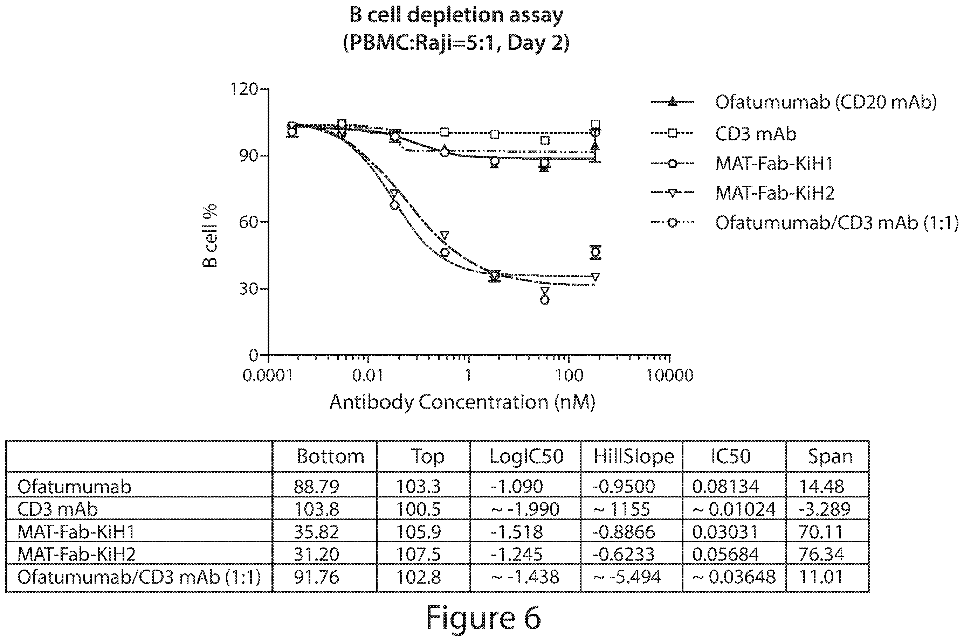

Figure 6 shows the results of an analysis of the ability of the MAT-Fab KiH1 and MAT-Fab KiH2 bispecific antibodies to bind CD20 on Raji cells (B cells) and induce T cell-mediated apoptosis of the B cells on day 2 of a B cell depletion assay. Graph key: "Ofatumumab" is a fully human anti-CD20 monoclonal antibody; "CD3 mAb" is an anti-CD3 monoclonal antibody; MAT-Fab KiH1 and MAT-Fab KiH2 are as described in Example 1; and "Ofatumumab/CD3 mAb" is a mixture of equal portions of the anti-CD20 ofatumumab and the anti-CD3 mAb. Both MAT-Fab bispecific antibodies were able to induce T cell mediated apoptosis of B cells by day 2 of the assay.

### Detailed Description

The technical disclosure set out below may in some respects go beyond the scope of the claims. Elements of the disclosure which do not fall within the scope of the claims are provided for information.

For many immune receptors, cellular activation is accomplished by cross-linking of a monovalent binding interaction. The mechanism of cross-linking is typically mediated by antibody/antigen immune complexes, or via effector cell to target cell engagement. For example, the low affinity activating Fc gamma receptors (FcyRs), such as CD16 (FcyRIIIa) and CD32a (FcyRIIa) that mediate cellular killing, bind monovalently to the antibody Fc region. While monovalent binding does not result in cellular signaling, upon effector cell engagement with the target cell, receptors are cross-linked and clustered on the cell surface, leading to activation. On T cells, CD3 activation occurs when its associated T cell receptor (TCR) engages antigen-loaded major histocompatibility complex (MHC) on antigen-presenting cells in an avid cell-to-cell synapse. Bivalent antibodies targeting CD3 can elicit massive cytokine release (often called a "cytokine storm"), and the consequent toxicity has presented challenges for the development of anti-CD3 antibodies as therapeutic drugs. In contrast, monovalent binding of CD3 in bispecific formats generates much lower levels of T cell activation. For bivalent monospecific antibodies, a consequence of this biology is that bivalent cross-linking of receptors can lead to non-specific activation of an effector cell in the absence of any target cell. Thus, when the therapeutic goal is the co-engagement of an immune receptor, monovalent binding is commonly highly preferred over bivalent binding. This mode is incompatible with the use of typical bivalent antibodies and the majority of multispecific but multivalent antibody formats, such as dual variable domain immunoglobulins (DVD-Igs) and Fabs-in-tandem immunoglobulins (FIT-Igs).

The present invention provides a solution to such problems as described above by providing a bispecific antibody format as claimed herein that comprises knobs-into-holes (KiH) Fc region heterodimerization of a tandem Fab heavy chain with a truncated Fc chain that enables the simultaneous monovalent binding of two different antigens or epitopes. A bispecific antibody of the invention is especially well suited for T cell retargeting as a mechanism for treating cancer.

The present invention provides engineered bispecific antibodies, as claimed herein, that comprise two Fab binding units fused in tandem and in which one Fab binding unit binds an epitope or antigen that is different from the epitope or antigen bound by the other Fab binding unit. Thus, a bispecific antibody according to the invention, as claimed herein, is referred to as "monovalent" with respect to each epitope or antigen that it binds. Moreover, although a bispecific antibody of the invention, as claimed herein, comprises a dimerized immunoglobulin Fc constant region (i.e., dimerized hinge-CH2-CH3), each half of the tandem Fab binding units is present on a single polypeptide arm extending from only one of the dimerized chains of the Fc region (see Fig. 1A). Hence, a bispecific antibody according to the invention, as claimed herein, is "monovalent" with respect to binding sites for each epitope or antigen bound; is "asymmetric" with respect to the location of the Fab units relative to the dimerized Fc region; and has "tandem" Fab binding units linked directly to one another, in N-terminal to C-terminal direction, through a common heavy chain. Accordingly, a bispecific antibody according to the invention, as claimed herein, is also referred to as a "monovalent, asymmetric, tandem Fab bispecific antibody". Alternative, but synonymous terms for a bispecific antibody of the invention are "MAT-Fab bispecific antibody", "MAT-Fab antibody", or simply a "MAT-Fab".

The terms "crystal" and "crystallized" as used herein, refer to an antibody, including a MAT-Fab bispecific antibody of the invention that exists in the form of a crystal. A crystal is one form of the solid state of matter that is distinct from other forms such as the amorphous solid state or the liquid crystalline state. Crystals are composed of regular, repeating, three-dimensional arrays of atoms, ions, molecules (e.g., proteins such as antibodies), or molecular assemblies (e.g., antigen/antibody complexes). These three-dimensional arrays are arranged according to specific mathematical relationships that are well understood in the field. The fundamental unit, or building block, that is repeated in a crystal is called the asymmetric unit. Repetition of the asymmetric unit in an arrangement that conforms to a given, well-defined crystallographic symmetry provides the "unit cell" of the crystal. Repetition of the unit cell by regular translations in all three dimensions provides the crystal. See Giegé et al., Chapter 1, in Crystallization of Nucleic Acids and Proteins, a Practical Approach, 2nd ed., Ducruix and Giegé, eds. (Oxford University Press, New York, 1999) pp. 1-16. Crystallized MAT-Fab bispecific antibodies of the invention may be produced according methods known in the art, such as those described by Shenoy and co-workers in International Publication No. WO 2002/072636, incorporated herein by reference.

Unless otherwise defined herein, scientific and technical terms used in connection with the present invention shall have the meanings that are commonly understood by those of ordinary skill in the art. However, in the event of any latent ambiguity, definitions provided herein take precedent over any dictionary or extrinsic definition. Further, unless otherwise required by context, singular terms shall include pluralities and plural terms shall include the singular. In this application, the use of "or" means "and/or" unless stated otherwise. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

Generally, nomenclatures used in connection with, and techniques of, cell and tissue culture, molecular biology, immunology, microbiology, genetics and protein and nucleic acid chemistry and hybridization described herein are those known and commonly used in the art. The methods and techniques of the present invention are generally performed according to conventional methods well known in the art and as described in various general and more specific references that are cited and discussed throughout the present specification unless otherwise indicated. Enzymatic reactions and purification techniques are performed as commonly accomplished in the art, according to a manufacturer's specifications, or as described herein. The nomenclatures used in connection with, and the laboratory procedures and techniques of, analytical chemistry, synthetic organic chemistry, and medicinal and pharmaceutical chemistry described herein are those known and commonly used in the art. Standard techniques include those used for chemical syntheses, chemical analyses, pharmaceutical preparation, formulation, and delivery, and treatment of patients.

As used herein, the terms "disorder" and "disease" are synonymous and refer to a pathophysiological condition.

As used herein, the term "a disorder (or disease) in which an antigen on a cell (or an activity of an antigen) is detrimental" is intended to include a disorder in which the presence of the antigen in a human subject suffering from the disorder has been shown to be or is suspected of being either responsible for the pathophysiology of the disorder or a has been shown to be or is suspected of being a factor that contributes to a worsening of the disorder. Accordingly, a disorder in which an antigen or an antigen activity is detrimental is a disorder in which reduction of antigen or antigen activity is expected to alleviate one or more symptoms of the disorder or progression of the disorder. Such disorders may be evidenced, for example, by an increase in the concentration of the antigen in the blood or other biological fluid of a human subject suffering from the disorder.

The terms "tumor" and "cancer" are synonymous, unless indicated otherwise. A cancer or tumor may be a blood cancer or tumor (including lymphoid cells) or solid cancer or tumor.

The terms "individual" and "subject" refer to a human subject.

As used herein the terms, "Fab", "Fab fragment", or "Fab binding unit" are synonymous and refer to an immunoglobulin epitope (of an antigen) binding unit that is formed by the association of a polypeptide chain comprising an immunoglobulin light chain variable domain (VL domain) linked to an immunoglobulin light chain constant domain (CL domain) and a polypeptide chain comprising an immunoglobulin heavy chain variable domain (VH domain) linked to an immunoglobulin heavy chain constant domain (CH1 domain), wherein a VL domain pairs with a VH domain to form the epitope (antigen) binding site and a CL domain pairs with a CH1 domain. Thus, as used herein, the terms "Fab", "Fab fragment", and "Fab binding unit" encompass natural Fab fragments produced by papain digestion of a naturally occurring immunoglobulin, such as IgG, wherein the papain digestion yields two Fab fragments that comprise a heavy chain fragment comprising VH-CH1 and a light chain fragment comprising VL-CL. As used herein, the terms also encompass recombinant Fabs in which a selected VL domain is linked to a selected CL domain and a selected VH domain is linked with a selected CH1 domain. In addition, as used herein, the terms also encompass a type of "cross Fab" in which VH-CH1 is present as a light chain polypeptide and VL-CL is present on a larger heavy chain. In particular, in a MAT-Fab bispecific antibody of the invention, the N-terminal (outer) Fab binding unit is this type of "cross Fab" in which a VL-CL is present on the heavy polypeptide chain and pairs with a light chain comprising VH-CH1. In contrast, the inner (C-proximal) Fab binding unit comprises VH-CH1 on the heavy chain that pairs with a light chain comprising VL-CL. This arrangement of outer and inner Fab binding units in a MAT-Fab bispecific antibody of the invention advantageously favors proper association of each VL-CL with its corresponding VH-CH1 to form two functional Fab binding units and disfavors non-functional associations.

A composition or method described herein as "comprising" one or more named elements or steps is open-ended, meaning that the named elements or steps are essential, but other elements or steps may be added within the scope of the composition or method. To avoid prolixity, it is also understood that any composition or method described as "comprising" (or "which comprises") one or more named elements or steps also describes the corresponding, more limited, composition or method "consisting essentially of" (or "which consists essentially of") the same named elements or steps, meaning that the composition or method includes the named essential elements or steps and may also include additional elements or steps that do not materially affect the basic and novel characteristic(s) of the composition or method. It is also understood that any composition or method described herein as "comprising" or "consisting essentially of" one or more named elements or steps also describes the corresponding, more limited, and closed-ended composition or method "consisting of" (or "which consists of") the named elements or steps to the exclusion of any other unnamed element or step. In any composition or method disclosed herein, known or disclosed equivalents of any named essential element or step may be substituted for that element or step.

An element or step "selected from the group consisting of" followed by a list of elements or steps refers to one or more of the elements or steps in the list that follows, including combinations of two or more of the listed elements or steps.

### Organization and Generation of MAT-Fab Bispecific Antibodies

A monovalent asymmetric tandem Fab bispecific antibody ("MAT-Fab" bispecific antibody) according to the invention comprises:
(a) a heavy polypeptide chain ("heavy chain"), wherein said heavy chain comprises (from amino (N-) terminus to carboxyl (C-) terminus): VL_{A}-CL-VH_{B}-CH1-hinge-CH2-CH3m1,
   wherein: VL_{A} is a human immunoglobulin light chain variable domain that is linked to CL, which is a human light chain constant domain, wherein VL_{A}-CL is one half of a first Fab binding unit (capable of binding antigen or epitope "A") and is linked (fused) directly to VH_{B}, wherein VHa is a human immunoglobulin heavy chain variable domain that is linked to CH1, which is a human immunoglobulin heavy chain CH1 constant domain, wherein VH_{B}-CH1 is one half of a second Fab binding unit (capable of binding to antigen or epitope "B"), and wherein VH_{B}-CH1 is linked to a hinge-CH2, wherein hinge-CH2 is the hinge-CH2 region of an immunoglobulin heavy chain and wherein the hinge-CH2 is linked to CH3m 1, which is a first human immunoglobulin heavy chain CH3 constant domain that has been mutated with one or more knobs-into-holes (KiH) mutations to form a structural knob or structural hole in said CH3m 1 constant domain, wherein A and B are different antigens or different epitopes;
(b) a first light chain comprising VH_{A}-CH1, wherein VH_{A} is a human immunoglobulin heavy chain variable domain that is linked to CH1, which is a human immunoglobulin heavy chain CH1 constant domain, and wherein VH_{A}-CH1 is the other half of said first Fab binding unit (capable of binding to antigen or epitope "A");
(c) a second light chain comprising VL_{B}-CL, wherein VL_{B} is a human immunoglobulin light chain variable domain that is linked to CL, which is a human immunoglobulin light chain constant domain, and wherein VL_{B}-CL is the other half of said second Fab binding unit (capable of binding to antigen or epitope "B"); and
(d) an Fc chain comprising hinge-CH2-CH3m2, wherein hinge-CH2 is the hinge-CH2 region of an immunoglobulin heavy chain constant region and wherein the hinge-CH2 is linked to CH3m2, which is a second human immunoglobulin heavy chain CH3 constant domain that has been mutated with one or more knobs-into-holes (KiH) mutations to form a structural knob or structural hole in said CH3m2 constant domain that is complementary to the corresponding structural hole or structural knob of the CH3m1 domain of the heavy chain (a);
with the proviso that:
when the CH3m1 domain of the heavy chain (a) has been mutated to form a structural knob, then the CH3m2 domain of the Fc chain (d) has been mutated to form a complementary structural hole to favor pairing of the CH3m1 domain with the CH3m2 domain; or
when the CH3m1 domain of the heavy chain (a) has been mutated to form a structural hole, then the CH3m2 domain of the Fc chain (d) has been mutated to form a complementary structural knob to favor pairing of the CH3m1 domain with the CH3m2 domain; and
optionally (i.e., with or without), comprising a mutation in both the CH3m1 domain and the CH3m2 domain to introduce a cysteine residue to favor disulfide bond formation in pairing the CH3m 1 domain with the CH3m2 domain.

The Fc regions of the heavy chain and the Fc chain may optionally be modified to modulate Fc effector function. For instance, mutation of ₂₃₄LeuLeu₂₃₅ of the CH2 domain, e.g., to ₂₃₄AlaAla₂₃₅ (EU numbering) is known to reduce or eliminate ADCC/CDC effector functions. Such a change is illustrated in the MAT-Fabs of the examples, *infra,* wherein a LeuLeu ----7 AlaAla switch is effected at residues 18-19 of the hinge-CH2 region in the MAT-Fab (KiH1) heavy chain and Fc chain and the MAT-Fab (KiH2) heavy chain and Fc chain (i.e., see, ₄₇₈AlaAla₄₇₉ of SEQ ID NO: 1 in Table 1 and ₄₇₈AlaAla₄₇₉ of SEQ ID NO:5 in Table 5; and ₄₀AlaAla₄₁ of SEQ ID NO:4 in Table 4 and ₄₀AlaAla₄₁ of SEQ ID NO: 8 in Table 8).

A feature of the structure of a MAT-Fab bispecific antibody described herein is that all adjacent immunoglobulin heavy and light chain variable and constant domains are linked directly to one another without an intervening synthetic amino acid or peptide linker. Such direct linking of adjacent immunoglobulin domains eliminates potentially immunogenic sites that could be formed by introducing one or more intervening amino acids. In engineered antibody molecules featuring tandemly linked binding domains, it has been a common understanding in the art that intervening flexible peptide linkers (such as GGGGS and repeats thereof) were necessary in order to permit adjacent binding sites to bind to antigens simultaneously and to avoid interfering with each other sterically. In contrast to such teachings, the absence of any linkers in the MAT-Fab format does not adversely affect the binding activity of either Fab binding unit of the MAT-Fab antibody. In particular, it has been discovered that adding linkers between CL and VH_{B} on the heavy polypeptide chain does not enhance the binding activity of either Fab binding unit. This means that the structural organization of a MAT-Fab antibody as described above inherently provides an optimal flexibility and three-dimensional configuration so that each Fab binding unit is accessible to and can bind its intended antigen or epitope.

According to the structure of a MAT-Fab bispecific antibody described above, the CH3m1 domain of the MAT-Fab heavy chain or the CH3m2 domain of the MAT-Fab Fc chain comprises a knobs-into-holes (KiH) mutation to form a structural knob to favor pairing of one of the CH3 domains (i.e., CH3m1 or CH3m2) comprising the structural knob with the other CH3 domain (i.e., CH3m2 or CH3m1) comprising a structural hole. Preferably, a mutation is made to form a structural knob in the CH3m1 domain of the heavy chain for pairing with a CH3m2 domain of the Fc chain that comprises a complementary structural hole. Examples of mutations that change an amino acid residue to form a structural knob in the CH3 domain of a MAT-Fab antibody described herein include, but are not limited to, a change from a threonine residue to a tyrosine residue or a change of a threonine residue to a tryptophan residue. Examples of particular mutations that change an amino acid residue to form a structural knob in a CH3 domain of a MAT-Fab antibody described herein include, but are not limited to, a change from a threonine366 residue to a tyrosine residue (T366Y) and a change of a threonine366 residue to a tryptophan residue (T366W). Ridgway et al., Protein Eng., 9: 617-621 (1996); Atwell et al., J. Mol. Biol., 270: 26-35 (1997). Such a particular change is illustrated in the MAT-Fabs of the examples, *infra,* wherein a Thr → Tyr switch is effected at residue 21 of the CH3 domain in MAT-Fab (KiH1) and a Thr → Trp switch is effected at residue 21 of the CH3 domain in MAT-Fab (KiH2) (i.e., see, Tyr₆₁₀ of SEQ ID NO: 1 in Table 1 and Trp₆₁₀ of SEQ ID NO:5 in Table 5).

According to the structure of a MAT-Fab bispecific antibody described above, the CH3m1 domain of the heavy chain or the CH3m2 domain of the Fc chain comprises a knobs-into-holes (KiH) mutation to form a structural hole to favor pairing of one of the CH3 domains comprising the structural hole with the other CH3 domain comprising a structural knob. A variety of knobs-into-holes mutations of the CH3 domains of the Fc regions of antibodies are known in the art. See, for example, Ridgway et al., Protein Eng., 9: 617-621 (1996); Atwell et al., J. Mol. Biol., 270: 26-35 (1997); Klein et al., mAbs, 4(6): 653-663 (2012). Examples of mutations that change one or more residues in a CH3 domain to form a structural hole in a CH3 domain of a MAT-Fab bispecific antibody described herein include, but are not limited to, a change of a tyrosine residue to a threonine residue and a combination of a change of a threonine residue to a serine residue, a change of a leucine residue to an alanine residue, and a change of a tyrosine residue to a valine residue. A preferred mutation to form a structural hole in a CH3 domain of a MAT-Fab antibody of the invention is a change of a tyrosine407 residue to a threonine residue (Y407T). Ridgway et al., Protein Eng., 9: 617-621 (1996). Such a change is illustrated in a MAT-Fab of the examples, *infra,* wherein a Tyr → Thr switch is effected at residue 62 of the CH3 domain in MAT-Fab (KiH1) (i.e., see, Thr₂₁₃ of SEQ ID NO:4 in Table 4). A preferred combination of mutations to form a structural hole in a CH3 domain of a MAT-Fab antibody of the invention comprises a change of a threonine366 residue to a serine residue (T366S), a change of a leucine368 residue to an alanine residue (L368A), and a change of a tyrosine407 residue to a valine residue (Y407V). Atwell et al., J. Mol. Biol., 270: 26-35 (1997). Such a particular 3-amino acid change is illustrated in a MAT-Fab of the examples, *infra,* wherein a Thr → Ser switch is effected at residue 21 of the CH3 domain in MAT-Fab (KiH2), a Leu → Ala switch is effected at residue 23 of the CH3 domain in MAT-Fab (KiH2), and a Tyr ----7 Val switch is effected at residue 62 of the CH3 domain in MAT-Fab (KiH2) (i.e., see, Ser₁₇₂, Ala₁₇₄, and Val₂₁₃ of SEQ ID NO:8 in Table 8). Preferably, the one or more mutations is made to form a structural hole in the CH3m2 domain of the Fc polypeptide chain for pairing with a CH3m1 comprising a structural knob.

A further mutation may be made in the CH3m1 and CH3m2 domains of a MAT-Fab antibody described herein to provide a cysteine residue to form an additional disulfide bond when the CH3m1 domain of the MAT-Fab heavy chain pairs with the CH3m2 domain of the MAT-Fab Fc chain and, thereby, further stabilize the Fc region heterodimer of the MAT-Fab antibody. Specific examples of such mutations include, without limitation, a change of a serine354 to cysteine (S354C) and a tyrosine349 to cysteine (Y349C). Merchant et al., Nat. Biotechnol., 16: 677-681 (1998). Such Cys substitutions are illustrated in a MAT-Fab of the examples, *infra,* wherein a Ser → Cys switch is effected at residue 9 of the CH3 domain in the MAT-Fab (KiH2) heavy chain, and a Tyr → Cys switch is effected at residue 4 of the CH3 domain in the MAT-Fab (KiH2) Fc chain (i.e., see, Cys₅₉₈ of SEQ ID NO:5 and Cys₁₅₅ of SEQ ID NO:8 in Table 8).

A further option is to engineer one or more salt bridges between the CH3m 1 and CH3m2 domains of a MAT-Fab antibody described herein, by mutating either or both domains such that a residue in one of the domains is able to hydrogen bond and electrostatically interact (bond) with a residue in the other domain. For example, a salt bridge may be introduced by changing (mutating) an amino acid residue in the CH3m1 domain to a glutamate or aspartate residue and changing (mutating) a residue in the CH3m2 domain to a lysine or arginine residue such that the glutamate or aspartate residue in the CH3m1 domain can hydrogen bond and electrostatically interact with the lysine or arginine residue in the CH3m2 domain.

In addition to the constant region modifications for MAT-Fab formation discussed above, the constant regions of the heavy chain and/or the Fc chain of the MAT-Fab bispecific antibody described above each optionally (i.e., with or without) comprises one or more mutations to reduce or eliminate at least one Fc effector function, such as antibody-dependent cytotoxicity (ADCC) or complement-mediated cytotoxicity (CDC). Such mutations include, but are not limited to, a change of a leucine234 to alanine (L234A) and a change of a leucine 235 to an alanine (L235A) (Canfield et al., J. Exp. Med., 173(6): 1483-91 (1991)). Preferably, a MAT-Fab bispecific antibody comprises heavy chain and Fc chain amino acid modifications in the CH2 domains to change leucine234 to alanine (L234A, EU numbering) and to change leucine 235 to an alanine (L235A, EU numbering). Such a change is illustrated in the MAT-Fabs of the examples, *infra,* wherein a LeuLeu ----7 AlaAla switch is effected at residues 18-19 of the hinge-CH2 region in MAT-Fab (KiH1) and MAT-Fab (KiH2) (i.e., see, ₄₇₈AlaAla₄₇₉ of SEQ ID NO: 1 in Table 1 and ₄₇₈AlaAla₄₇₉ of SEQ ID NO:5 in Table 5).

MAT-Fab bispecific antibodies described herein are capable of binding each epitope or antigen with high affinity. Specifically, the present invention provides an approach to construct a MAT-Fab bispecific antibody using two parental antibodies, wherein one parental antibody binds a first epitope or antigen and the other parental antibody binds a second epitope or antigen.

Immunoglobulin heavy and light chain variable domains (VH, VL) and heavy and light chain constant domains (such as CL, CH1, CH2, CH3) for use in a MAT-Fab bispecific antibody of the invention may be obtained or derived from known or produced immunoglobulins or genetically altered (mutated) versions thereof. For example, Fab binding units, individual variable domains, and individual constant domains may be readily derived from "parental" antibodies that bind target epitopes or antigens for which a MAT-Fab bispecific antibody is intended to bind. Individual immunoglobulin constant domains may also be derived from parental antibodies that do not bind the same epitope or antigen of the intended MAT-Fab antibody as such constant domains can be linked to variable domains obtained from a different parental antibody that binds a desired target epitope or antigen of the intended MAT-Fab antibody. Other sources of Fab binding units or individual immunoglobulin variable and constant domains include genetically engineered parental antibodies or binding proteins that bind one or two target epitopes or antigens for which a MAT-Fab bispecific antibody is intended to bind. Parental antibodies that may be used as sources of Fab binding units or individual variable and constant domains for use in a producing a MAT-Fab antibody of the invention include clinically approved therapeutic antibodies.

The antigen-binding variable domains and Fab binding units of a MAT-Fab bispecific antibody described herein can be obtained from parental antibodies, including but not limited to, monoclonal antibodies, polyclonal antibodies, and any of a variety of genetically engineered antibodies. Such parental antibodies may be naturally occurring or may be generated by recombinant technology. Persons skilled in the art are familiar with many methods for producing antibodies, including, but not limited to using hybridoma techniques, selected lymphocyte antibody method (SLAM), use of libraries (for example, a phage, yeast, or RNA-protein fusion display or other libraries), immunizing a non-human animal comprising at least some of the human immunoglobulin locus, and preparation of chimeric, CDR-grafted, and humanized antibodies. See, e.g., Neuberger, M.S., et al., Nature 314 (1985) 268-270; Riechmann, L., et al., Nature 332 (1988) 323-327; and EP 2 443 154 B1 (2013).

Variable domains may also be prepared using affinity maturation techniques.

According to the invention, a method of making a MAT-Fab antibody comprises selecting parent antibodies with at least one property desired in the MAT-Fab antibody. Preferably, the desired property is one or more of those used to characterize the MAT-Fab antibody, such as, for example, antigen or epitope specificity, affinity to antigen or epitope, potency, biological function, epitope recognition, stability, solubility, production efficiency, reduced immunogenicity, pharmacokinetics, bioavailability, tissue cross reactivity, or orthologous antigen binding.

Variable domains may be obtained using recombinant DNA techniques from parental antibodies. In an embodiment, a variable domain is a murine heavy or light chain variable domain. In another embodiment, a variable domain is a CDR-grafted or a humanized heavy or light chain variable domain. In another embodiment, a variable domain used in a MAT-Fab antibody of the invention is a human heavy or light chain variable domain.

One or more constant domains may be linked to one another or to variable domains using recombinant DNA techniques, PCR, or other methods available in the art suitable for recombining immunoglobulin domains. In an embodiment, a sequence comprising a heavy chain variable domain is linked to a heavy chain constant domain and a sequence comprising a light chain variable domain is linked to a light chain constant domain. In another embodiment, the constant domains are human immunoglobulin heavy chain constant domains and human immunoglobulin light chain constant domains, respectively.

Variable and constant domains may also be altered to improve a feature of the intended MAT-Fab bispecific antibody. For example, as noted above, the CH3 domains of the Fc regions of a MAT-Fab antibody of the invention possess knob-into-hole mutations that favor proper association of Fc regions of the MAT-Fab heavy polypeptide chain and the MAT-Fab Fc polypeptide chain. In addition, the CH3 domains may be further mutated to introduce cysteine residues that form an additional disulfide bond when the two CH3 domains associate and form a stable Fc heterodimer of the MAT-Fab antibody. Still further, an Fc region of a MAT-Fab antibody may comprise one or more amino acid modifications to enhance or diminish an Fc function including, but not limited to, antibody-dependent cytotoxicity (ADCC), complement-mediated cytotoxicity (CDC), phagocytosis, opsonization, or cell or receptor binding.

As noted above, each of the two Fc domains of a MAT-Fab bispecific antibody described herein comprises knobs-into-holes (KiH) mutations to favor association of the Fc region of the MAT-Fab heavy polypeptide chain with the MAT-Fab Fc chain. However, additional features or modifications of the Fc regions may also be desired. For example, the Fc region of a MAT-Fab antibody may be derived from an Fc region from an immunoglobulin selected from the group consisting of: IgG1, IgG2, IgG3, IgG4, IgA, IgM, IgE, or IgD. In another embodiment, one or more amino acids may be modified in an Fc region to enhance or diminish the affinity of the Fc region of a MAT-Fab bispecific antibody for an FcyR (Fc receptor) relative to the unmodified Fc region. For example, the modification(s) of the Fc region may alter affinity for FcγRI, FcγRII, and/or FcγRIII.

In another embodiment, one or more amino acids may be modified in an Fc region in order to modulate *in vivo* functional or physical half- life of a MAT-Fab bispecific antibody.

The structural organization of each of the polypeptide chains for a MAT-Fab bispecific antibody of the invention allows the correct intracellular assembly of the MAT-Fab antibody using the natural protein expression, folding, and secretion mechanisms present within the cell without having to employ post production processing techniques to obtain the functional MAT-Fab antibody. Particularly preferred is the use of an isolated mammalian host cell comprising one or more expression vectors encoding the four polypeptide chains of a desired MAT-Fab bispecific antibody described herein for producing and expressing a functional MAT-Fab antibody.

### Target Antigen Binding

A MAT-Fab bispecific antibody of the invention is capable of binding one or two target epitopes or antigens. Usually, a MAT-Fab bispecific antibody is engineered to bind an epitope on one antigen and an epitope on a different antigen; thereby enabling the MAT-Fab antibody to serve as an artificial link between to two antigens to achieve a particular result due to the linking of the antigens. A MAT-Fab antibody of the invention may be engineered to bind a cytokine, a polypeptide ligand, a cell surface receptor, a non-receptor cell surface protein, an enzyme, a complex of two or more of the foregoing, or combinations thereof.

In another embodiment, a MAT-Fab bispecific antibody described herein is capable of modulating a biological function of one or two target antigens. More preferably, a MAT-Fab bispecific antibody described herein is capable of neutralizing one or more target antigens.

A MAT-Fab bispecific antibody described herein is also capable of binding two different cytokines. Such cytokines may be selected from the group consisting of: lymphokines, monokines, and polypeptide hormones.

In another embodiment, a MAT-Fab bispecific antibody of the invention is capable of binding at least one target antigen expressed on a surface of a cell. More preferably, a MAT-Fab bispecific antibody of the invention binds two cell surface antigens. The two cell surface antigens may be on the same cell or two cells of the same type. More preferably, however, a MAT-Fab bispecific antibody of the invention binds an antigen expressed on the surface of a first cell and binds a second antigen expressed on the surface of a second cell, wherein the first and second cells are different types of cells. Preferably, a MAT-Fab bispecific antibody described herein binds a first cell surface antigen expressed on an effector cell of the immune system and also binds a second cell surface antigen that is expressed on the surface of a cell that is considered detrimental to an individual and therefore is desired to be eliminated or reduced in size of its population. Effector cells that may be bound by a MAT-Fab bispecific antibody of the invention include T cells, natural killer (NK) cells, monocytes, neutrophils, and macrophages. Examples of cells that are considered detrimental to an individual and that may be bound by a MAT-Fab bispecific antibody of the invention include tumor cells, auto-reactive cells, and virus infected cells.

In another embodiment, a MAT-Fab bispecific antibody of the invention binds a surface antigen expressed on an effector cell. Preferably, the surface antigen is selected from the group consisting of: CD3, CD 16 (also referred to as "FcyRIII"), and CD64 (also referred to as "FcyRI"). More preferably, a MAT-Fab antibody binds CD3 as expressed on T cells, CD16 as expressed on natural killer (NK) cells, or a CD64 as expressed on macrophages, neutrophils, or monocytes.

In another embodiment, a MAT-Fab bispecific antibody of the invention binds a surface antigen that is tumor-associated antigen. Preferred tumor-associated antigens bound by a MAT-Fab antibody of the invention are selected from the group consisting of: CD 19, CD20, human epidermal growth factor receptor 2 ("HER2"), carcinoembryonic antigen ("CEA"), epithelial cell adhesion molecule (EpCAM), and receptor tyrosine kinase-like orphan receptor 1 (ROR 1).

In another embodiment, a MAT-Fab bispecific antibody described herein binds CD3 on an effector cell.

In another embodiment, a MAT-Fab bispecific antibody described herein binds CD20 present on a cancer cell.

In another embodiment, a MAT-Fab bispecific antibody described herein binds a surface antigen expressed on an effector cell, as described herein, and a tumor-associated antigen expressed on a tumor cell as described herein. In a preferred embodiment, a MAT-Fab bispecific antibody described herein binds CD3 on a T cell and CD20 on a malignant B cell.

In a preferred embodiment, a MAT-Fab bispecific antibody described herein binds CD3 and CD20. More preferably, the MAT-Fab bispecific antibody binds CD20 at its outer (N-terminal) Fab binding unit and binds CD3 at its inner (C-proximal) Fab binding unit.

In a preferred embodiment, a MAT-Fab bispecific antibody binds CD20 and CD3 and comprises four polypeptide chains that comprise the amino acid sequences in Tables 1-4 (SEQ ID NOs: 1, 2, 3, 4) or the amino acid sequences in Tables 5-8 (SEQ ID NOs:5, 6, 7, 8).

In a preferred embodiment, a MAT-Fab bispecific antibody of the invention binds a pair of target antigens selected from the group of antigen pairs consisting of: CD20 and CD3, CD3 and CD19, CD3 and Fc-gamma-RIIIA, CD3 and TPBG, CD3 and Epha10, CD3 and IL-5Rα, CD3 and TASCTD-2, CD3 and CLEC12A, CD3 and Prominin-1, CD3 and IL-23R, CD3 and ROR1, CD3 and IL-3Rα, CD3 and PSA, CD3 and CD8, CD3 and Glypican 3, CD3 and FAP, CD3 and EphA2, CD3 and ENPP3, CD3 and CD33, CD3 and CD133, CD3 and EpCAM, CD3 and CD19, CD3 and Her2, CD3 and CEA, CD3 and GD2, CD3 and PSMA, CD3 and BCMA, CD3 and A33, CD3 and B7-H3, CD3 and EGFR, CD3 and P-cadherin, CD3 and HMW-MAA, CD3 and TIM-3, CD3 and CD38, CD3 and TAG-72, CD3 and SSTR, CD3 and FRA, CD16 and CD30, CD64 and Her2, CD 137 and CD20, CD138 and CD20, CD19 and CD20, CD38 and CD20, CD20 and CD22, CD40 and CD20, CD47 and CD20, CD 137 and EGFR, CD137 and Her-2, CD 137 and PD-1, CD 137 and PDL-1, PD-1 and PD-L1, VEGF and PD-L1, Lag-3 and TIM- 3, OX40 and PD-1, TIM-3 and PD-1, TIM-3 and PDL-1, EGFR and DLL-4, VEGF and EGFR, HGF and VEGF, a first epitope of VEGF and a different second epitope of VEGF, VEGF and Ang2, EGFR and cMet, PDGF and VEGF, VEGF and DLL-4, OX40 and PD-L1, ICOS and PD-1, ICOS and PD-L1, Lag-3 and PD-1, Lag-3 and PD-L1, Lag-3 and CTLA-4, ICOS and CTLA- 4, CD138 and CD40, CD38 and CD138, CD38 and CD40, CD-8 and IL-6, CSPGs and RGM A, CTLA-4 and BTN02, CTLA-4 and PD-1, IGFl and IGF2, IGFl/2 and Erb2B, IGF-IR and EGFR, EGFR and CD13, IGF-IR and ErbB3, EGFR-2 and IGFR, a first epitope of Her2 and a second different epitope of Her2, Factor IXa and Met, Factor X and Met, VEGFR-2 and Met, VEGF-A and Angiopoietin-2 (Ang-2), IL-12 and TWEAK, IL-13 and IL-lβ, MAG and RGM A, NgR and RGM A, NogoA and RGM A, OMGp and RGM A, PDL-1 and CTLA-4, PD-1 and TIM-3, RGM A and RGM B, Te38 and TNFα, TNFα and Blys, TNFα and CD22, TNFα and a CTLA-4, TNFα and GP130, TNFα and IL-12p40, and TNFα and RANK ligand.

In yet another embodiment, a MAT-Fab bispecific antibody of the invention is capable of binding one or two cytokines, cytokine-related proteins, or cytokine receptors.

In another embodiment, a MAT-Fab bispecific antibody of the invention is capable of binding one or more chemokines, chemokine receptors, and chemokine-related proteins.

In another embodiment, a MAT-Fab bispecific antibody of the invention is also capable of binding receptors, including lymphokine receptors, monokine receptors, and polypeptide hormone receptors.

### Glycosylated MAT-Fab Bispecific Antibodies

A MAT-Fab antibody of the invention may comprise one or more carbohydrate residues. Preferably, a MAT-Fab bispecific antibody described above is glycosylated. More preferably, the glycosylation is a human glycosylation pattern.

Nascent *in vivo* protein production may undergo further processing, known as post-translational modification. In particular, sugar (glycosyl) residues may be added enzymatically, a process known as glycosylation. The resulting proteins bearing covalently linked oligosaccharide side chains are known as glycosylated proteins or glycoproteins.

Naturally occurring antibodies are glycoproteins with one or more carbohydrate residues in the Fc domain, as well as the variable domain. Carbohydrate residues in the Fc domain have important effects on the effector function of the Fc domain, with minimal effect on antigen binding or half-life of the antibody (Jefferis, Biotechnol. Prog., 21: 11-16 (2005)). In contrast, glycosylation of the variable domain may have an effect on the antigen binding activity of the antibody. Glycosylation in the variable domain may have a negative effect on antigen binding affinity, likely due to steric hindrance (Co, M.S., et al., Mol. Immunol., 30: 1361- 1367 (1993)), or may result in increased affinity for the antigen (Wallick et al., J. Exp. Med., 168:1099-1109 (1988); Wright, A., et al., EMBO J., 10: 2717-2723 (1991)).

One aspect of the present invention is directed to generating glycosylation site mutants in which the O- or N-linked glycosylation site of a MAT-Fab antibody has been mutated. One skilled in the art can generate such mutants using standard well-known technologies. Glycosylation site mutants that retain the biological activity but have increased or decreased binding activity are another object of the present invention.

In still another embodiment, the glycosylation of a MAT-Fab antibody of the invention is modified. For example, an aglycoslated MAT-Fab antibody can be made (i.e., the antibody lacks glycosylation). Glycosylation can be altered, for example, to increase the affinity of the MAT-Fab antibody for one or both antigens. Such carbohydrate modifications can be accomplished by, for example, altering one or more sites of glycosylation within the antibody sequence. For example, one or more amino acid substitutions can be made that result in elimination of one or more variable region glycosylation sites to thereby eliminate glycosylation at that site. Such aglycosylation may increase the affinity of a MAT-Fab antibody for antigen. Such an approach is described in further detail in International Publication No. WO 2003/016466, and U.S. Patent Nos. 5,714,350 and 6,350,861.

Additionally, or alternatively, a modified MAT-Fab antibody of the invention can be made that has an altered type of glycosylation, such as a hypofucosylated antibody having reduced amounts of fucosyl residues (see Kanda et al., J. Biotechnol., 130(3): 300-310 (2007)) or an antibody having increased bisecting GlcNAc structures. Such altered glycosylation patterns have been demonstrated to increase the ADCC ability of antibodies. Such carbohydrate modifications can be accomplished, for example, by expressing the antibody in a host cell with altered glycosylation machinery. Cells with altered glycosylation machinery have been described in the art and can be used as host cells in which to express recombinant antibodies of the invention to thereby produce an antibody with altered glycosylation. See, for example, Shields et al., J. Biol. Chem., 277: 26733-26740 (2002); Umana et al., Nat. Biotech., 17: 176-180 (1999), as well as, European Patent No: EP 1 176 195; International Publication Nos. WO 2003/035835 and WO 1999/54342.

Protein glycosylation depends on the amino acid sequence of the protein of interest, as well as the host cell in which the protein is expressed. Different organisms may produce different glycosylation enzymes (e.g., glycosyltransferases and glycosidases), and have different substrates (nucleotide sugars) available. Due to such factors, protein glycosylation pattern, and composition of glycosyl residues, may differ depending on the host system in which the particular protein is expressed. Glycosyl residues useful in the invention may include, but are not limited to, glucose, galactose, mannose, fucose, N-acetylglucosamine and sialic acid. Preferably the glycosylated MAT-Fab antibody comprises glycosyl residues such that the glycosylation pattern is human.

It is known to those skilled in the art that differing protein glycosylation may result in differing protein characteristics. For instance, the efficacy of a therapeutic protein produced in a microorganism host, such as yeast, and glycosylated utilizing the yeast endogenous pathway may be reduced compared to that of the same protein expressed in a mammalian cell, such as a CHO cell line. Such glycoproteins may also be immunogenic in humans and show reduced half-life *in vivo* after administration. Specific receptors in humans and other animals may recognize specific glycosyl residues and promote the rapid clearance of the protein from the bloodstream. Other adverse effects may include changes in protein folding, solubility, susceptibility to proteases, trafficking, transport, compartmentalization, secretion, recognition by other proteins or factors, antigenicity, or allergenicity. Accordingly, a practitioner may prefer a MAT-Fab antibody with a specific composition and pattern of glycosylation, for example glycosylation composition and pattern identical, or at least similar, to that produced in human cells or in the species-specific cells of the intended subject animal.

Expressing glycosylated MAT-Fab antibodies different from that of a host cell may be achieved by genetically modifying the host cell to express heterologous glycosylation enzymes. Using techniques known in the art, a practitioner may generate a MAT-Fab antibody exhibiting human protein glycosylation. For example, yeast strains have been genetically modified to express non-naturally occurring glycosylation enzymes such that glycosylated proteins (glycoproteins) produced in these yeast strains exhibit protein glycosylation identical to that of animal cells, especially human cells (for example, U.S. Patent Publication Nos. 2004/0018590 and 2002/0137134).

### Nucleic Acids, Vectors, Host Cells

The invention provides an isolated polynucleotide or isolated polynucleotides encoding all four of the polypeptide chains of a MAT-Fab bispecific antibody described herein.

The invention also provides a vector comprising the isolated polynucleotide or polynucleotides encoding all four of the polypeptides of a MAT-Fab bispecific antibody described herein. A vector may be an autonomously replicating vector or a vector that incorporates the isolated nucleic acid that is present in the vector into a host cell genome. The isolated polynucleotide or isolated polynucleotides encoding all four polypeptide chains of a MAT-Fab antibody may also be inserted into a vector for carrying out various genetic analyses, for expressing a MAT-Fab antibody, or for characterizing or improving one or more properties of a MAT-Fab antibody described herein.

A vector according to the invention may be used to replicate the isolated polynucleotide or isolated polynucleotides encoding all four polypeptide chains of a MAT-Fab antibody described herein.

A vector according to the invention may be used to express the isolated polynucleotide or isolated polynucleotides encoding a MAT-Fab bispecific antibody described herein or to express the isolated polynucleotide or isolated polynucleotides encoding one or more polypeptide chains of the MAT-Fab antibody. Preferred vectors for cloning and expressing nucleic acids described herein include, but are not limited, pcDNA, pTT (Durocher et al, Nucleic Acids Res., 30(2e9): 1-9 (2002)), pTT3 (pTT with additional multiple cloning sites), pEFBOS (Mizushima and Nagata, Nucleic Acids Res., 18(17): 5322 (1990)), pBV, pJV, pcDNA3.1 TOPO, pEF6 TOPO and pBJ.

The invention also provides an isolated host cell comprising a vector described above. Such an isolated host cell comprising a vector described herein may be an isolated prokaryotic cell or an isolated eukaryotic cell.

An isolated prokaryotic host cell comprising a vector described herein may be a bacterial host cell. The bacterial host cell may be a Gram positive, Gram negative, or Gram variable bacterial host cell. Preferably, a bacterial host cell comprising a vector described herein is a Gram negative bacterium. Even more preferably, a bacterial host cell comprising a vector described herein is an *Escherichia coli* cell.

An isolated host cell comprising a vector described herein may be a eukaryotic host cell. Preferred isolated eukaryotic host cells comprising a vector described herein may include, without limitation, a mammalian host cell, an insect host cell, a plant host cell, a fungal host cell, a eukaryotic algal host cell, a nematode host cell, a protozoan host cell, and a fish host cell. Preferably, an isolated mammalian host cell comprising a vector described herein is selected from the group consisting of: a Chinese Hamster Ovary (CHO) cell, a COS cell, a Vero cell, an SP2/0 cell, an NS/0 myeloma cell, a human embryonic kidney (HEK293) cell, a baby hamster kidney (BHK) cell, a HeLa cell, a human B cell, a CV-1/EBNA cell, an L cell, a 3T3 cell, an HEPG2 cell, a PerC6 cell, and an MDCK cell. Preferred isolated fungal host cells comprising a vector described herein are selected from the group consisting of: *Aspergillus, Neurospora, Saccharomyces, Pichia, Hansenula, Schizosaccharomyces, Kluyveromyces, Yarrowia,* and *Candida.* More preferably, a *Saccharomyces* host cell comprising a vector described herein is a *Saccharomyces cerevisiae* cell.

Also provided is a method of producing a MAT-Fab bispecific antibody described herein comprising culturing an isolated host cell comprising a vector that comprises nucleic acid encoding the MAT-Fab antibody under conditions sufficient to produce the MAT-Fab antibody.

Another aspect of the invention is a MAT-Fab bispecific antibody produced by a method described above.

### Conjugates

Primarily owing to the presence of a dimerized Fc region, a MAT-Fab bispecific antibody of the invention can be conjugated to any of a variety of agents that are currently conjugated to IgG antibodies. For example, a MAT-Fab bispecific antibody described above can be conjugated to an selected from the group consisting of: an immunoadhesion molecule, an imaging agent, a therapeutic agent, and a cytotoxic agent. A preferred imaging agent that may be conjugated to a MAT-Fab bispecific antibody of the invention is selected from the group consisting of: a radiolabel, an enzyme, a fluorescent label, a luminescent label, a bioluminescent label, a magnetic label, biotin, streptavidin, or avidin. Radiolabels that may be conjugated to a MAT-Fab bispecific antibody described herein include, but are not limited to, ³H, ¹⁴C, ³⁵S, ⁹⁰Y, ⁹⁹Tc, ¹¹¹In, ¹³¹I, ¹⁷⁷Lu, ¹⁶⁶Ho, and ¹⁵³Sm. Preferred cytotoxic or therapeutic compounds that may conjugated to a MAT-Fab bispecific antibody described herein include, but are not limited to, an anti-metabolite, an alkylating agent, an antibiotic, a growth factor, a cytokine, an anti-angiogenic agent, an anti-mitotic agent, an anthracycline, a toxin, and an apoptotic agent. In some cases, a MAT-Fab antibody is conjugated directly to the agent. Alternatively, a MAT-Fab antibody is conjugated to the agent via a linker. Suitable linkers include, but are not limited to, amino acid and polypeptide linkers disclosed herein. Linkers may be cleavable or non-cleavable.

### Crystallized Forms

A MAT-Fab bispecific antibody of the invention may be used as a crystallized form prepared using a large-scale crystallization method such as, for example, described by Shenoy and co-workers in International Publication No. WO 2002/072636, incorporated herein by reference. Such methods provide crystals of antibody molecules that differ considerably from those employed in classic X-ray crystallographic studies. Whereas crystal quality is very important for precise measurements in X-ray crystallographic studies, such is not the case for crystals produced by large-scale crystallization methods for pharmaceutical use. Crystals of antibody molecules produced using large-scale crystallization methods are sufficiently pure for pharmaceutical studies and manufacturing, retain biological activity, are particularly well-suited for storage (as antibody crystals are less subject to undesirable interactions that can occur in solutions), can provide parenterally administrable preparations containing very high concentrations of the biologically active antibody molecule, can provide advantageous dosage preparations (including high concentrations in non-aqueous suspensions), can provide increased half-life *in vivo,* and can be formulated with (encapsulated by) polymeric carriers for controlled release of the antibody molecules *in vivo.*

Particularly preferred is a crystallized MAT-Fab antibody that retains any binding activity as well as any desirable biological activity of the non-crystal form. A crystallized MAT-Fab bispecific antibody may also provide carrier-free controlled release of the MAT-Fab when administered to an individual.

A preferred composition for the release of a crystallized MAT-Fab bispecific antibody according to the invention comprises a crystallized MAT-Fab bispecific antibody as described herein, an excipient ingredient, and at least one polymeric carrier. Preferably the excipient ingredient is selected from the group consisting of albumin, sucrose, trehalose, lactitol, gelatin, hydroxypropyl-β-cyclodextrin, methoxypolyethylene glycol and polyethylene glycol. Preferably the polymeric carrier is a polymer selected from one or more of the group consisting of: poly(acrylic acid), poly(cyanoacrylates), poly(amino acids), poly(anhydrides), poly(depsipeptide), poly(esters), poly(lactic acid), poly(lactic-co-glycolic acid) or PLGA, poly(b-hydroxybutryate), poly(caprolactone), poly(dioxanone); poly(ethylene glycol), poly((hydroxypropyl) methacrylamide, poly[(organo)phosphazene], poly(ortho esters), poly(vinyl alcohol), poly(vinylpyrrolidone), maleic anhydride/alkyl vinyl ether copolymers, pluronic polyols, albumin, alginate, cellulose and cellulose derivatives, collagen, fibrin, gelatin, hyaluronic acid, oligosaccharides, glycaminoglycans, sulfated polysaccharides, blends thereof, and copolymers thereof.

### Uses of MAT-Fab Bispecific Antibodies

The invention provides a MAT-Fab bispecific antibody as claimed herein for use in a method of treating a disease or disorder in a human subject, wherein said MAT-Fab bispecific antibody binds one or two target epitopes, one or two target antigens, or a combination of a target epitope and a target antigen that are detrimental to a human subject wherein binding of epitopes or antigens by the MAT-Fab bispecific antibody provides a treatment for the disease or disorder.

A disorder in which an epitope, an antigen, or an activity of an antigen is detrimental is intended to include a disorder in which the presence of an epitope (for example, an epitope of a cell surface protein or of a soluble protein) or antigen or activity of an antigen in a human subject suffering from the disorder has been shown to be or is suspected of being either responsible for the pathophysiology of the disorder or a has been shown to be or is suspected of being a factor that contributes to a worsening of the disorder. Accordingly, a disorder in which an epitope, an antigen, or an antigen activity is detrimental is a disorder in which reduction of the epitope, antigen, or the antigen activity is expected to alleviate one or more symptoms of the disorder or progression of the disorder and thereby providing treatment of the disorder. Such disorders may be evidenced, for example, by an increase in the concentration of the antigen (or epitope) in the blood or other biological fluid of a human subject suffering from the disorder.

In another embodiment, the invention provides a MAT-Fab bispecific antibody as claimed herein for use in a method of treating a human subject suffering from a disorder in which one or two target antigens or epitopes capable of being bound by the MAT-Fab bispecific antibody is detrimental to the human subject, wherein the method comprises administering to the human subject the MAT-Fab bispecific antibody such that the activity of the one or two target antigens (or epitopes) in the human subject is inhibited and treatment is achieved.

A MAT-Fab bispecific antibody described herein is particularly useful in a method of treating a disorder comprising a "retargeting" (or "recruiting") of effector cells (such as T cells, NK cells, monocytes, neutrophils, macrophages) to attack specific target cells that express a disorder-associated antigen and that are detrimental to a human subject and, therefore, where it is desirable to eliminate or substantially reduced the population of the detrimental target cells. Preferred examples of such detrimental target cells are tumor cells (e.g., blood (including lymph) tumor cells and solid tumor cells), auto-reactive cells, and virus infected cells. In a retargeting method of the invention, a MAT-Fab antibody binds an antigen expressed on the surface of an effector cell and an antigen expressed on the surface of a target cell that is detrimental to a human subject, wherein binding of the MAT-Fab antibody to the antigen on the effector cell and to the antigen on the detrimental cell activates the effector cell to attack the detrimental target cell.

Accordingly, the invention provides a MAT-Fab bispecific antibody as claimed herein for use in a method of treating a disorder in a human subject, wherein the MAT-Fab bispecific antibody described herein binds an antigen on an effector cell and that binds an antigen associated with the disorder expressed on a target cell that is detrimental to the human subject, wherein the binding of the MAT-Fab bispecific antibody to both effector cell and the target cell activates the effector cell to attack the detrimental target cell.

The simultaneous binding of a MAT-Fab bispecific antibody described herein to a single target antigen on an effector cell and to a single target antigen on a detrimental target cell can activate the effector cell to attack the target cell advantageously without also eliciting a massive detrimental release of cytokines (cytokine storm). A massive release of cytokines (cytokine storm), can have deleterious effects not only on the local tissue but also on more remote tissues of the body of a patient resulting in potential complications and untoward side effects. Such a cytokine storm may occur in a natural immune response in which an effector cell, such as a T cell, binds an antigen-presenting cell (APC), or when a bivalent or multivalent antibody artificially cross-links two or more antigens on the surface of an effector cell. In contrast, owing to the fact that a MAT-Fab bispecific antibody of the invention can be engineered so that only one of its Fab binding units binds an antigen on the effector cell, the possibility of a non-specific T cell activation and subsequent cytokine storm is greatly diminished, while it retains the ability to activate the effector cell to attack the detrimental target cell that is bound by the other Fab binding unit of the MAT-Fab antibody.

Preferably, a method of retargeting an effector cell to attack a detrimental target cell comprises contacting the effector cell and the detrimental target cell with a MAT-Fab bispecific antibody described herein that binds an antigen on the detrimental target cell and binds an antigen expressed on an effector cell. Preferred effector cell antigens include CD3, CD 16 (also referred to as "FcyRIII"), and CD64 (also referred to as "FcyRI"). More preferably, the method comprises a MAT-Fab antibody that binds CD3 as expressed on a T cell, CD 16 as expressed on a natural killer (NK) cell, or a CD64 as expressed on a macrophage, neutrophil, or monocyte.

In another embodiment, the invention provides a MAT-Fab bispecific antibody as claimed herein for use in a method of treating a tumor in a human subject, wherein the MAT-Fab antibody binds an antigen on an effector cell and also binds an antigen on a target tumor cell, wherein binding of the MAT-Fab antibody to the effector cell and the target tumor cell activates the effector cell to attack the tumor cell. Preferably, the antigen on the effector cell is CD3 as expressed on a T cell.

In a preferred embodiment, a MAT-Fab bispecific antibody as claimed herein for use in a method of treating a tumor in a human subject comprises retargeting an effector cell to attack a target tumor cell comprising the step of administering to the human subject a MAT-Fab bispecific antibody described herein that binds an antigen on the effector cell and an antigen on the target tumor cell, wherein the antigen on the target tumor cell is a tumor-associated antigen selected from the group consisting of: CD19, CD20, human epidermal growth factor receptor 2 ("HER2"), carcinoembryonic antigen ("CEA"), epithelial cell adhesion molecule (EpCAM), and receptor tyrosine kinase-like orphan receptor 1 (ROR 1).

In another embodiment, the invention provides a MAT-Fab bispecific antibody as claimed herein that binds an antigen on an effector cell and that binds an antigen on malignant B cells for use in a method of treating a human subject for a B cell-associated tumor. Preferably, the MAT-Fab bispecific antibody of the invention binds an antigen on malignant B cells of a cancer disorder selected from the group consisting of: acute lymphoblastic leukemia, Hodgkin's lymphoma, non-Hodgkin's lymphoma (NHL), precursor B cell lymphoblastic leukemia/lymphoma, mature B cell neoplasms, B cell chronic lymphocytic leukemialsmall lymphocytic lymphoma, B cell prolymphocytic leukemia, lymphoplasmacytic lymphoma, mantle cell lymphoma, follicular lymphoma, cutaneous follicle center lymphoma, marginal zone B cell lymphoma, hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma.

In a particularly preferred embodiment, the invention provides a MAT-Fab bispecific antibody as claimed herein that binds CD3 on a T cell and that binds CD20 on a target tumor B cell for use in a method of treating a human subject for a B cell-associated tumor. More preferably, the MAT-Fab bispecific antibody binds CD20 at its outer (N-terminal) Fab binding unit (e.g., see, in Fig. 1A, Fab comprising domains VHA-CH1 and VLA-CL) and binds CD3 at its inner (C-proximal) Fab binding unit (e.g., in Fig. 1A, Fab comprising domains VHB-CH1 and VLB-CL).

In another embodiment, a MAT-Fab bispecific antibody as claimed herein for use in a method of treating a disorder according to the invention may comprise bringing the MAT-Fab antibody into contact with effector cells and detrimental target cells in a type of *ex vivo* procedure in which effector cells extracted from a human subject in need of treatment are contacted with a MAT-Fab antibody outside the human subject and, after providing time for binding of the MAT-Fab antibody to the effector cells, the effector cells bound to the MAT-Fab antibody are then administered to the human subject so that complexes of MAT-Fab antibody bound to effector cells can then seek to bind detrimental target cells, such as tumor cells, inside the human subject. In another embodiment, effector cells and tumor cells extracted from a human subject in need of treatment are contacted with a MAT-Fab antibody outside the human subject and, after providing time for binding of the MAT-Fab antibody to the effector cells and tumor cells, the effector cells and tumor cells bound to MAT-Fab antibody are administered to the human subject.

In another embodiment, a MAT-Fab bispecific antibody as claimed herein may also be engineered to deliver a cytotoxic agent to a detrimental target cell, such as a tumor cell. In this embodiment, one Fab binding unit of a MAT-Fab antibody contains a binding site for a target antigen on a detrimental target cell and the other Fab binding unit contains a binding site for a cytotoxic agent. Such an engineered MAT-Fab antibody of the invention can be mixed with or otherwise contacted with the cytotoxic agent to which it will bind at its engineered Fab binding unit. The MAT-Fab antibody carrying the bound cytotoxic agent can then be brought into contact with the detrimental target cell to deliver the cytotoxic agent to the detrimental target cell. Such a delivering system is particularly effective when the MAT-Fab antibody binds to the detrimental target cell and then is internalized into the cell along with the bound cytotoxic agent so that the cytotoxic agent can be released inside the detrimental target cell.

Additionally, MAT-Fab bispecific antibodies provided herein can be employed for tissue-specific delivery (target a tissue marker and a disease mediator for enhancing local pharmacokinetics and thus higher efficacy and/or lower toxicity), including intracellular delivery (targeting an internalizing receptor and an intracellular molecule), delivering to inside brain (for example, targeting transferrin receptor and a central nervous system (CNS) disease mediator for crossing the blood-brain barrier). MAT-Fab antibodies can also serve as a carrier protein to deliver an antigen to a specific location via binding to a non-neutralizing epitope of that antigen and also to increase the half-life of the antigen.

Furthermore, MAT-Fab bispecific antibodies can be designed to either be physically linked to medical devices implanted into patients or to target these medical devices (see Burke et al. (2006) Advanced Drug Deliv. Rev. 58(3): 437-446; Hildebrand et al. (2006) Surface and Coatings Technol. 200: 6318-6324; "Drug/device combinations for local drug therapies and infection prophylaxis," Wu, Peng, et al., (2006) Biomaterials, 27(11):2450-2467; "Mediation of the cytokine network in the implantation of orthopedic devices," Marques et al., in Biodegradable Systems in Tissue Engineering and Regenerative Medicine, Reis et al., eds. (CRC Press LLC, Boca Raton, 2005) pp. 377-397). Directing appropriate types of cell to the site of medical implant may promote healing and restoring normal tissue function. Alternatively, MAT-Fab bispecific antibodies may be used to inhibit mediators (including but not limited to cytokines) that are released upon device implantation. The disclosure herein also provides diagnostic applications including, but not limited to, diagnostic assay methods, diagnostic kits containing one or more binding proteins, and adaptation of the methods and kits for use in automated and/or semi-automated systems. The methods, kits, and adaptations provided may be employed in the detection, monitoring, and/or treatment of a disease or disorder in an individual. This is further elucidated below.

A MAT-Fab bispecific antibody described herein is readily adapted to any of a variety of immunodetection assays and purification formats available in the art for detecting, quantitating, or isolating a target antigen or cell expressing a target antigen. Such formats include, but are not limited to, immunoblot assays (for example, a Western blot); immunoaffinity chromatography, for example, wherein a MAT-Fab bispecific antibody is adsorbed or linked to a chromatography resin or bead; immunoprecipitation assays; immunochips; tissue immunohistochemistry assays; flow cytometry (including fluorescence activated cell sorting); sandwich immunoassays; immunochips, wherein a MAT-Fab antibody is immobilized or bound to a substrate; radioimmunoassays (RIAs); enzyme immunoassays (EIAs); enzyme-linked immunosorbent assay (ELISAs); competitive-inhibition immunoassays; fluorescence polarization immunoassay (FPIA); enzyme multiplied immunoassay technique (EMIT); bioluminescence resonance energy transfer (BRET); and homogenous chemiluminescent assays. Methods employing mass spectrometry are provided by the present disclosure and include, but are not limited to MALDI (matrix-assisted laser desorption/ionization) or by SELDI (surface-enhanced laser desorption/ ionization) that comprise a MAT-Fab antibody that binds a target antigen or epitope on an antigen or fragment thereof.

The invention further provides a MAT-Fab bispecific antibody as claimed herein for use in a method for detecting an antigen in a sample (such as, for example, a mixture, composition, solution, or biological sample) comprising contacting the sample with the MAT-Fab bispecific antibody that binds a target antigen. Biological samples that can serve as a sample for an immunodetection assay of the invention include, without limitation, whole blood, plasma, serum, various tissue extracts, tears, saliva, urine, and other bodily fluids. Preferably, an immunodetection assay of the invention detects any one of the following: the MAT-Fab bispecific antibody bound to the target antigen, the complex of the MAT-Fab bispecific antibody and the target antigen, or the MAT-Fab bispecific antibody that remains unbound. Methods to detect a binding complex comprising a target antigen and a MAT-Fab antibody preferably employ a detection system that uses one or more signal-generating molecules (detectable labels) that will generate a signal that is easily detected by the human eye or is readily detected or measured by a signal detection instrument (for example, spectrophotometer). The MAT-Fab bispecific antibody may be labeled directly or indirectly with a detectable signal-generating system to facilitate detection of the bound or unbound MAT-Fab bispecific antibody. Detectable signals that may be used in detecting a binding complex comprising a target antigen and a MAT-Fab bispecific antibody include, but are not limited to, a fluorescent signal (for example, as generated from a fluorescent dye or cyanin molecule that can be bound by one of the Fab binding units of a MAT-Fab antibody or attached by other means to the MAT-Fab antibody); a visible color signal (e.g., as generated with an enzyme or colored molecule (e.g., a pigment) that can also be attached directly or indirectly to the MAT-Fab antibody); a radioactive signal (e.g., as generated by a radioisotope that can be attached directly or indirectly to a MAT-Fab antibody); and a light signal (e.g., as generated by a chemiluminescent or bioluminescent system). An example of a bioluminescent system is a luciferin-luciferase system in which a luciferase may be attached directly or indirectly to a MAT-Fab antibody or a secondary detection antibody to generate a detectable light signal in the presence of the luciferin substrate.

A preferred enzyme useful in an immunodetection assay of the invention is one that can provide a detectable signal when brought into contact with one more reagents. Such enzymes include, but are not limited to, horseradish peroxidase, alkaline phosphatase, β-galactosidase, or acetylcholinesterase.

Examples of suitable fluorescent materials that may be used in an immunodetection assay of the invention include, but are not limited to, umbelliferone, fluorescein, fluorescein isothiocyanate, rhodamine, dichlorotriazinylamine fluorescein, dansyl chloride, and phycoerythrin.

An example of a luminescent material that may be used in an immunodetection assay of the invention is luminol.

Examples of suitable radioactive species that may be used in an immunodetection assay of the invention include, but are not limited to, ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, and ¹⁵³Sm.

### Pharmaceutical Compositions

The invention provides pharmaceutical compositions for use in treating a human subject comprising a MAT-Fab bispecific antibody as claimed herein. Such compositions are prepared using techniques and ingredients well-known in the art for preparing pharmaceutical compositions for administering a therapeutic antibody to human subjects. A composition comprising a MAT-Fab antibody described herein may be formulated for administration by any of a variety routes or modes of administration. A composition comprising a MAT-Fab antibody may be formulated for parenteral or non-parenteral administration. A composition comprising a MAT-Fab antibody for use in treating a cancer, autoimmune disease, or inflammatory disease may be formulated for parenteral administration, for example, but not limited to, intravenous, subcutaneous, intraperitoneal, or intramuscular administration. More preferably, a composition is formulated for intravenous administration. Such parenteral administration is preferably carried out by injection or infusion of the composition.

Compositions comprising a MAT-Fab antibody for administration to a human individual may comprise an effective amount of the MAT-Fab antibody in combination with one or more pharmaceutically acceptable components such as a pharmaceutically acceptable carrier (vehicle, buffer), excipient, or other ingredient. By "pharmaceutically acceptable" is meant that a compound, component, or ingredient of a composition is compatible with the physiology of a human subject and also is not deleterious to the effective activity of the MAT-Fab antibody component or to a desired property or activity of any other component that may be present in a composition that is to be administered to a human subject. Examples of pharmaceutically acceptable carriers include, but are not limited to, water, saline, phosphate buffered saline, dextrose, glycerol, ethanol and the like, as well as combinations thereof. In some cases, it may be preferable to include isotonic agents, including, but not limited to, sugars; polyalcohols, such as mannitol or sorbitol; sodium chloride; and combinations thereof. Pharmaceutically acceptable carriers may further comprise minor amounts of auxiliary substances such as wetting or emulsifying agents, preservatives, or buffers to enhance the shelf life or effectiveness of the composition. An excipient is generally any compound or combination of compounds that provides a desired feature to a composition other than a primary therapeutic activity. The pH may be adjusted in a composition as necessary, for example, to promote or maintain solubility of component ingredients, to maintain stability of one or more ingredients in the formulation, and/or to deter undesired growth of microorganisms that potentially may be introduced at some point in the procedure.

Compositions comprising a MAT-Fab antibody may also include one or more other ingredients such as other medicinal agents (for example, an anti-cancer agent, an antibiotic, an anti-inflammatory compound, an anti-viral agent), fillers, formulation adjuvants, and combinations thereof.

The compositions according to the invention may be in a variety of forms. These include, but are not limited to, liquid, semi-solid, and solid dosage forms, dispersions, suspensions, tablets, pills, powders, liposomes, and suppositories. The preferred form depends on the intended route of administration. Preferred compositions are in the form of injectable or infusible solutions, such as compositions similar to those used administration of therapeutic antibodies approved for use in humans. In a preferred embodiment, a MAT-Fab antibody described herein is administered by intravenous injection or infusion. In another embodiment, a MAT-Fab antibody is administered by intramuscular or subcutaneous injection.

Therapeutic compositions must be sterile and stable under the conditions of manufacture and storage. The composition can be formulated as a solution, microemulsion, dispersion, liposome, or other structure suitable for high drug concentration. Sterile injectable solutions may be prepared by incorporating the active compound, i.e., a MAT-Fab antibody, in the required amount in an appropriate solvent, optionally with one or a combination of ingredients that provide a beneficial feature to the composition, as required, followed by filtered sterilization. Generally, dispersions are prepared by incorporating the active ingredient into a sterile vehicle that contains a basic dispersion medium (for example, sterile water, sterile isotonic saline, and the like) and optionally one or more other ingredients that may be required for adequate dispersion. In the case of sterile, lyophilized powders for the preparation of sterile injectable solutions, preferred methods of preparation include vacuum drying and spray-drying that produce a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion, and by the use of surfactants. Prolonged absorption of injectable compositions can be brought about by including in the composition an agent that delays absorption, for example, a monostearate salt and/or gelatin.

A MAT-Fab antibody may be administered by a variety of methods known in the art. As will be appreciated by the skilled practitioner, the route or mode of administration will vary depending upon the desired results. In certain embodiments, a MAT-Fab antibody may be prepared with a carrier that will protect the antibody against rapid release, such as a controlled release formulation, including implants, transdermal patches, and microencapsulated delivery systems. Biodegradable, biocompatible polymers can be used, such as ethylene vinyl acetate, a polyanhydride, a polyglycolic acid, a collagen, a polyorthoester, and a polylactic acid. A variety of methods for the preparation of such formulations are known to those skilled in the art.

A pharmaceutical composition disclosed above may be prepared for administration to an individual by at least one mode selected from the group consisting of: parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal.

Additional embodiments and features of the invention will be apparent from the following non-limiting examples.

### EXAMPLES

Example 1. Construction, expression, purification, and analysis of CD20/CD3 MAT-Fab bispecific antibody.

To demonstrate the MAT-Fab technology, examples of a CD20/CD3 MAT-Fab bispecific antibody, varying in knob-into-hole Fc region mutations, were generated: MAT-Fab (KiH1) and MAT-Fab (KiH2).

To generate the MAT-Fab antibodies, a DNA encoding each polypeptide chain was synthesized *de novo.*

The DNA construct used to generate MAT-Fab antibodies capable of binding CD3 and CD20 encoded the variable and constant domains of parental monoclonal antibodies (mAbs). Each MAT-Fab antibody consisted of four polypeptides as diagrammed below:

| | |
|---|---|
| Heavy chain: | VL_{A}-CL-VH_{B}-CH1-hinge-CH2-CH3 (KiH) |
| First light chain: | VH_{A}-CH1 |
| Second light chain: | VL_{B}-CL |
| Fc chain: | hinge-CH2-CH3 (KiH) |

wherein "(KiH)" indicates the presence of one or more mutations to favor or stabilize CH3 domain heterodimerization of the heavy chain and the Fc chain, antigen "A" is CD20, and antigen "B" is CD3.

Two constructs, designated MAT-Fab (KiH1) and MAT-Fab (KiH2), were made differing only in the knob-into-hole mutations made in the Fc regions to favor heterodimerization.

Briefly, parental mAbs included two high affinity antibodies, an anti-CD20 mAb (ofatumumab) and an anti-CD3 mAb (U.S. Patent Publication No. 2009/0252683).

### Constructs for Heavy Chains of MAT-Fab KiH1 and MAT-Fab KiH2

For producing heavy chains for the MAT-Fab (KiH1) and MAT-Fab (KiH2) bispecific antibodies, a DNA construct encoded a 22-amino acid signal peptide linked to the VL-CL of the light chain of the parental anti-CD20 mAb (ofatumumab), which was linked to the N-terminus of the VH-CH1 region of the parental anti-CD3 mAb (US Patent Publication No. 2009/0252683), which was linked to the hinge-CH2 of the parental anti-CD3 mAb, which was linked to a mutated IgG1 CH3 region derived from the parental anti-CD3 mAb.

The CH3 domain of the heavy chain for the MAT-Fab (KiH1) bispecific antibody was mutated to change a threonine (T) residue to a tyrosine (Y) residue to form a structural knob at residue 21 of the CH3 domain. See, Y₆₁₀ in SEQ ID NO: 1 in Table 1 below (residue underlined), reflecting this mutation. The corresponding position in the CH3 domain of the Fc chain for MAT-Fab (KiH1) was mutated to change a tyrosine (Y) residue to a threonine (T) residue to form a structural knob at residue 62 of the CH3 domain. See, T₂₁₃ in SEQ ID NO:4 in Table 4 below (residue underlined), reflecting this mutation.

The CH3 domain of the heavy chain for the MAT-Fab (KiH2) bispecific antibody was mutated to change a threonine (T) residue to tryptophan (W) residue to form a structural knob in the CH3 domain. See, W₆₁₀ in SEQ ID NO:5 in Table 5 below (residue underlined), reflecting this mutation. The CH3 domain of the Fc chain for MAT-Fab (KiH2) was mutated to change threonine (T) to a serine (S), a leucine (L) to an alanine (A), and a tyrosine (Y) to a valine to form a structural hole in the CH3 domain. See, S₁₇₂, A₁₇₄ and V₂₁₃ in SEQ ID NO:8 in Table 8 below (residues underlined), reflecting this mutations.

The CH3 domain of the heavy chain for the MAT-Fab (KiH2) bispecific antibody was also mutated to change a serine (S) residue to a cysteine (C) residue; and a corresponding mutation was made in the Fc chain for the MAT-Fab (KiH2) bispecific antibody to change a tyrosine (Y) residue to a cystein (C) residue. The introduction of these cysteine (C) residues permits disulfide bond formation with the complementary mutated CH3 domain of the Fc polypeptide chain, resulting in improved stability of the heterodimer. See, C₅₉₈ in SEQ ID NO:5 in Table 5 (residue underlined) and C₁₅₅ in SEQ ID NO:8 in Table 8 below (residue underlined), reflecting these cysteine substitutions.

The heavy chains and Fc chains in both of the MAT-Fab KiH1 and MAT-Fab KiH2 also were mutated to change leucine-leucine at positions 18-19 of the hinge-CH2 region to alanine-alanine to reduce or eliminate ADCC/CDC effector functions (Canfield et al. J. Exp. Med., 173(6): 1483-1491 (1991)). ₄₇₈AA₄₇₉ in SEQ ID NO:1 in Table 1, ₄₀AA₄₁ in SEQ ID NO:4 in Table 4, ₄₇₈AA₄₇₉ in SEQ ID NO:5 in Table 5, and ₄₀AA₄₁ in SEQ ID NO:8 in Table 8, reflect these leucine to alanine mutations.

### Constructs for First Light Chains of MAT-Fab KiH1 and MAT-Fab KiH2

For producing first light chains for the MAT-Fab (KiH1) and MAT-Fab (KiH2) bispecific antibodies, a DNA construct encoded a 19-amino acid signal peptide linked to the VH-CH1 fragment of the parental anti-CD20 mAb (ofatumumab).

### Constructs for Second Light Chains of MAT-Fab KiH1 and MAT-Fab KiH2

For producing second light chains for the MAT-Fab (KiH1) and MAT-Fab (KiH2) bispecific antibodies, a DNA construct encoded a 20-amino acid signal peptide linked to the VL-CL of the light chain of the parental anti-CD3 mAb (US Patent Publication No. 2009/0252683).

### Constructs for Fc Chains of MAT-Fab KiH1 and MAT-Fab KiH2

For producing the Fc polypeptide chains for the MAT-Fab (KiH1) and MAT-Fab (KiH2) bispecific antibodies, a DNA construct encoded a 22 amino acid signal peptide (same as used for the heavy chain constructs) linked to the hinge-CH2 of the parental anti-CD3 mAb, which was linked to a mutated CH3 region of an IgG1 isotype comprising the mutations discussed above in the discussion of MAT-Fab heavy chain constructs.

The CH3 domain of the heavy chain for the Fc chain for the MAT-Fab (KiH1) bispecific antibody was mutated to change a tyrosine (Y) residue to a threonine (T) residue to form a structural hole in the CH3 domain, thereby complementing the structural knob mutation in the CH3 domain of the heavy chain (see, above).

The CH3 domain of the Fc chain for the MAT-Fab (KiH2) bispecific antibody was mutated to change a threonine (T) residue to a serine (S) residue, to change a leucine (L) residue to an alanine (A) residue, and to change a tyrosine (Y) residue to a valine (V) residue to form a structural hole in the CH3 domain. The CH3 domain was also mutated to change a tyrosine (Y) residue to a cysteine (C) residue. The introduction of the cysteine (C) residue permits disulfide bond formation with the complementary mutated CH3 domain of the MAT-Fab (KiH2) heavy chain described above which further stabilizes the heterodimerization.

The amino acid sequences for the four polypeptide chains for the MAT-Fab (KiH1) and MAT-Fab (KiH2) bispecific antibodies are shown in the tables below.

**Table 1. Heavy Chain of CD20/CD3 MAT-Fab (KiH1) Bispecific Antibody**

| | 12345678901234567890123456789012345678901234567890 |
|---|---|
| MAT-Fab (KiH1) Heavy Chain | MDMRVPAQLLGLLLLWFPGSRCEIVLTQSPATLSLSPGERATLSCRASQS VSSYLAWYQQKPGQAPRLLIYDASNRATGIPARFSGSGSGTDFTLTISSL EPEDFAVYYCQQRSNWPITFGQGTRLEIKRTVAAPSVFIFPPSDEQLKSG |
| | TASVVCLLNNFYPREAKVQWKVDNALQSGNSQESVTEQDSKDSTYSLSST LTLSKADYEKHKVYACEVTHQGLSSPVTKSFNRGECEVQLLESGGGLVQP GGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAY WGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPEAAGGPSVFLFPPKPKDTLMISRT PEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRE EMTKNQVSLYCLVKGFYPSDIAVEWESNGQPENNYKTTPPVLDSDGSFFL YSKLTVDKSRWQQGNVFSCSVMHEALHNHYTQKSLSLSPGK (SEQ ID NO:1) |
| signal sequence | residues 1-22 of SEQ ID NO:1 |
| anti-CD20 ofatumumab VL region | residues 23-129 of SEQ ID NO:1 |
| ofatumumb CL | residues 130-236 of SEQ ID NO:1 |
| anti-CD3 VH | residues 237-361 of SEQ ID NO:1 |
| CH1 | residues 362-460 of SEQ ID NO:1 |
| hinge-CH2 | residues 461-588 of SEQ ID NO:1 |
| mutated(KiH1) CH3 | residues 589-691 of SEQ ID NO:1 |

**Table 2. First Light Chain of CD20/CD3 MAT-Fab (KiH1) Bispecific Antibody**

| | 12345678901234567890123456789012345678901234567890 |
|---|---|
| MAT-Fab (KiH1) First Light Chain | MEFGLSWLFLVAILKGVQCEVQLVESGGGLVQPGRSLRLSCAASGFTFND YAMHWVRQAPGKGLEWVSTISWNSGSIGYADSVKGRFTISRDNAKKSLYL QMNSLRAEDTALYYCAKDIQYGNYYYGMDVWGQGTTVTVSSASTKGPSVF PLAPSSKSTSGGTAALGCLVKDYFPEPVTVSWNSGALTSGVHTFPAVLQS SGLYSLSSVVTVPSSSLGTQTYICNVNHKPSNTKVDKKVEPKSC  (SEQ ID NO:2) |
| signal sequence | residues 1-19 of SEQ ID NO:2 |
| anti-CD20 ofatumumab VH region | residues 20-141 of SEQ ID NO:2 |
| ofatumumb CH1 | residues 142-244 of SEQ ID NO:2 |

**Table 3. Second Light Chain of CD20/CD3 MAT-Fab (KiH1) Bispecific Antibody**

| | 12345678901234567890123456789012345678901234567890 |
|---|---|
| MAT-Fab (KiH1) Second Light Chain | MTWTPLLFLTLLLHCTGSLSELVVTQEPSLTVSPGGTVTLTCRSSTGAVT TSNYANWVQQKPGQAPRGLIGGTNKRAPGTPARFSGSLLGGKAALTLSGV QPEDEAEYYCALWYSNLWVFGGGTKLTVLGQPKAAPSVTLFPPSSEELQA NKATLVCLISDFYPGAVTVAWKADSSPVKAGVETTTPSKQSNNKYAASSY LSLTPEQWKSHRSYSCQVTHEGSTVEKTVAPTECS (SEQ ID NO:3) |
| signal sequence | residues 1-20 of SEQ ID NO:3 |
| anti-CD3 VL region | residues 21-129 of SEQ ID NO:3 |
| anti-CD3 CL | residues 130-235 of SEQ ID NO:3 |

**Table 4. CD20/CD3 MAT-Fab (KiH1) Fc polypeptide chain**

| | 12345678901234567890123456789012345678901234567890 |
|---|---|
| MAT-Fab (KiH1) Fc Polypeptide Chain | MDMRVPAQLLGLLLLWFPGSRCPKSCDKTHTCPPCPAPEAAGGPSVFLFP PKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPR EPQVYTLPPSREEMTKNQVSLTCLVKGFYPSDIAVEWESNGQPENNYKTT PPVLDSDGSFFLTSKLTVDKSRWQQGNVFSCSVMHEALHNHYTQKSLSLS PGK (SEQ ID NO:4) |
| signal sequence | residues 1-22 of SEQ ID NO:4 |
| hinge-CH2 | residues 23-150 of SEQ ID NO:4 |
| mutated (KiH1) CH3 | residues 151-253 of SEQ ID NO:4 |

**Table 5. Heavy Chain of CD20/CD3 MAT-Fab (KiH2) Bispecific Antibody**

| | 12345678901234567890123456789012345678901234567890 |
|---|---|
| MAT-Fab (KiH2) Heavy Chain | MDMRVPAQLLGLLLLWFPGSRCEIVLTQSPATLSLSPGERATLSCRASQS VSSYLAWYQQKPGQAPRLLIYDASNRATGIPARFSGSGSGTDFTLTISSL EPEDFAVYYCQQRSNWPITFGQGTRLEIKRTVAAPSVFIFPPSDEQLKSG TASVVCLLNNFYPREAKVQWKVDNALQSGNSQESVTEQDSKDSTYSLSST LTLSKADYEKHKVYACEVTHQGLSSPVTKSFNRGECEVQLLESGGGLVQP GGSLKLSCAASGFTFNTYAMNWVRQAPGKGLEWVARIRSKYNNYATYYAD SVKDRFTISRDDSKNTAYLQMNNLKTEDTAVYYCVRHGNFGNSYVSWFAY WGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKP SNTKVDKKVEPKSCDKTHTCPPCPAPEAAGGPSVFLFPPKPKDTLMISRT PEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPCRE EMTKNQVSLWCLVKGFYPSDIAVEWESNGQPENNYKTTPPVLDSDGSFFL YSKLTVDKSRWQQGNVFSCSVMHEALHNHYTQKSLSLSPGK (SEQ ID NO:5) |
| signal sequence | residues 1-22 of SEQ ID NO:5 |
| anti-CD20 ofatumumab VL region | residues 23-129 of SEQ ID NO:5 |
| ofatumumb CL | residues 130-236 of SEQ ID NO:5 |
| anti-CD3 VH | residues 237-361 of SEQ ID NO:5 |
| CH1 | residues 362-460 of SEQ ID NO:5 |
| hinge-CH2 | residues 461-588 of SEQ ID NO:5 |
| mutated(KiH2) CH3 | residues 589-691 of SEQ ID NO:5 |

**Table 6. First Light Chain of CD20/CD3 MAT-Fab (KiH2) Bispecific Antibody**

| | 12345678901234567890123456789012345678901234567890 |
|---|---|
| MAT-Fab (KiH2) First Light Chain | MEFGLSWLFLVAILKGVQCEVQLVESGGGLVQPGRSLRLSCAASGFTFND YAMHWVRQAPGKGLEWVSTISWNSGSIGYADSVKGRFTISRDNAKKSLYL QMNSLRAEDTALYYCAKDIQYGNYYYGMDVWGQGTTVTVSSASTKGPSVF PLAPSSKSTSGGTAALGCLVKDYFPEPVTVSWNSGALTSGVHTFPAVLQS SGLYSLSSVVTVPSSSLGTQTYICNVNHKPSNTKVDKKVEPKSC (SEQ ID NO:6) |
| signal sequence | residues 1-19 of SEQ ID NO:6 |
| anti-CD20 ofatumumab VH region | residues 20-122 of SEQ ID NO:6 |
| ofatumumb CH1 | residues 123-244 of SEQ ID NO:6 |

**Table 7. Second Light Chain of CD20/CD3 MAT-Fab (KiH2) Bispecific Antibody**

| | |
|---|---|
| | 12345678901234567890123456789012345678901234567890 |
| MAT-Fab (KiH2) | MTWTPLLFLTLLLHCTGSLSELVVTQEPSLTVSPGGTVTLTCRSSTGAVT TSNYANWVQQKPGQAPRGLIGGTNKRAPGTPARFSGSLLGGKAALTLSGV QPEDEAEYYCALWYSNLWVFGGGTKLTVLGQPKAAPSVTLFPPSSEELQA NKATLVCLISDFYPGAVTVAWKADSSPVKAGVETTTPSKQSNNKYAASSY LSLTPEQWKSHRSYSCQVTHEGSTVEKTVAPTECS (SEQ ID NO:7) |
| Second Light Chain | |
| signal sequence | residues 1-20 of SEQ ID NO:7 |
| anti-CD3 VL region | residues 21-129 of SEQ ID NO:7 |
| anti-CD3 CL | residues 130-235 of SEQ ID NO:7 |

**Table 8. CD20/CD3 MAT-Fab (KiH2) Fc polypeptide chain**

| | |
|---|---|
| | 12345678901234567890123456789012345678901234567890 |
| MAT-Fab (KiH2) | MDMRVPAQLLGLLLLWFPGSRCPKSCDKTHTCPPCPAPEAAGGPSVFLFP PKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPR EPQVCTLPPSREEMTKNQVSLSCAVKGFYPSDIAVEWESNGQPENNYKTT PPVLDSDGSFFLVSKLTVDKSRWQQGNVFSCSVMHEALHNHYTQKSLSLS PGK (SEQ ID NO:8) |
| Fc Polypeptide Chain | |
| signal sequence | residues 1-22 of SEQ ID NO:8 |
| hinge-CH2 | residues 23-150 of SEQ ID NO:8 |
| mutated (KiH2) CH3 | residues 151-253 of SEQ ID NO:8 |

### Expression Levels

The DNA constructs described above encoding each of the four polypeptide chains for MAT-Fab (KiH1) and MAT-Fab (KiH2) were cloned into the pTT expression vector by standard methods. The resulting recombinant pTT vectors encoding SEQ ID NOs: 1-4 or encoding SEQ ID NOs:5-8 were then co-transfected into HEK 293E cells for expression of the respective MAT-Fab (KiH1) and the MAT-Fab (KiH2) bispecific antibodies. The levels of expression in cell cultures are shown in the table below.

**Table 9.**

| Construct | Level of Expression |
|---|---|
| MAT-Fab (KiH1) | 20.6 mg/L |
| MAT-Fab (KiH2) | 36.7 mg/L |

### Purification and Characterization

Once the production phase was completed, cell cultures were collected and subjected to protein purification by Protein A affinity chromatography.

The monomeric fraction of each of the MAT-Fab antibody preparations after a single step purification by Protein A chromatography was determined by size exclusion chromatograph (SEC). The results of the SEC analyses are shown in Figures 2 and 3.

The SEC analysis revealed that 98.19% of the MAT-Fab (KiH1) antibody preparation and 95.7% of the MAT-Fab (KiH2) antibody preparation was present as a single species ("monomeric faction").

### Fluorescence-Activated Cell Sorting (FACS)

The ability of purified MAT-Fab (KiH1) and MAT-Fab (KiH2) to bind the CD20 and CD3 target antigens expressed on the surface of cells was examined by fluorescence activated cell sorting (FACS) using the protocols described below.

### Equipment

BD FACSVerse serial number: 01131005894
Olympus CKX41 serial number: 01121005367
Eppendorf centrifuge 5810R serial number: 01121005414
Thermo Series II water jacket, serial number: 01121005408

### Materials and Reagents

BD Falcon Round-Bottom Tube, Cat.No.352052 Lot.No.3070549
Tissue Culture Plate 96 Well, U Bottom With Low Evaporation, Cat.No.353077 Lot.No.34285048 FBS, GIBCO Cat. No.10099, Lot.1652792
PBS, GIBCO Cat. No.10010, Lot. 1710584
RPMI Medium 1640(1×), GIBCO Cat. No. A10491, Lot. No. 1747206
Alexa Fluor^{®} 488 mouse anti-human lgG1. Invitrogen, Cat.No.A-10631, Lot.1744792

### Cell Lines

Jurkat, ATCC Cat. No. TIB-152, is a T cell leukemia cell line expressing CD3 surface antigen. Raji, ATCC Cat. No. CCL-86, lot 60131961, is a Burkitt's lymphoma B cell line expressing CD20 surface antigen.

### Antibodies for FACS

| Antibody Descriptions | Target | Molecular Weight (kilodaltons) | mg/ml |
|---|---|---|---|
| anti-RAC human IgG1 | small molecule | 150 | 1.44 |
| MAT-Fab (KiH1) | CD20 and CD3 | 150 | 2.19 |
| MAT-Fab (KiH2) | CD20 and CD3 | 150 | 2 |

### FACS Procedure

1. Aliquoted 5×10⁵ cells in ice-cold PBS.
2. Cells were blocked in 2% FBS/PBS for 30 minutes on ice.
3. Cells were incubated with anti-CD20 antibodies for 1h on ice. The initial concentration of antibody was (133.33 nM) 20ug/ml and was diluted 1:5 serially. Each volume was 200 µL.
4. Washed in PBS for 3 times, 2000 rpm for 5 min at 4°C.
5. Cells were incubated with Mouse anti-Human IgG1 Fc Secondary Antibody, Alexa Fluor^{®} 488 (AF488) mouse anti-human lgG1 100 µL (10µg/mL) (1:100 diluted) for 1 hour on ice and kept from light.
6. Washed in PBS for 3 times, 2000 rpm for 5 min at 4°C.
7. Cells were re-suspended in PBS. Mean Fluorescence Intensity (MFI) was detected by BD FACSVerse flow cytometer.

### Results

The results are shown in Figures 4 and 5. Both MAT-Fab bispecific antibodies were able to bind CD20 and CD3 on B cells (CD20 Raji cells) and T cells (CD3 Jurkat cells), respectively.

### Functional Activity to Induce B Cell Apoptosis in the Presence of T Cells

The ability of purified MAT-Fab (KiH1) and MAT-Fab (KiH2) to bind the CD20 and CD3 target antigens expressed on cell surfaces was examined by in a B cell apoptosis assay using the protocols described below.

### Equipment

BD FACSVerse serial number: 01131005894
Olympus CKX41 serial number: 01121005367
Eppendorf centrifuge 5810R serial number: 01121005414
Thermo Series II water jacket, serial number: 01121005408

### Materials and Reagents

BD Falcon Round-Bottom Tube, Cat.No.352052 Lot.No.3070549
Tissue Culture Plate 96 Well, U Bottom with Low Evaporation, Cat.No.353077 Lot.No.4292046 FBS, GIBCO Cat. No.10099, Lot.1652792
PBS, GIBCO Cat. No.10010, Lot.1710584
RPMI Medium 1640(1×), GIBCO Cat. No. 11875, Lot. No. 1731226
Ficoll Paque Plus, GE HEALTHCARE, cat: GE17144002, lot:10237843
PE Mouse Anti-Human CD19, BD Pharmingen, cat.555413, lot: 5274713

### Cell Lines

Raji, ATCC Cat. No. CCL-86, lot 60131961.
Donor number: 00123

### Antibodies for B Cell Apoptosis Assay

| Antibody Name | Lot No. | Antibody Specificity |
|---|---|---|
| MAT-Fab (KiH1) | 160411002 | CD20/CD3 |
| Ofatumumab | 20140225B | CD20 |
| CD3 mAb | Pr2016012817 | CD3 |
| MAT-Fab (KiH2) | 160429004 | CD20/CD3 |

### Procedures

### Procedure for isolation of mononuclear cells

### Preparation of the blood sample

1. 100 ml of human blood was freshly extracted to anticoagulant-treated tubes.
2. An equal volume of RPMI1640 (100 ml) was added to the blood and mixed gently.
3. Ficoll-Paque density gradient media was warmed to 18°C to 20°C before use.
4. Ficoll-Paque media bottle was inverted several times to ensure thorough mixing.
5. Ficoll-Paque media (15 ml) was added to 50ml centrifuge tube.
6. Diluted blood sample (20 ml) was carefully layered onto the Ficoll-Paque media solution without mixing the Ficoll-Paque media solution and the diluted blood sample.
7. The Ficoll-Paque with blood sample was centrifuged at 400g for 30 to 40 min at 18°C to 20°C.
8. The layer of mononuclear cells was transferred from the gradient to a sterile centrifuge tube.

### Washing the cell isolate

1. The volume of the transferred mononuclear cells was estimated and at least 3 volumes of PBS were added to the mononuclear cells in the centrifuge tube.
2. The cells and PBS were centrifuged at 400 to 500 x g for 10 min at 18°C to 20°C and the supernatant removed.
3. The washing was repeated.
4. The supernatant was removed, and the cell pellet resuspended in assay buffer.

### B cell depletion

Assay medium was RPMI1640 plus 10% FBS
1. Target cells (Raji cells) were harvested in logarithmic growth phase and washed once with assay medium. The cell density was adjusted to 5 × 10⁵ cells/ml, and 100 µl of the cell suspension was applied to each well of an assay plate.
2. The testing antibody was serially diluted, and 50 µl was added to each well of the above assay plate. The final starting concentration of antibody was 50 µg/ml and then serially diluted.
3. The PBMC cell density was adjusted to 2.5 × 10⁶ cells/ml and 100 µl of the cell suspension was applied to each well of an assay plate (effector to target ratio 5:1)
4. Assay plates were incubated at 37°C with 5% CO₂ for 1 day, 2 days, and 3 days. At 1 day, 2 days, and 3 days, the samples were collected by centrifuging at 2000 rpm for 5 min at 4°C, and washed once with PBS.
5. Each sample was incubated with 50 µl of 1:5 diluted PE-conjugated anti-human CD19 detection antibody for 30 minutes on ice.
6. The samples were washed with PBS and test by FACSVerse.

### Results

The results are shown in Figure 6. By day 2 of the cell depletion assay, both MAT-Fab bispecific antibodies were able to induce T cell-mediated apoptosis of B cells.

## Claims

1. A monovalent asymmetric tandem Fab bispecific antibody (MAT-Fab antibody) comprising polypeptide chains (a), (b), (c) and (d), wherein:
(a) is a heavy polypeptide chain (heavy chain), wherein said heavy chain comprises (from amino to carboxyl terminus): VL_{A}-CL-VH_{B}-CH1-hinge-CH2-CH3m1, wherein:
VL_{A} is a human immunoglobulin light chain variable domain that is fused directly to CL, which is a human light chain constant domain, wherein VL_{A}-CL is an immunoglobulin light chain component of a first Fab binding unit recognizing a first antigen or epitope and is fused directly to VH_{B}, wherein VH_{B} is a human immunoglobulin heavy chain variable domain that is fused directly to CH1, which is a human immunoglobulin heavy chain CH1 constant domain, wherein VH_{B}-CH1 is the immunoglobulin heavy chain component of a second Fab binding unit recognizing a second antigen or epitope, and wherein VH_{B}-CH1 is fused directly to a hinge-CH2, wherein hinge-CH2 is the hinge-CH2 region of an immunoglobulin heavy chain and wherein the hinge-CH2 is fused directly to CH3m1, which is a first human immunoglobulin heavy chain CH3 constant domain that has been mutated with one or more knobs-into-holes (KiH) mutations to form a structural knob or structural hole in said CH3m1 constant domain, wherein A and B are different antigens or different epitopes;
(b) is a first MAT-Fab light chain comprising VH_{A}-CH1, wherein VH_{A} is a human immunoglobulin heavy chain variable domain that is fused directly to CH1, which is a human immunoglobulin heavy chain CH1 constant domain, and wherein VH_{A}-CH1 is the immunoglobulin heavy chain component of said first Fab binding unit;
(c) is a second MAT-Fab light chain comprising VL_{B}-CL, wherein VL_{B} is a human immunoglobulin light chain variable domain that is fused directly to CL, which is a human immunoglobulin light chain CL domain, and wherein VL_{B}-CL is the immunoglobulin light chain component of said second Fab binding unit; and
(d) is an Fc polypeptide chain (Fc chain) comprising hinge-CH2-CH3m2, wherein hinge-CH2 is the hinge-CH2 region of an immunoglobulin heavy chain and wherein the hinge-CH2 is fused directly to CH3m2, which is a second human immunoglobulin heavy chain CH3 constant domain that has been mutated with one or more knobs-into-holes (KiH) mutations to form a structural knob or structural hole in said CH3m2 constant domain;
with the proviso that:
when the CH3m1 domain of the heavy chain has been mutated to form a structural knob, then the CH3m2 domain of the Fc chain has been mutated to form a complementary structural hole to favor pairing of the CH3m1 domain with the CH3m2 domain; and
when the CH3m1 domain of said heavy chain has been mutated to form a structural hole, then the CH3m2 domain of the Fc chain has been mutated to form a complementary structural knob to favor pairing of the CH3m1 domain with the CH3m2 domain; and
said MAT-Fab antibody optionally comprises a mutation in the CH3m1 domain and the CH3m2 domain to introduce a cysteine residue to favor disulfide bond formation in pairing the CH3m1 domain with the CH3m2 domain.

2. The MAT-Fab antibody according to Claim 1, wherein the one or more KiH mutations in the CH3m1 domain or the CH3m2 domain to form a structural knob is a change from a threonine residue to a tyrosine residue or a change of a threonine residue to a tryptophan residue,
preferably:
(i) wherein the KiH mutation changes a threonine to a tyrosine at position 610 of SEQ ID NO: 1 of the CH3 domain of the heavy chain of the MAT-Fab antibody, or
(ii) wherein the KiH mutation changes a threonine to a tryptophan at position 610 of SEQ ID NO:5 of the CH3 domain of the heavy chain of the MAT-Fab antibody.

3. The MAT-Fab antibody according to Claim 1, wherein a mutation in the CH3m1 domain or the CH3m2 domain to form a structural hole is a change of a tyrosine residue to a threonine residue or a combination of a change of a threonine residue to a serine residue, a change of a leucine residue to an alanine residue, and a change of a tyrosine residue to a valine residue;
preferably
(i) wherein the mutation to change a tyrosine residue to a threonine residue changes tyrosine to threonine at position 213 of SEQ ID NO:4 of the CH3 domain of the Fc chain of the MAT-Fab antibody,
or
(ii) wherein the combination of a change of a threonine residue to a serine residue, a change of a leucine residue to an alanine residue, and a change of a tyrosine residue to a valine residue is a combination of a change of a threonine to a serine at position 172 of SEQ ID NO:8, a change of a leucine to an alanine at position 174 of SEQ ID NO:8, and a change of a tyrosine to a valine at position 213 of SEQ ID NO:8 of the CH3 domain of the Fc chain of the MAT-Fab antibody.

4. The MAT-Fab antibody according to any one of Claims 1-3, wherein each of the CH3m1 domain and the CH3m2 domain further comprises a mutation to replace an amino acid residue with a cysteine to promote a disulfide bond formation between the CH3m1 and CH3m2 domains, preferably
(i) wherein the mutation is a change of a serine to a cysteine or a change of a tyrosine to a cysteine; or
(ii) wherein the mutation is a change of a serine residue to a cysteine residue at position 598 of SEQ ID NO: 5 of the CH3 domain of the heavy chain of the MAT-Fab antibody, and/or wherein the mutation is a change of a tyrosine residue to a cysteine residue at position 155 of SEQ ID NO:8 of the CH3 domain of the Fc chain of the MAT-Fab antibody.

5. The MAT-Fab antibody according to any one of Claims 1 to 4, wherein the CH2 domains of the heavy chain (a) and the Fc chain (d) each comprises one or more mutations to reduce or eliminate at least one Fc effector function,
preferably wherein the CH2 domain of the heavy chain and the CH2 domain of the Fc chain each comprises two mutations to change leucine234 to alanine and to change leucine235 to alanine (EU numbering).

6. The MAT-Fab antibody according to Claim 1, comprising:
(a) a heavy chain comprising the amino acid sequence of amino acid residues 23 - 691 of SEQ ID NO: 1,
(b) a first light chain comprising the amino acid sequence of amino acid residues 20 - 244 of SEQ ID NO:2,
(c) a second light chain comprising the amino acid sequence of amino acid residues 21 - 235 of SEQ ID NO:3, and
(d) an Fc chain comprising the amino acid sequence of amino acid residues 23 - 253 of SEQ ID NO:4;
or
(a) a heavy chain comprising the amino acid sequence of amino acid residues 23 - 691 of SEQ ID NO:5,
(b) a first light chain comprising the amino acid sequence of amino acid residues 20 - 244 of SEQ ID NO:6,
(c) a second light chain comprising the amino acid sequence of amino acid residues 21 - 235 of SEQ ID NO:7, and
(d) an Fc chain comprising the amino acid sequence of amino acid residues 23 - 253 of SEQ ID NO:8.

7. The MAT-Fab antibody according to any one of Claims 1-5, wherein the MAT-Fab antibody binds two different target antigens or two different epitopes, preferably wherein the two different target antigens or two different epitopes are selected from the group of antigen pairs consisting of: CD20 and CD3, CD3 and CD19, CD3 and Fc-gamma-RIIIA, CD3 and TPBG, CD3 and Epha10, CD3 and IL-5Rα, CD3 and TASCTD-2, CD3 and CLEC12A, CD3 and Prominin-1, CD3 and IL-23R, CD3 and ROR1, CD3 and IL-3Rα, CD3 and PSA, CD3 and CD8, CD3 and Glypican 3, CD3 and FAP, CD3 and EphA2, CD3 and ENPP3, CD3 and CD33, CD3 and CD133, CD3 and EpCAM, CD3 and CD19, CD3 and Her2, CD3 and CEA, CD3 and GD2, CD3 and PSMA, CD3 and BCMA, CD3 and A33, CD3 and B7-H3, CD3 and EGFR, CD3 and P-cadherin, CD3 and HMW-MAA, CD3 and TIM-3, CD3 and CD38, CD3 and TAG-72, CD3 and SSTR, CD3 and FRA, CD16 and CD30, CD64 and Her2, CD 137 and CD20, CD138 and CD20, CD19 and CD20, CD38 and CD20, CD20 and CD22, CD40 and CD20, CD47 and CD20, CD 137 and EGFR, CD137 and Her-2, CD 137 and PD-1, CD 137 and PDL-1, PD-1 and PD-L1, VEGF and PD-L1, Lag-3 and TIM- 3, OX40 and PD-1, TIM-3 and PD-1, TIM-3 and PDL-1, EGFR and DLL-4, VEGF and EGFR, HGF and VEGF, a first epitope of VEGF and a different second epitope of VEGF, VEGF and Ang2, EGFR and cMet, PDGF and VEGF, VEGF and DLL-4, OX40 and PD-L1, ICOS and PD-1, ICOS and PD-L1, Lag-3 and PD-1, Lag-3 and PD-L1, Lag-3 and CTLA-4, ICOS and CTLA- 4, CD138 and CD40, CD38 and CD138, CD38 and CD40, CD-8 and IL-6, CSPGs and RGM A, CTLA-4 and BTN02, CTLA-4 and PD-1, IGF1 and IGF2, IGF1/2 and Erb2B, IGF-IR and EGFR, EGFR and CD13, IGF-IR and ErbB3, EGFR-2 and IGFR, a first epitope of Her2 and a second different epitope of Her2, Factor IXa and Met, Factor X and Met, VEGFR-2 and Met, VEGF-A and Angiopoietin-2 (Ang-2), IL-12 and TWEAK, IL-13 and IL-1β, MAG and RGM A, NgR and RGM A, NogoA and RGM A, OMGp and RGM A, PDL-1 and CTLA-4, PD-1 and TIM-3, RGM A and RGM B, Te38 and TNFα, TNFα and Blys, TNFα and CD-22, TNFα and a CTLA-4, TNFα and GP130, TNFα and IL-12p40, and TNFα and RANK ligand.

8. The MAT-Fab antibody according to any one of Claims 1-6, wherein one of the two different target antigens bound by the MAT-Fab antibody is an antigen,
wherein said antigen is preferably selected from the group consisting of: CD3, CD16, and CD64, expressed on the surface of an effector cell, and
wherein said effector cell is preferably selected from the group consisting of: a T cell, a natural killer (NK) cell, a monocyte, a neutrophil and a macrophage, and
wherein the other target antigen bound by the MAT-Fab antibody is a disorder-associated antigen expressed on the surface of a target cell that is considered detrimental to a human subject, and
wherein said detrimental target cell is preferably selected from the group consisting of: a tumor cell, an auto-reactive cell, and a virus infected cell.

9. The MAT-Fab antibody according to Claim 8, wherein the disorder-associated antigen expressed on the surface of the detrimental target cell is a tumor-associated antigen expressed on a tumor cell,
preferably:
(i) wherein the tumor-associated antigen is selected from the group consisting of: CD19, CD20, human epidermal growth factor receptor 2 (HER2), carcinoembryonic antigen (CEA), epithelial cell adhesion molecule (EpCAM), and receptor tyrosine kinase-like orphan receptor 1 (ROR 1); and/or
(ii) wherein said tumor cell is a malignant B cell and more preferably wherein said malignant B cell is a cell of a cancer disorder selected from the group consisting of: acute lymphoblastic leukemia, Hodgkin's lymphoma, non-Hodgkin's lymphoma (NHL), precursor B cell lymphoblastic leukemia/lymphoma, mature B cell neoplasms, B cell chronic lymphocytic leukemialsmall lymphocytic lymphoma, B cell prolymphocytic leukemia, lymphoplasmacytic lymphoma, mantle cell lymphoma, follicular lymphoma, cutaneous follicle center lymphoma, marginal zone B cell lymphoma, hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma.

10. The MAT-Fab antibody according to any one of Claims 1-9, conjugated to an agent selected from the group consisting of: a therapeutic agent, an imaging agent, and a cytotoxic agent, preferably
(i) wherein the imaging agent is selected from the group consisting of: a radiolabel, an enzyme, a fluorescent label, a luminescent label, a bioluminescent label, a magnetic label, biotin, streptavidin, and avidin,
and
(ii) wherein the therapeutic or cytotoxic agent is selected from the group consisting of: an antimetabolite, an alkylating agent, an antibiotic, a growth factor, a cytokine, an anti-angiogenic agent, an anti-mitotic agent, an anthracycline, a toxin, and an apoptotic agent.

11. A pharmaceutical composition comprising a MAT-Fab antibody according to any one of Claims 1-9 and a pharmaceutically acceptable carrier, wherein said pharmaceutical composition is prepared for parenteral or non-parenteral administration to an individual, and
preferably for at least one mode of administration selected from the group consisting of: parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, and transdermal.

12. An isolated polynucleotide or isolated polynucleotides encoding all four of the polypeptides of a MAT-Fab antibody according to any one of Claims 1-9.

13. A vector comprising the isolated polynucleotide or isolated polynucleotides according to Claim 12, preferably wherein said vector is selected from the group consisting of: pcDNA, pTT pTT3, pEFBOS, pBV, pJV, pcDNA3.1 TOPO, pEF6 TOPO, and pBJ.

14. An isolated host cell comprising a vector according to Claim 13.

15. An isolated host cell according to Claim 14, which is a mammalian host cell.

16. A method of producing a MAT-Fab antibody comprising culturing a host cell described in Claim 14 or Claim 15 in culture medium under conditions sufficient to produce the MAT-Fab antibody.

17. A MAT-Fab antibody produced according to the method of Claim 16.

18. A MAT-Fab antibody according to any one of Claims 1-9 and 17 or a pharmaceutical composition according to Claim 11 for use as a medicament.

19. A MAT-Fab antibody according to any one of Claims 1-9 and 17 or a composition according to Claim 11 for use in therapy of a disease or disorder in an individual, wherein the MAT-Fab antibody binds an epitope or antigen expressed on the surface of a cancer cell, an auto-reactive cell, or a virus-infected cell, wherein binding of the MAT-Fab antibody to the cancer cell, an auto-reactive cell, or a virus-infected cell provides a treatment for the disease or disorder.

20. A MAT-Fab antibody according to any one of Claims 1-9 and 17 or a composition according to Claim 11 for use in a method of treating a human subject for a malignant B cell tumor wherein said method comprises administering to the individual in need of such treatment said MAT-Fab antibody that binds an antigen on an effector cell, wherein preferably the effector cell is selected from the group consisting of: a T cell, a natural killer (NK) cell, a monocyte, a neutrophil, and a macrophage, and that binds a cell surface antigen on a malignant B cell,
preferably wherein said MAT-Fab antibody binds an antigen on malignant B cells of a cancer disorder selected from the group consisting of: acute lymphoblastic leukemia, Hodgkin's lymphoma, non-Hodgkin's lymphoma (NHL), precursor B cell lymphoblastic leukemia/lymphoma, mature B cell neoplasms, B cell chronic lymphocytic leukemialsmall lymphocytic lymphoma, B cell prolymphocytic leukemia, lymphoplasmacytic lymphoma, mantle cell lymphoma, follicular lymphoma, cutaneous follicle center lymphoma, marginal zone B cell lymphoma, hairy cell leukemia, diffuse large B cell lymphoma, Burkitt's lymphoma, plasmacytoma, plasma cell myeloma, post-transplant lymphoproliferative disorder, Waldenstrom's macroglobulinemia, and anaplastic large-cell lymphoma.

## Patentansprüche

1. Ein bispezifischer monovalenter asymmetrischer Tandem-Fab-Antikörper (MAT-Fab-Antikörper), umfassend Polypeptidketten (a), (b), (c) und (d), wobei:
(a) eine schwere Polypeptidkette (schwere Kette) ist, wobei diese schwere Kette umfasst (vom Amino- zum Carboxyl-Rest): VL_{A}-CL-VH_{B}-CH1-Gelenk-CH2-CH3m1, wobei: VLa, eine variable Domäne einer humanen Immunglobulin-Leichtkette ist, die direkt an CL fusioniert ist, welches eine konstante Domäne einer humanen Leichtkette ist, wobei VL_{A}-CL eine Immunglobulin-Leichtkettenkomponente einer ersten Fab-Bindungseinheit ist, die ein erstes Antigen oder Epitop erkennt, und die direkt an VH_{B} gebunden ist, wobei VH_{B} eine variable Domäne einer humanen Immunglobulin-Schwerkette ist, die direkt an CH1 fusioniert ist, welches eine konstante Domäne CH1 einer humanen Immunglobulin-Schwerkette ist, wobei VH_{B}-CH1 die Immunglobulin-Schwerkettenkomponente einer zweiten Fab-Bindungseinheit ist, die ein zweites Antigen oder Epitop erkennt, und wobei VH_{B}-CH1 direkt an ein Gelenk-CH2 fusioniert ist, wobei Gelenk-CH2 die Gelenk-CH2-Region einer Immunglobulin-Schwerkette ist, und wobei das Gelenk-CH2 direkt an CH3m1 fusioniert ist, welches eine erste konstante Domäne CH3 einer humanen Immunglobulin-Schwerkette ist, die mit einer oder mehreren Noppen-in-Löcher-Mutationen (Knobs-into-Holes, KiH) mutiert wurde, um eine Strukturnoppe oder ein Strukturloch in dieser konstanten Domäne CH3m1 zu bilden, wobei A und B verschiedene Antigene oder verschiedene Epitope sind;
(b) eine erste MAT-Fab-Leichtkette ist, die VH_{A}-CH1 umfasst, wobei VH_{A} eine variable Domäne einer humanen Immunglobulin-Schwerkette ist, die direkt an CH1 fusioniert ist, welches eine konstante Domäne CH1einer humanen Immunglobulin-Schwerkette ist, und wobei VH_{A}-CH1 die Immunglobulin-Schwerkettenkomponente der ersten Fab-Bindungseinheit ist;
(c) eine zweite MAT-Fab-Leichtkette ist, die VL_{B}-CL umfasst, wobei VL_{B} eine variable Domäne einer humanen Immunglobulin-Leichtkette ist, die direkt an CL fusioniert ist, welches eine CL-Domäne einer humanen Immunglobulin-Leichtkette ist, und wobei VL_{B}-CL die Immunglobulin-Leichtkettenkomponente der zweiten Fab-Bindungseinheit ist; und
(d) eine Fc-Polypeptidkette (Fc-Kette) ist, die Gelenk-CH2-CH3m2 umfasst, wobei Gelenk-CH2 die Gelenk-CH2-Region einer Immunglobulin-Schwerkette ist, und wobei das Gelenk-CH2 direkt an CH3m2 fusioniert ist, welches eine zweite konstante Domäne CH3 einer humanen Immunglobulin-Schwerkette ist, die mit einer oder mehreren Noppen-in-Löcher-Mutationen (Knobs-into-Holes, KiH) mutiert wurde, um eine Strukturnoppe oder ein Strukturloch in dieser konstanten Domäne CH3m2 zu bilden;
mit der Maßgabe, dass:
wenn die CH3m1-Domäne der schweren Kette mutiert wurde, um eine Strukturnoppe zu bilden, die CH3m2-Domäne der Fc-Kette mutiert wurde, um ein komplementäres Strukturloch zu bilden, so dass die Paarung der CH3m1-Domäne mit der CH3m2-Domäne begünstigt wird; und
wenn die CH3m1-Domäne der schweren Kette mutiert wurde, um ein Strukturloch zu bilden, die CH3m2-Domäne der Fc-Kette mutiert wurde, um eine komplementäre Strukturnoppe zu bilden, so dass die Paarung der CH3m1-Domäne mit der CH3m2-Domäne begünstigt wird; und
wobei der MAT-Fab-Antikörper gegebenenfalls eine Mutation in der CH3m1-Domäne und der CH3m2-Domäne umfasst, um einen Cystein-Rest einzuführen, so dass die Disulfidbindungsbildung bei der Paarung der CH3m1-Domäne mit der CH3m2-Domäne begünstigt wird.

2. Der MAT-Fab-Antikörper gemäß Anspruch 1, wobei die ein oder mehreren KiH-Mutationen in der CH3m1-Domäne oder der CH3m2-Domäne zur Bildung einer Strukturnoppe einen Wechsel von einem Threonin-Rest zu einem Tyrosin-Rest oder ein Wechsel von einem Threonin-Rest zu einem Tryptophan-Rest ist,
vorzugsweise:
(i) wobei die KiH-Mutation ein Threonin in ein Tyrosin an Position 610 von SEQ ID NO:1 der CH3-Domäne der schweren Kette des MAT-Fab-Antikörpers abändert, oder
(ii) wobei die KiH-Mutation ein Threonin in ein Tryptophan an Position 610 von SEQ ID NO:5 der CH3-Domäne der schweren Kette des MAT-Fab-Antikörpers abändert.

3. Der MAT-Fab-Antikörper gemäß Anspruch 1, wobei eine Mutation in der CH3m1-Domäne oder der CH3m2-Domäne zur Bildung eines Strukturloches ein Wechsel von einem Tyrosin-Rest zu einem Threonin-Rest oder eine Kombination aus einem Wechsel von einem Threonin-Rest zu einem Serin-Rest, einem Wechsel von einem Leucin-Rest zu einem Alanin-Rest und einem Wechsel von einem Tyrosin-Rest zu einem Valin-Rest ist; vorzugsweise:
(i) wobei die Mutation zum Wechsel von einem Tyrosin-Rest zu einem Threonin-Rest Tyrosin in Threonin an Position 213 von SEQ ID NO:4 der CH3-Domäne der Fc-Kette des MAT-Fab-Antikörpers abändert,
oder
(ii) wobei die Kombination aus einem Wechsel von einem Threonin-Rest zu einem Serin-Rest, einem Wechsel von einem Leucin-Rest zu einem Alanin-Rest und einem Wechsel von einem Tyrosin-Rest zu einem Valin-Rest eine Kombination aus einem Wechsel von einem Threonin zu einem Serin an Position 172 von SEQ ID NO:8, einem Wechsel von einem Leucin zu einem Alanin an Position 174 von SEQ ID NO:8 und einem Wechsel von einem Tyrosin zu einem Valin an Position 213 von SEQ ID NO:8 der CH3-Domäne der Fc-Kette des MAT-Fab-Antikörpers ist.

4. Der MAT-Fab-Antikörper gemäß einem der Ansprüche 1-3, wobei die CH3m1-Domäne und die CH3m2-Domäne jeweils ferner eine Mutation umfassen, um einen Aminosäurerest durch ein Cystein zu ersetzen, so dass eine Disulfidbindungsbildung zwischen den CH3m1- und CH3m2-Domänen begünstigt wird,
vorzugsweise
(i) wobei die Mutation ein Wechsel von einem Serin zu einem Cystein oder ein Wechsel von einem Tyrosin zu einem Cystein ist; oder
(ii) wobei die Mutation ein Wechsel von einem Serin-Rest zu einem Cystein-Rest an Position 598 von SEQ ID NO: 5 der CH3-Domäne der schweren Kette des MAT-Fab-Antikörpers ist, und/oder wobei die Mutation ein Wechsel von einem Tyrosin-Rest zu einem Cystein-Rest an Position 155 von SEQ ID NO:8 der CH3-Domäne der Fc-Kette des MAT-Fab-Antikörpers ist.

5. Der MAT-Fab-Antikörper gemäß einem der Ansprüche 1 bis 4, wobei die CH2-Domänen der schweren Kette (a) und der Fc-Kette (d) jeweils ein oder mehrere Mutationen umfassen, um wenigstens eine Fc-Effektor-Funktion zu verringern oder zu eliminieren, vorzugsweise wobei die CH2-Domäne der schweren Kette und die CH2-Domäne der Fc-Kette jeweils zwei Mutationen umfassen, um Leucin234 in Alanin abzuändern und Leucin235 in Alanin abzuändern (EU-Nummerierung).

6. Der MAT-Fab-Antikörper gemäß Anspruch 1, umfassend:
(a) eine schwere Kette, umfassend die Aminosäuresequenz der Aminosäurereste 23 - 691 von SEQ ID NO:1,
(b) eine erste leichte Kette, umfassend die Aminosäuresequenz der Aminosäurereste 20 - 244 von SEQ ID NO:2,
(c) eine zweite leichte Kette, umfassend die Aminosäuresequenz der Aminosäurereste 21 - 235 von SEQ ID NO:3, und
(d) eine Fc-Kette, umfassend die Aminosäuresequenz der Aminosäurereste 23 - 253 von SEQ ID NO:4;
oder
(a) eine schwere Kette, umfassend die Aminosäuresequenz der Aminosäurereste 23 - 691 von SEQ ID NO:5,
(b) eine erste leichte Kette, umfassend die Aminosäuresequenz der Aminosäurereste 20 - 244 von SEQ ID NO:6,
(c) eine zweite leichte Kette, umfassend die Aminosäuresequenz der Aminosäurereste 21 - 235 von SEQ ID NO:7, und
(d) eine Fc-Kette, umfassend die Aminosäuresequenz der Aminosäurereste 23 - 253 von SEQ ID NO:8.

7. Der MAT-Fab-Antikörper gemäß einem der Ansprüche 1-5, wobei der MAT-Fab-Antikörper zwei verschiedene Zielantigene oder zwei verschiedene Epitope bindet, vorzugsweise wobei die zwei verschiedenen Zielantigene oder die zwei verschiedenen Epitope ausgewählt sind aus der Gruppe von Antigen-Paaren, bestehend aus:
CD20 und CD3, CD3 und CD19, CD3 und Fc-Gamma-Rllla, CD3 und TPBG, CD3 und Epha10, CD3 und IL-5Rα, CD3 und TASCTD-2, CD3 und CLEC12A, CD3 und Prominin-1, CD3 und IL-23R, CD3 und ROR1, CD3 und IL-3Rα, CD3 und PSA, CD3 und CD8, CD3 und Glypican 3, CD3 und FAP, CD3 und EphA2, CD3 und ENPP3, CD3 und CD33, CD3 und CD133, CD3 und EpCAM, CD3 und CD19, CD3 und Her2, CD3 und CEA, CD3 und GD2, CD3 und PSMA, CD3 und BCMA, CD3 und A33, CD3 und B7-H3, CD3 und EGFR, CD3 und P-Cadherin, CD3 und HMW-MAA, CD3 und TIM-3, CD3 und CD38, CD3 und TAG-72, CD3 und SSTR, CD3 und FRA, CD16 und CD30, CD64 und Her2, CD 137 und CD20, CD138 und CD20, CD19 und CD20, CD38 und CD20, CD20 und CD22, CD40 und CD20, CD47 und CD20, CD 137 und EGFR, CD137 und Her-2, CD 137 und PD-1, CD 137 und PDL-1, PD-1 und PD-L1, VEGF und PD-L1, Lag-3 und TIM-3, OX40 und PD-1, TIM-3 und PD-1, TIM-3 und PDL-1, EGFR und DLL-4, VEGF und EGFR, HGF und VEGF, ein erstes Epitop von VEGF und ein anderes zweites Epitop von VEGF, VEGF und Ang2, EGFR und cMet, PDGF und VEGF, VEGF und DLL-4, OX40 und PD-L1, ICOS und PD-1, ICOS und PD-L1, Lag-3 und PD-1, Lag-3 und PD-L1, Lag-3 und CTLA-4, ICOS und CTLA-4, CD138 und CD40, CD38 und CD138, CD38 und CD40, CD-8 und IL-6, CSPGs und RGM A, CTLA-4 und BTN02, CTLA-4 und PD-1, IGF1 und IGF2, IGF1/2 und Erb2B, IGF-IR und EGFR, EGFR und CD13, IGF-IR und ErbB3, EGFR-2 und IGFR, ein erstes Epitop von Her2 und ein zweites anderes Epitop von Her2, Faktor IXa und Met, Faktor X und Met, VEGFR-2 und Met, VEGF-A und Angiopoietin-2 (Ang-2), IL-12 und TWEAK, IL-13 und IL-1β, MAG und RGM A, NgR und RGM A, NogoA und RGM A, OMGp und RGM A, PDL-1 und CTLA-4, PD-1 und TIM-3, RGM A und RGM B, Te38 und TNFα, TNFα und Blys, TNFα und CD-22, TNFα und ein CTLA-4, TNFα und GP130, TNFα und IL-12p40, und TNFα und RANK-Ligand.

8. Der MAT-Fab-Antikörper gemäß einem der Ansprüche 1-6, wobei eines der zwei verschiedenen Zielantigene, die durch den MAT-Fab-Antikörper gebunden werden, ein Antigen ist,
wobei das Antigen vorzugsweise ausgewählt ist aus der Gruppe bestehend aus: CD3, CD16 und CD64, exprimiert an der Oberfläche einer Effektorzelle, und
wobei die Effektorzelle vorzugsweise ausgewählt ist aus der Gruppe bestehend aus: einer T-Zelle, einer natürlichen Killerzelle (NK-Zelle), einem Monozyten, einem Neutrophilen und einem Makrophagen, und
wobei das andere Zielantigen, das durch den MAT-Fab-Antikörper gebunden wird, ein störungsassoziiertes Antigen ist, das an der Oberfläche einer Zielzelle exprimiert wird, welche als für ein humanes Subjekt schädlich erachtet wird, und wobei die schädliche Zielzelle vorzugsweise ausgewählt ist aus der Gruppe bestehend aus: einer Tumorzelle, einer autoreaktiven Zelle und einer virusinfizierten Zelle.

9. Der MAT-Fab-Antikörper gemäß Anspruch 8, wobei das störungsassoziierte Antigen, das an der Oberfläche der schädlichen Zielzelle exprimiert wird, ein tumorassoziiertes Antigen ist, das an einer Tumorzelle exprimiert wird,
vorzugsweise:
(i) wobei das tumorassoziierte Antigen ausgewählt ist aus der Gruppe bestehend aus: CD19, CD20, humanem epidermalem Wachstumsfaktor-Rezeptor 2 (HER2), carcinoembryonalem Antigen (CEA), epithelialem Zelladhäsionsmolekül (EpCAM) und Rezeptor-Tyrosinkinase-ähnlichem Orphan-Rezeptor 1 (ROR 1); und/oder
(ii) wobei die Tumorzelle eine maligne B-Zelle ist, und besonders bevorzugt wobei die maligne B-Zelle eine Zelle einer Krebserkrankung ist, ausgewählt aus der Gruppe bestehend aus: akuter lymphatischer Leukämie, Hodgkin-Lymphom, Nicht-Hodgkin-Lymphom (NHL), lymphoblastischer Vorläufer-B-Zell-Leukämie/Lymphom, reifen B-Zell-Neoplasien, chronischer lymphatischer B-Zell-Leukämie/kleinem lymphatischen Lymphom, prolymphatischer B-Zell-Leukämie, lymphoplasmozytischem Lymphom, Mantelzelllymphom, follikulärem Lymphom, kutanem Follikelzentrumslymphom, Randzonen-B-Zell-Lymphom, Haarzellleukämie, diffusem großzelligem B-Zell-Lymphom, Burkitt-Lymphom, Plasmozytom, Plasmazellmyelom, lymphoproliferativer Erkrankung nach Transplantation, Waldenström-Makroglobulinämie und anaplastischem großzelligem Lymphom.

10. Der MAT-Fab-Antikörper gemäß einem der Ansprüche 1-9, konjugiert an ein Mittel, ausgewählt aus der Gruppe bestehend aus: einem therapeutischen Mittel, einem bildgebenden Mittel und einem zytotoxischen Mittel,
vorzugsweise
(i) wobei das bildgebende Mittel ausgewählt ist aus der Gruppe bestehend aus: einer radioaktiven Markierung, einem Enzym, einer fluoreszierenden Markierung, einer lumineszierenden Markierung, einer biolumineszierenden Markierung, einer magnetischen Markierung, Biotin, Streptavidin und Avidin,
und
(ii) wobei das therapeutische oder zytotoxische Mittel ausgewählt ist aus der Gruppe bestehend aus: einem Antimetaboliten, einem Alkylierungsmittel, einem Antibiotikum, einem Wachstumsfaktor, einem Zytokin, einem antiangiogenetischem Mittel, einem antimitotischen Mittel, einem Anthracyclin, einem Toxin und einem apoptotischen Mittel.

11. Eine pharmazeutische Zusammensetzung, umfassend einen MAT-Fab-Antikörper gemäß einem der Ansprüche 1-9 und einen pharmazeutisch annehmbaren Träger, wobei die pharmazeutische Zusammensetzung zur parenteralen oder nichtparenteralen Verabreichung an ein Individuum hergestellt wurde, und
vorzugsweise für wenigstens einen Verabreichungsweg, ausgewählt aus der Gruppe bestehend aus: parenteral, subkutan, intramuskulär, intravenös, intraartikulär, intrabronchial, intraabdominal, intrakapsulär, intrakartilaginär, intrakavitär, intrazelial, intrazerebellär, intrazerebroventrikulär, intrakolonisch, intrazervikal, intragastrisch, intrahepatisch, intramyokardial, intraossal, intrapelvisch, intraperikardial, intraperitoneal, intrapleural, intraprostatisch, intrapulmonal, intrarektal, intrarenal, intraretinal, intraspinal, intrasynovial, intrathorakal, intrauterin, intravesikal, mittels Bolus, vaginal, rektal, bukkal, sublingual, intranasal und transdermal.

12. Ein isoliertes Polynukleotid oder isolierte Polynukleotide, das/die alle vier Polypeptide eines MAT-Fab-Antikörpers gemäß einem der Ansprüche 1-9 kodiert/kodieren.

13. Ein Vektor, umfassend das isolierte Polynukleotid oder die isolierten Polynukleotide gemäß Anspruch 12, vorzugsweise wobei der Vektor ausgewählt ist aus der Gruppe bestehend aus: pcDNA, pTT pTT3, pEFBOS, pBV, pJV, pcDNA3.1 TOPO, pEF6 TOPO und pBJ.

14. Eine isolierte Wirtszelle, umfassend einen Vektor gemäß Anspruch 13.

15. Eine isolierte Wirtszelle gemäß Anspruch 14, die eine Säuger-Wirtszelle ist.

16. Ein Verfahren zur Herstellung eines MAT-Fab-Antikörpers, umfassend das Kultivieren einer Wirtszelle wie in Anspruch 14 oder Anspruch 15 beschrieben in einem Kulturmedium unter Bedingungen, die ausreichen, um den MAT-Fab-Antikörper zu erzeugen.

17. Ein MAT-Fab-Antikörper, der gemäß dem Verfahren nach Anspruch 16 erzeugt wurde.

18. Ein MAT-Fab-Antikörper gemäß einem der Ansprüche 1-9 und 17 oder eine pharmazeutische Zusammensetzung gemäß Anspruch 11 zur Verwendung als Medikament.

19. Ein MAT-Fab-Antikörper gemäß einem der Ansprüche 1-9 und 17 oder eine Zusammensetzung gemäß Anspruch 11 zur Verwendung bei der Therapie einer Erkrankung oder Störung bei einem Individuum, wobei der MAT-Fab-Antikörper ein Epitop oder Antigen bindet, das an der Oberfläche einer Krebszelle, einer autoreaktiven Zelle oder einer virusinfizierten Zelle exprimiert wird, wobei die Bindung des MAT-Fab-Antikörpers an die Krebszelle, eine autoreaktive Zelle oder eine virusinfizierte Zelle eine Behandlung der Erkrankung oder Störung ergibt.

20. Ein MAT-Fab-Antikörper gemäß einem der Ansprüche 1-9 und 17 oder eine Zusammensetzung gemäß Anspruch 11 zur Verwendung bei einem Verfahren zur Behandlung eines malignen B-Zell-Tumors bei einem menschlichen Subjekt, wobei das Verfahren die Verabreichung des MAT-Fab-Antikörpers, der ein Antigen an einer Effektorzelle bindet, wobei vorzugsweise die Effektorzelle ausgewählt ist aus der Gruppe bestehend aus: einer T-Zelle, einer natürlichen Killerzelle (NK-Zelle), einem Monozyten, einem Neutrophilen und einem Makrophagen, und der ein Zelloberflächenantigen an einer malignen B-Zelle bindet, an das Individuum, das eine solche Behandlung benötigt, umfasst, vorzugsweise wobei der MAT-Fab-Antikörper ein Antigen an malignen B-Zellen einer Krebserkrankung bindet, ausgewählt aus der Gruppe bestehend aus: akuter lymphatischer Leukämie, Hodgkin-Lymphom, Nicht-Hodgkin-Lymphom (NHL), lymphoblastischer Vorläufer-B-Zell-Leukämie/Lymphom, reifen B-Zell-Neoplasien, chronischer lymphatischer B-Zell-Leukämie/kleinem lymphatischen Lymphom, prolymphatischer B-Zell-Leukämie, lymphoplasmozytischem Lymphom, Mantelzelllymphom, follikulärem Lymphom, kutanem Follikelzentrumslymphom, Randzonen-B-Zell-Lymphom, Haarzellleukämie, diffusem großzelligem B-Zell-Lymphom, Burkitt-Lymphom, Plasmozytom, Plasmazellmyelom, lymphoproliferativer Erkrankung nach Transplantation, Waldenström-Makroglobulinämie und anaplastischem großzelligem Lymphom.

## Revendications

1. Anticorps bispécifique tandem asymétrique monovalent Fab (anticorps MAT-Fab) comprenant des chaînes polypeptidiques (a), (b), (c) et (d), dans lequel :
(a) est une chaîne polypeptidique lourde (chaîne lourde), dans laquelle ladite chaîne lourde comprend (de la terminaison amino à la terminaison carboxyle) : VL_{A}-CL-VH_{B}-CH1-charnière-CH2-CH3m1, dans lequel :
VL_{A} est un domaine variable de chaîne légère d'immunoglobuline humaine qui est fusionné directement à CL, qui est un domaine constant de chaîne légère humaine, où VL_{A}-CL est un composant de chaîne légère d'immunoglobuline d'une première unité de liaison Fab reconnaissant un premier antigène ou épitope et est fusionné directement à VH_{B}, où VH_{B} est un domaine variable de chaîne lourde d'immunoglobuline humaine fusionné directement à CH1, qui est un domaine constant CH1 de chaîne lourde d'immunoglobuline humaine, où VH_{B}-CH1 est le composant de chaîne lourde d'immunoglobuline d'une deuxième unité de liaison Fab reconnaissant un deuxième antigène ou épitope, et où VH_{B}-CH1 est fusionné directement à une charnière-CH2, où la charnière-CH2 est la région charnière-CH2 d'une chaîne lourde d'immunoglobuline et où la charnière-CH2 est fusionné directement à CH3m1, qui est un premier domaine constant CH3 de chaîne lourde d'immunoglobuline humaine qui a été muté avec une ou plusieurs mutations boutons-dans-trous (KiH) pour former un bouton structurel ou un trou structurel dans ledit domaine constant CH3m1, où A et B sont des antigènes différents ou des épitopes différents ;
(b) est une première chaîne légère MAT-Fab comprenant VH_{A}-CH1, où VH_{A} est un domaine variable de chaîne lourde d'immunoglobuline humaine qui est fusionné directement à CH1, qui est un domaine constant CH1 de chaîne lourde d'immunoglobuline humaine, et où VH_{A}-CH1 est le composant de chaîne lourde d'immunoglobuline de ladite première unité de liaison Fab ;
(c) une deuxième chaîne légère MAT-Fab comprenant VL_{B}-CL, où VL_{B} est un domaine variable de chaîne légère d'immunoglobuline humaine qui est fusionné directement à CL, qui est un domaine CL de chaîne légère d'immunoglobuline humaine, et où VL_{B}-CL est le composant de chaîne légère d'immunoglobuline de ladite deuxième unité de liaison Fab ; et
(d) est une chaîne polypeptidique Fc (chaîne Fc) comprenant charnière-CH2-CH3m2, où charnière-CH2 est la région charnière-CH2 d'une chaîne lourde d'immunoglobuline et où charnière-CH2 est fusionnée directement à CH3m2, qui est un deuxième domaine constant CH3 de chaîne lourde d'immunoglobuline humaine qui a été muté avec une ou plusieurs mutations boutons-dans-trous (KiH) pour former un bouton structurel ou un trou structurel dans ledit domaine constant CH3m2 ;
à condition que :
lorsque le domaine CH3m1 de la chaîne lourde a été muté pour former un bouton structurel, alors le domaine CH3m2 de la chaîne Fc a été muté pour former un trou structurel complémentaire afin de favoriser l'appariement du domaine CH3m1 avec le domaine CH3m2 ; et
lorsque le domaine CH3m1 de ladite chaîne lourde a été muté pour former un trou structurel, alors le domaine CH3m2 de la chaîne Fc a été muté pour former un bouton structurel complémentaire afin de favoriser l'appariement du domaine CH3m1 avec le domaine CH3m2 ; et
ledit anticorps MAT-Fab comprend facultativement une mutation dans le domaine CH3m1 et le domaine CH3m2 pour introduire un résidu cystéine afin de favoriser la formation de liaison disulfure lors de l'appariement du domaine CH3m1 avec le domaine CH3m2.

2. Anticorps MAT-Fab selon la revendication 1, dans lequel les une ou plusieurs mutations KiH dans le domaine CH3m1 ou le domaine CH3m2 pour former un bouton structurel est ou sont un changement d'un résidu thréonine en un résidu tyrosine ou un changement d'un résidu thréonine en un résidu tryptophane,
préférentiellement :
(i) dans lequel la mutation KiH change une thréonine en une tyrosine en position 610 de SEQ ID NO:1 du domaine CH3 de la chaîne lourde de l'anticorps MAT-Fab, ou
(ii) dans lequel la mutation KiH change une thréonine en un tryptophane en position 610 de SEQ ID NO:5 du domaine CH3 de la chaîne lourde de l'anticorps MAT-Fab.

3. Anticorps MAT-Fab selon la revendication 1, dans lequel une mutation dans le domaine CH3m1 ou le domaine CH3m2 pour former un trou structurel est un changement d'un résidu tyrosine en un résidu thréonine ou une combinaison d'un changement d'un résidu thréonine en un résidu sérine, d'un changement d'un résidu leucine en un résidu alanine, et d'un changement d'un résidu tyrosine en un résidu valine ;
préférentiellement
(i) dans lequel la mutation visant à changer un résidu tyrosine en un résidu thréonine change la tyrosine en thréonine en position 213 de SEQ ID NO:4 du domaine CH3 de la chaîne Fc de l'anticorps MAT-Fab,
ou
(ii) dans lequel la combinaison d'un changement d'un résidu thréonine en un résidu sérine, d'un changement d'un résidu leucine en un résidu alanine, et d'un changement d'un résidu tyrosine en un résidu valine est une combinaison d'un changement d'une thréonine en une sérine en position 172 de SEQ ID NO : 8, d'un changement d'une leucine en une alanine en position 174 de SEQ ID NO:8, et d'un changement d'une tyrosine en une valine en position 213 de SEQ ID NO:8 du domaine CH3 de la chaîne Fc de l'anticorps MAT-Fab.

4. Anticorps MAT-Fab selon l'une quelconque des revendications 1-3, dans lequel chacun du domaine CH3m1 et du domaine CH3m2 comprend en outre une mutation visant à remplacer un résidu d'acide aminé par une cystéine afin de favoriser la formation de liaison disulfure entre les domaines CH3m1 et CH3m2,
préférentiellement
(i) dans lequel la mutation est un changement d'une sérine en une cystéine ou une tyrosine en une cystéine ; ou
(ii) dans lequel la mutation est un changement d'un résidu sérine en un résidu cystéine en position 598 de SEQ ID NO:5 du domaine CH3 de la chaîne lourde de l'anticorps MAT-Fab, et/ou dans lequel la mutation est un changement d'un résidu tyrosine en un résidu cystéine en position 155 de SEQ ID NO:8 du domaine CH3 de la chaîne Fc de l'anticorps MAT-Fab.

5. Anticorps MAT-Fab selon l'une quelconque des revendications 1 à 4, dans lequel les domaines CH2 de la chaîne lourde (a) et de la chaîne Fc (d) comprennent chacun une ou plusieurs mutations permettant de réduire ou d'éliminer au moins une fonction effectrice Fc,
préférentiellement dans laquelle le domaine CH2 de la chaîne lourde et le domaine CH2 de la chaîne Fc comprennent chacun deux mutations pour changer la leucine234 en alanine et pour changer la leucine235 en alanine (numérotation EU).

6. Anticorps MAT-Fab selon la revendication 1, comprenant :
(a) une chaîne lourde comprenant la séquence d'acides aminés des résidus d'acides aminés 23-691 de SEQ ID NO:1,
(b) une première chaîne légère comprenant la séquence d'acides aminés des résidus d'acides aminés 20 - 244 de SEQ ID NO:2,
(c) une deuxième chaîne légère comprenant la séquence d'acides aminés des résidus d'acides aminés 21 - 235 de SEQ ID NO:3, et
(d) une chaîne Fc comprenant la séquence d'acides aminés des résidus d'acides aminés 23 -253 de SEQ ID NO:4 ;
ou
(a) une chaîne lourde comprenant la séquence d'acides aminés des résidus d'acides aminés 23 ¬691 de SEQ ID NO:5,
(b) une première chaîne légère comprenant la séquence d'acides aminés des résidus d'acides aminés 20 - 244 de SEQ ID NO:6,
(c) une deuxième chaîne légère comprenant la séquence d'acides aminés des résidus d'acides aminés 21 - 235 de SEQ ID NO:7, et
(d) une chaîne Fc comprenant la séquence d'acides aminés des résidus d'acides aminés 23 ¬253 de SEQ ID NO:8.

7. Anticorps MAT-Fab selon l'une quelconque des revendications 1 à 5, dans lequel l'anticorps MAT-Fab lie deux antigènes cibles différents ou deux épitopes différents, préférentiellement dans lequel les deux antigènes cibles différents ou les deux épitopes différents sont choisis dans le groupe des paires d'antigènes consistant en : CD20 et CD3, CD3 et CD19, CD3 et Fc-gamma-RIIIA, CD3 et TPBG, CD3 et Ephal0, CD3 et IL-5Rα, CD3 et TASCTD-2, CD3 et CLEC12A, CD3 et Prominin-1, CD3 et IL-23R, CD3 et ROR1, CD3 et IL-3Rα, CD3 et PSA, CD3 et CD8, CD3 et Glypican 3, CD3 et FAP, CD3 et EphA2, CD3 et ENPP3, CD3 et CD33, CD3 et CD133, CD3 et EpCAM, CD3 et CD19, CD3 et Her2, CD3 et CEA, CD3 et GD2, CD3 et PSMA, CD3 et BCMA, CD3 et A33, CD3 et B7-H3, CD3 et EGFR, CD3 et P-cadhérine, CD3 et HMW-MAA, CD3 et TIM-3, CD3 et CD38, CD3 et TAG-72, CD3 et SSTR, CD3 et FRA, CD16 et CD30, CD64 et Her2, CD 137 et CD20, CD138 et CD20, CD19 et CD20, CD38 et CD20, CD20 et CD22, CD40 et CD20, CD47 et CD20, CD 137 et EGFR, CD137 et Her-2, CD 137 et PD-1, CD 137 et PDL-1, PD-1 et PD-L1, VEGF et PD-L1, Lag-3 et TIM- 3, OX40 et PD-1, TIM-3 et PD-1, TIM-3 et PDL-1, EGFR et DLL-4, VEGF et EGFR, HGF et VEGF, un premier épitope du VEGF et un second épitope différent du VEGF, VEGF et Ang2, EGFR et cMet, PDGF et VEGF, VEGF et DLL-4, OX40 et PD-L1, ICOS et PD-1, ICOS et PD-L1, Lag-3 et PD-1, Lag-3 et PD-L1, Lag-3 et CTLA-4, ICOS et CTLA-4, CD138 et CD40, CD38 et CD138, CD38 et CD40, CD-8 et IL-6, CSPGs et RGM A, CTLA-4 et BTN02, CTLA-4 et PD-1, IGF1 et IGF2, IGF1/2 et Erb2B, IGF-IR et EGFR, EGFR et CD13, IGF-IR et ErbB3, EGFR-2 et IGFR, un premier épitope de Her2 et un second épitope différent de Her2, Facteur IXa et Met, facteur X et Met, VEGFR-2 et Met, VEGF-A et Angiopoietin-2 (Ang-2), IL-12 et TWEAK, IL-13 et IL-1β, MAG et RGM A, NgR et RGM A, NogoA et RGM A, OMGp et RGM A, PDL-1 et CTLA-4, PD-1 et TIM-3, RGM A et RGM B, Te38 et TNFα, TNFα et Blys, TNFα et CD-22, TNFα et CTLA-4, TNFα et GP130, TNFα et IL-12p40, et TNFα et ligand RANK.

8. Anticorps MAT-Fab selon l'une quelconque des revendications 1 à 6, dans lequel l'un des deux antigènes cibles différents liés par l'anticorps MAT-Fab est un antigène,
dans lequel ledit antigène est préférentiellement choisi dans le groupe consistant en : CD3, CD16 et CD64, exprimés à la surface d'une cellule effectrice, et
dans lequel ladite cellule effectrice est préférentiellement sélectionnée dans le groupe consistant en : une cellule T, une cellule tueuse naturelle (NK), un monocyte, un neutrophile et un macrophage, et
dans lequel l'autre antigène cible lié par l'anticorps MAT-Fab est un antigène associé à un trouble exprimé à la surface d'une cellule cible considérée comme néfaste pour un sujet humain, et dans lequel ladite cellule cible nuisible est préférentiellement sélectionnée dans le groupe consistant en : une cellule tumorale, une cellule autoréactive, et une cellule infectée par un virus.

9. Anticorps MAT-Fab selon la revendication 8, dans lequel l'antigène associé à un trouble exprimé à la surface de la cellule cible néfaste est un antigène associé à une tumeur exprimé sur une cellule tumorale, préférentiellement :
(i) dans lequel l'antigène associé à une tumeur est choisi dans le groupe consistant en : CD19, CD20, récepteur 2 du facteur de croissance épidermique humain (HER2), antigène carcinoembryonnaire (CEA), molécule d'adhésion des cellules épithéliales (EpCAM) et récepteur 1 orphelin de type tyrosine kinase (ROR 1) ; et/ou
(ii) dans lequel ladite cellule tumorale est une cellule B maligne et plus préférentiellement dans lequel ladite cellule B maligne est une cellule d'un trouble cancéreux choisi dans le groupe consistant en : leucémie lymphoblastique aiguë, lymphome de Hodgkin, lymphome non hodgkinien (LNH), leucémie/lymphome lymphoblastique à cellules B précurseurs, néoplasmes à cellules B matures, leucémie lymphocytaire chronique à cellules B/lymphome lymphocytaire de petite taille, leucémie prolymphocytaire à cellules B, lymphome lymphoplasmocytaire, lymphome à cellules du manteau, lymphome folliculaire, lymphome cutané des centres folliculaires, lymphome à cellules B de la zone marginale, leucémie à tricholeucocytes, lymphome diffus à grandes cellules B, lymphome de Burkitt, plasmocytome, myélome à cellules plasmatiques, trouble lymphoprolifératif post-transplantation, macroglobulinémie de Waldenstrom et lymphome anaplasique à grandes cellules.

10. Anticorps MAT-Fab selon l'une quelconque des revendications 1 à 9, conjugué à un agent choisi dans le groupe consistant en : un agent thérapeutique, un agent d'imagerie, et un agent cytotoxique, préférentiellement
(i) dans lequel l'agent d'imagerie est choisi dans le groupe consistant en : un marqueur radioactif, une enzyme, un marqueur fluorescent, un marqueur luminescent, un marqueur bioluminescent, un marqueur magnétique, la biotine, la streptavidine et l'avidine,
et
(ii) dans lequel l'agent thérapeutique ou cytotoxique est choisi dans le groupe consistant en : un anti-métabolite, un agent d'alkylation, un antibiotique, un facteur de croissance, une cytokine, un agent anti-angiogénique, un agent anti-mitotique, une anthracycline, une toxine et un agent apoptotique.

11. Composition pharmaceutique comprenant un anticorps MAT-Fab selon l'une des revendications 1 à 9 et un support pharmaceutiquement acceptable, dans laquelle ladite composition pharmaceutique est préparée pour une administration parentérale ou non parentérale à un individu, et
préférentiellement pour au moins un mode d'administration choisi dans le groupe consistant en : parentérale, sous-cutanée, intramusculaire, intraveineuse, intrarticulaire, intrabronchique, intraabdominale, intracapsulaire, intracartilagineuse, intracavitaire, intracéliale, intracérébelleuse, intracérébroventriculaire, intracolique, intracervicale, intragastrique, intrahépatique, intramyocardique, intra-ostéale, intrapelvienne, intrapéricardique, intrapéritonéale, intrapleurale, intraprostatique, intrapulmonaire, intra-rectale, intrarénale, intrarétinienne, intraspinale, intrasynoviale, intrathoracique, intra-utérine, intravésicale, en bolus, vaginale, rectale, buccale, sublinguale, intranasale et transdermique.

12. Polynucléotide isolé ou polynucléotides isolés encodant chacun des quatre polypeptides d'un anticorps MAT-Fab selon l'une quelconque des revendications 1 à 9.

13. Vecteur comprenant le polynucléotide isolé ou les polynucléotides isolés selon la revendication 12, préférentiellement dans lequel ledit vecteur est choisi dans le groupe consistant en : pcDNA, pTT pTT3, pEFBOS, pBV, pJV, pcDNA3.1 TOPO, pEF6 TOPO, et pBJ.

14. Cellule hôte isolée comprenant un vecteur selon la revendication 13.

15. Cellule hôte isolée selon la revendication 14, qui est une cellule hôte de mammifère.

16. Procédé de production d'un anticorps MAT-Fab comprenant la mise en culture d'une cellule hôte décrite dans la revendication 14 ou la revendication 15 dans un milieu de culture dans des conditions suffisantes pour produire l'anticorps MAT-Fab.

17. Anticorps MAT-Fab produit selon le procédé de la revendication 16.

18. Anticorps MAT-Fab selon l'une des revendications 1 à 9 et 17 ou composition pharmaceutique selon la revendication 11 pour utilisation comme médicament.

19. Anticorps MAT-Fab selon l'une des revendications 1 à 9 et 17 ou composition selon la revendication 11 pour utilisation en thérapie thérapie d'une maladie ou d'un trouble chez un individu, dans lequel ou laquelle l'anticorps MAT-Fab se liant à un épitope ou à un antigène exprimé à la surface d'une cellule cancéreuse, d'une cellule autoréactive ou d'une cellule infectée par un virus, dans lequel ou laquelle la liaison de l'anticorps MAT-Fab à la cellule cancéreuse, à une cellule autoréactive ou à une cellule infectée par un virus offre un traitement pour la maladie ou le trouble.

20. Anticorps MAT-Fab selon l'une quelconque des Revendications 1 à 9 et 17 ou composition selon la Revendication 11 pour utilisation dans un procédé de traitement d'un sujet humain pour une tumeur maligne à cellules B, dans lequel ou laquelle ledit procédé comprend l'administration à l'individu ayant besoin d'un tel traitement dudit anticorps MAT-Fab qui se lie à un antigène sur une cellule effectrice, dans lequel ou laquelle de préférence la cellule effectrice est choisie dans le groupe consistant en : une cellule T, une cellule tueuse naturelle (NK), un monocyte, un neutrophile et un macrophage, et qui lie un antigène de surface cellulaire sur une cellule B maligne, préférentiellement dans lequel ledit anticorps MAT-Fab lie un antigène sur des cellules B malignes d'un trouble cancéreux choisi dans le groupe consistant en : leucémie lymphoblastique aiguë, lymphome de Hodgkin, lymphome non hodgkinien (LNH), leucémie/lymphome lymphoblastique à cellules B précurseurs, néoplasmes à cellules B matures, leucémie lymphocytaire chronique à cellules B/lymphome lymphocytaire de petite taille, leucémie prolymphocytaire à cellules B, lymphome lymphoplasmocytaire, lymphome à cellules du manteau, lymphome folliculaire, lymphome cutané des centres folliculaires, lymphome à cellules B de la zone marginale, leucémie à tricholeucocytes, lymphome diffus à grandes cellules B, lymphome de Burkitt, plasmocytome, myélome à cellules plasmatiques, trouble lymphoprolifératif post-transplantation, macroglobulinémie de Waldenstrom et lymphome anaplasique à grandes cellules.
